# EUROPÄISCHE PATENTSCHRIFT

(11) **EP 2 225 192 B1**
(45) Veröffentlichungstag und Bekanntmachung des Hinweises auf die Patenterteilung: **03.05.2017**
(21) Anmeldenummer: 08854428.3
(22) Anmeldetag: 13.11.2008
(51) Int. Cl.: C07C 29/56, C07C 29/78, C07C 45/62, C07C 45/82, C07C 29/17

(54) **VERFAHREN ZUR HERSTELLUNG VON OPTISCH AKTIVEM UND RACEMISCHEM MENTHOL**
METHOD FOR PRODUCING OPTICALLY ACTIVE, RACEMIC MENTHOL
PROCÉDÉ DE PRODUCTION DE MENTHOL RACÉMIQUE ET OPTIQUEMENT ACTIF

(30) Priorität: 30.11.2007 EP 07122036
(43) Veröffentlichungstag der Anmeldung: 08.09.2010
(73) Patentinhaber: BASF SE, 67056 Ludwigshafen am Rhein (DE)
(72) Erfinder: HEYDRICH, Gunnar, 67117 Limburgerhof (DE); GRALLA, Gabriele, 68161 Mannheim (DE); RAULS, Matthias, 66440 Blieskastel (DE); SCHMIDT-LEITHOFF, Joachim, 68165 Mannheim (DE); EBEL, Klaus, 68623 Lampertheim (DE); KRAUSE, Wolfgang, 68782 Brühl-Rohrhof (DE); OEHLENSCHLÄGER, Steffen, 67063 Ludwigshafen (DE); JÄKEL, Christoph, 67117 Limburgerhof (DE); FRIEDRICH, Marko, 64653 Lorsch (DE); BERGNER, Eike Johannes, 77948 Friesenheim-Heiligenzell (DE); KASHANI-SHIRAZI, Nawid, 68161 Mannheim (DE); PACIELLO, Rocco, 67098 Bad Dürkheim (DE)
(74) Vertreter: BASF IP Association
(86) Internationale Anmeldenummer: PCT/EP2008/065503
(87) Internationale Veröffentlichungsnummer: WO 2009/068444

(56) Entgegenhaltungen:
- EP-A- 0 694 514
- EP-A- 1 053 974
- EP-A- 1 225 163
- WO-A-2006/092433
- WO-A-2008/025851
- DE-A1- 10 239 274
- US-A- 4 237 072
- TRASARTI ET AL: "Design of catalyst systems for the one-pot synthesis of menthols from citral" JOURNAL OF CATALYSIS, ACADEMIC PRESS, DULUTH, MN, US, Bd. 247, Nr. 2, 30. März 2007 (2007-03-30), Seiten 155-165, XP022005868 ISSN: 0021-9517
- TRASARTI A F ET AL: "Highly selective synthesis of menthols from citral in a one-step process" JOURNAL OF CATALYSIS, ACADEMIC PRESS, DULUTH, MN, US, Bd. 224, Nr. 2, 10. Juni 2004 (2004-06-10), Seiten 484-488, XP004506509 ISSN: 0021-9517

## Beschreibung

Die vorliegende Erfindung betrifft ein besonders wirtschaftliches Gesamtverfahren zur Herstellung von Menthol, speziell zur Herstellung von optisch aktivem, im wesentlichen enantiomeren- und diastereomerenreinem L-Menthol und racemischem Menthol ausgehend von dem im technischem Maßstab wohlfeil zugänglichen Ausgangsstoff Citral. Speziell betrifft die vorliegende Erfindung ein Verfahren zur Herstellung von optisch aktivem, im wesentlichen enantiomerenreinen Menthol und racemischem Menthol unter weitgehender Vermeidung unerwünschter Abfall und Nebenprodukte.

Menthol, speziell das linksdrehende Enantiomer L-(-)-Menthol stellt aufgrund seiner wohlbekannten kühlenden Eigenschaften eine der wirtschaftlich bedeutendsten Aromachemikalien dar. Der weltweit auf etwa 16500 t geschätzte Bedarf an optisch aktivem Menthol wird dabei nach wie vor weitgehend aus natürlichen Quellen wie beispielsweise durch Kristallisation des L-Menthols aus natürlichen Ölen, speziell dem aus Mentha arvensis gepressten Öl bei tiefer Temperatur gedeckt. Verfügbarkeit und Qualität des so verfügbaren Menthols sind daher stark von Schwankungen der jährlichen Ernteerträge und somit von schwer vorhersehbaren klimatischen Faktoren abhängig, was sich unvorteilhaft auf die Preisstabilität der genannten Rohware auswirken kann. Daneben besteht weltweit eine hohe Nachfrage nach racemischem Menthol in guter Qualität, das aufgrund seines geringeren Preises üblicherweise eingesetzt wird, wenn dies für die jeweilige Anwendung geeignet erscheint.

Verfahren zur Synthese von racemischem oder optisch aktivem Menthol sind dem Fachmann seit langem bekannt und ausführlich beispielsweise in "Mint: the genus mentha" von R. Hopp und B. M. Lawrence, CRC Press, Taylor & Francis Group, 2007, Seiten 371 - 397 beschrieben. Die bekannten Verfahren zur Herstellung von Menthol führen entweder zu racemischem oder zu optisch aktivem Menthol, wobei optisch aktives Menthol partialsynthetisch ausgehend von in optisch aktiver Form aus natürlichen Quellen verfügbaren Ausgangsstoffen erhalten wird. Daneben existiert ein Verfahren zur Herstellung von L-Menthol ausgehend von Myrcen, welches zunächst in ein Enamin überführt und anschließend einer asymmetrischen Isomerisierung unterworfen wird wie beispielsweise in "Catalytic Asymmetric Synthesis", von S. Akutagawa, K. Tani, Wiley-VCH, 2000, Kapitel 3, S. 145-161 und S. Otsuka, K. Tani, Synthesis 1992, 665-680 beschrieben.

Ein Ziel der enantioselektiven Synthesen von L-Menthol besteht darin, die genannte Verbindung in hoher Enantiomerenreinheit bereitzustellen. Dabei wird bei enantioselektiven totalsynthetischen Verfahren zur Herstellung von Menthol das Ausmaß, in dem ein Enantiomer bevorzugt vor dem anderen erhalten wird, durch die asymmetrische Induktion der Reaktion bestimmt, bei der das erste asymmetrische Kohlenstoffatom entsteht, der so genannten chirogenen Stufe.

Bei in technischem Maßstab durchzuführenden Synthesen sind solche chirogenen Stufen bzw. Reaktionen zu bevorzugen, bei denen enantioselektive Katalysatoren zum Einsatz kommen. Diese sind den ebenfalls möglichen Umsetzungen unter Einsatz stöchiometrischer Mengen chiraler Auxiliare aus wirtschaftlicher Sicht üblicherweise deutlich überlegen. Das Ausmaß der asymmetrischen Induktion wird bei den bevorzugt durchzuführenden enantioselektiv katalysierten Reaktionen durch die Leistungsfähigkeit des chiralen Katalysatorsystems bestimmt und ist somit für einen jeweiligen Katalysator und die jeweils gewählten Reaktionsbedingungen festgelegt. Dadurch ist auch das Verhältnis, bei dem in einem im technischen Maßstab durchgeführten Verfahren optisch aktives Menthol neben dem als Resultat einer unvollständigen asymmetrischen Induktion gebildeten racemischen Menthol gebildet wird, festgelegt und kann nur durch aufwändigen Austausch des Katalysatorsystems oder durch Änderung der Reaktionsbedingungen angepasst werden. Beides ist insbesondere bei Umsetzungen im technischen Maßstab nur mit erheblichem Aufwand zu bewerkstelligen.

Vor diesem Hintergrund bestand die Aufgabe der vorliegenden Erfindung darin, ein Verfahren bereitzustellen, das es ermöglicht, ohne Austausch des enantioselektiven Katalysatorsystems bzw. ohne Notwendigkeit zur Änderung der Reaktionsbedingungen, optisch aktives Menthol, bevorzugt L-Menthol und racemisches Menthol in veränderlichen, bedarfsorientierten Mengen herzustellen, ohne dass dabei eines der Produkte im Überschuss erhalten wird. Das Verfahren soll für Umsetzungen im technischen Maßstab geeignet sein und den Einsatz gut verfügbarer, wohlfeiler Ausgangsstoffe ermöglichen. Das Verfahren soll darüber hinaus bei geringer Gesamtstufenzahl eine hohe Gesamtausbeute liefern. Darüber hinaus soll im Rahmen der chirogenen Reaktionstufe ein wohlfeiles und gut verfügbares enantioselektives Katalysatorsystem zum Einsatz kommen.

Die Aufgabe wurde erfindungsgemäß gelöst durch die Bereitstellung eines Verfahrens zur Herstellung von Menthol, umfassend die Verfahrensschritte
a.1) katalytische Hydrierung von Neral und/oder Geranial zu Citronellal,
b.1) Cyclisierung von Citronellal zu Isopulegol in Gegenwart eines sauren Katalysators,
c.1) Aufreinigung von Isopulegol durch Kristallisation und
d.1) katalytische Hydrierung von Isopulegol zu Menthol.

Als Ausgangsstoffe zur Durchführung des erfindungsgemäßen Verfahrens dienen die α,β-ungesättigten Aldehyde Neral der Formel (II) 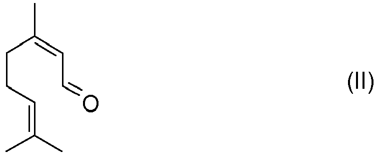 und/oder Geranial der Formel (III) 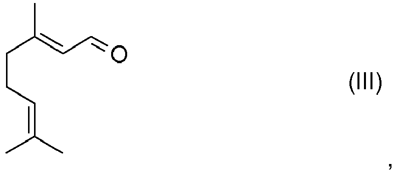 jeweils in reiner Form oder bevorzugt in Form von Gemischen untereinander. Bevorzugte Gemische von Geranial und Neral sind solche, die über 90 Gew.%, bevorzugt 95 bis 99,5 Gew.-% und besonders bevorzugt zu 96 und am meisten bevorzugt 97 bis 99,5 Gew.-% Geranial und Neral enthalten, wobei noch geringe Mengen von Nebenkomponenten, beispielsweise Wasser oder Lösemittelreste enthalten sein können und wobei sich die Angaben in Gew.-% hier wie im Rahmen der gesamten Offenbarung auf die Gesamtmenge der jeweiligen Gemische beziehen.

Die Zusammensetzung der erfindungsgemäß einsetzbaren Neral- und Geranialhaltigen Stoffgemische kann in weiten Grenzen variiert werden. Erfingsgemäß bevorzugt sind solche Gemische, die etwa 0,1 bis etwa 20 Gew.-%, bevorzugt etwa 0,1 bis etwa 10 Gew.-%, besonders bevorzugt etwa 0,5 bis etwa 5 Gew.-% und ganz besonders bevorzugt etwa 0,5 bis 3 Gew.-% Geranial und etwa 80 Gew.-% bis etwa 99,9 Gew.-%, bevorzugt etwa 90 Gew.-% bis etwa 99,9 Gew.-%, besonders bevorzugt etwa 95 Gew.-% bis etwa 99,5 Gew.-% und ganz besonders bevorzugt etwa 96 bis 99 Gew.-% Neral enthalten, wobei sich alle Angabe in Gew.-% auf die Gesamtmenge der jeweiligen Stoffgemische beziehen.

Als Ausgangsstoff zur Durchführung des erfindungsgemäßen Verfahrens können auch Geranial- und Neral-haltige Gemische eingesetzt werden, die zu einem größeren Anteil als vorstehend beschrieben Geranial enthalten wie beispielsweise das als Citral bekannte Gemisch von Geranial und Neral. Citral besteht im Gleichgewicht zu etwa 50 Gew.-% aus Geranial und zu etwa 50 Gew.-% aus Neral und ist in technischen Maßstab gut verfügbar, beispielsweise durch thermische Spaltung von 3-Methyl-2-buten-1-al-diprenylacetal unter Abspaltung von Prenol zu cis/trans-Prenyl-(3-methyl-butadienyl)-ether, Claisen-Umlagerung desselben zu 2,4,4-Trimethyl-3-formyl-1,5-hexadien und anschließende Cope-Umlagerung desselben, wie beispielsweise in der EP 0 992 477 beschrieben, auf die hiermit vollumfänglich Bezug genommen wird und die somit Bestandteil der vorliegenden Offenbarung ist, sowie den darin zitierten Stellen.

### Verfahrensschritt 0): Destillative Trennung von Neral- und geranial-haltigen Stoffgemischen

Bei Einsatz von Geranial- und Neral-haltigen Gemischen, beispielsweise bei Einsatz des vorstehend beschriebenen Citrals, hat es sich als vorteilhaft erwiesen, dass sich die beiden isomeren Verbindungen, die sich lediglich in der Konfiguration der α,β-ständigen Doppelbindung unterscheiden durch destillative Verfahren trennen, aufreinigen bzw. anreichern lassen. Dies ermöglicht, beispielsweise ausgehend von dem wie vorstehend beschriebenen Citral, Gemische von Neral und Geranial in nahezu beliebigem Mischungsverhältnis bereitzustellen. Auf diese Weise ist, ausgehend von Neral- und Geranial-haltigen Gemischen, bevorzugt ausgehend von wie vorstehend beschriebenem Citral angereichertes oder reines Geranial oder Neral, bevorzugt Neral, zugänglich.

Im Rahmen einer bevorzugten Ausführungsform umfasst das erfindungsgemäße Verfahren zur Herstellung von Menthol dementsprechend zusätzlich als optionalen Verfahrensschritt 0) die destillative Trennung von Geranial- und Neral-haltigen Gemischen unter Erhalt von angereichertem oder reinem Geranial oder Neral, bevorzugt Neral.

Es hat sich darüber hinaus gezeigt, dass sich die destillative Trennung von Geranial- und Neral-haltigen Gemischen besonders vorteilhaft mittels einer Trennwandkolonne oder einer Verschaltung thermisch gekoppelter Kolonnen durchführen lässt. Auf diese Weise ist insbesondere Neral in reiner oder angereicherter Form durch destillative Trennung von Stoffgemischen enthaltend Geranial und Neral zugänglich.

Vorteilhaft führt man die genannte destillative Trennung von Geranial- und Neralhaltigen Gemischen kontinuierlich durch. Im Rahmen einer bevorzugten Ausführungsform führt man zur destillativen Trennung von Geranial- und Neral-haltigen Gemischen ein kontinuierliches Verfahren zur Herstellung von Neral der Formel (II) in reiner oder angereicherter Form durch destillative Abtrennung von Neral aus Stoffgemischen enthaltend Neral und Geranial der Formel (III) ein, wobei man die destillative Abtrennung in einer Trennwandkolonne oder in einer Zusammenschaltung von zwei Destillationskolonnen in Form einer thermischen Kopplung mit 80 bis 200 theoretischen Trennstufen und einer oder mehrerer Seitenabzugsstellen bei einem absoluten Betriebsdruck von 5 bis 200 mbar durchführt.

Als Einsatzstoffe zur Durchführung dieser bevorzugten Ausführungsform des destillativen Trennverfahrens von Neral- und Geranial-haltigen Gemischen eignen sich Stoffgemische die Neral und Geranial enthalten, bevorzugt solche, die zum überwiegenden Teil aus den Doppelbindungsisomeren Neral und Geranial bestehen. Bevorzugt sind darunter solche Stoffgemische, die zu mindestens 90 Gew.-% bis 100 Gew.-%, besonders bevorzugt zu mindestens 95 bis 98 Gew.-% (jeweils bezogen auf die Gesamtmenge des jeweiligen Stoffgemisches) Geranial und Neral enthalten bzw. zu den genannten Anteilen aus denselben bestehen und daneben in geringem Ausmaß, d.h. zu einem Anteil von bis zu 10 Gew.-%, bevorzugt bis zu 5 Gew.-% (jeweils bezogen auf die Gesamtmenge des jeweiligen Stoffgemisches) noch weitere Komponenten wie beispielsweise Isomere, Nebenprodukte oder Verunreinigungen enthalten können. Ein bevorzugter Einsatzstoff ist synthetisch hergestelltes Citral, insbesondere solches, dass durch thermische Spaltung von 3-Methyl-2-buten-1-al-diprenylacetal unter Abspaltung von Prenol zu cis/trans-Prenyl-(3-methyl-butadienyl)-ether, Claisen-Umlagerung desselben zu 2,4,4-Trimethyl-3-formyl-1,5-hexadien und anschließende Cope-Umlagerung desselben, wie beispielsweise in der EP 0 992 477 beschrieben, erhalten wurde. Dieses enthält typischerweise etwa 45 bis etwa 55 Gew.-% Neral neben etwa 55 bis etwa 45 Gew.-% und etwa 1 bis 5 Gew.-% weiterer Verbindungen bzw. Verunreinigungen.

Im Rahmen einer besonderen Ausführungsform umfasst das erfindungsgemäße Verfahren als zusätzlichen vorgelagerten Verfahrenschritt das vorstehend genannte Herstellverfahren von Citral ausgehend von 3-Methyl-2-buten-1-al-diprenylacetal.

Im Rahmen einer bevorzugten Ausführungsform des erfindungsgemäß einsetzbaren Trennverfahrens von Geranial- und Neral-haltigen Gemischen setzt man ein Stoffgemisch ein, das zu 30 bis 70 Gew.-%, bevorzugt zu 40 bis 60 Gew.-% aus Neral, zu 70 bis 30 Gew.-%, bevorzugt zu 60 bis 40 Gew.-% aus Geranial und zu 0 bis 5 Gew.-% aus weiteren Komponenten besteht, wobei sich die Prozentangaben zu 100 Gew.-% ergänzen.

Die erfindungsgemäß bevorzugt durchzuführende destillative Abtrennung wird üblicherweise so durchgeführt, dass man das eingesetzte Neral und Geranial enthaltende Stoffgemisch in jeweils eine oder mehrere Leichtsieder-, Mittelsieder- und Schwersiederfraktion bzw. -fraktionen auftrennt und Neral in reiner oder angereicherter Form als Mittelsiederfraktion an der Seitenabzugsstelle der eingesetzten Trennwandkolonne oder der Zusammenschaltung von zwei Destillationskolonnen in Form einer thermischen Kopplung in flüssiger oder gasförmiger Form entnommen wird.

Bei dem im Rahmen des optionalen Verfahrensschritts 0) zur Trennung von Neral- und Geranial-haltigen Stoffgemischen bevorzugt einsetzbaren destillativen Trennverfahren handelt es sich demnach auch um ein kontinuierliches Verfahren zur Isolierung von Neral, vorzugsweise um ein kontinuierliches Verfahren zur Isolierung von Neral in reiner oder angereicherter Form durch destillative Abtrennung von Neral aus Stoffgemischen enthaltend Neral und Geranial, wobei man die destillative Abtrennung in einer Trennwandkolonne oder in einer Zusammenschaltung von zwei Destillationskolonnen in Form einer thermischen Kopplung mit 80 bis 200 theoretischen Trennstufen und einer oder mehrerer Seitenabzugsstellen bei einem absoluten Betriebsdruck, d.h. bei einem absoluten Druck in der Trennwandkolonne oder der Zusammenschaltung von zwei Destillationskolonnen in Form einer thermischen Kopplung von 5 bis 200 mbar durchführt.

Die zur destillativen Trennung im Rahmen des optionalen Verfahrensschritts 0) zur Trennung von Neral- und Geranial-haltigen Stoffgemischen bevorzugt einzusetzende Trennwandkolonne bzw. die Zusammenschaltung von zwei Destillationskolonnen in Form einer thermischen Kopplung weist bzw. weisen 80 bis 200, bevorzugt 100 bis 180 theoretische Trennstufen und eine oder mehrere, bevorzugt 1 bis 3, besonders bevorzugt 1 oder 2 Seitenabzugsstellen auf. Bevorzugt setzt man eine wie vorstehend beschriebene Trennwandkolonne ein.

Das im Rahmen des erfindungsgemäßen Verfahrens bevorzugt durchzuführende Verfahren zur Herstellung von reinem oder angereichertem Neral wird bei einem absoluten Betriebsdruck in der Trennwandkolonne bzw. in der Zusammenschaltung von zwei Destillationskolonnen in Form einer thermischen Kopplung von 5 bis 200 mbar, bevorzugt von 5 bis 100 mbar, besonders bevorzugt von 5 bis 70 mbar und ganz besonders bevorzugt von von 10 bis 50 mbar und insbesondere bevorzugt von 10 bis 40 mbar durchgeführt. Bevorzugt betreibt man dabei die Trennwandkolonne oder die Zusammenschaltung von zwei Destillationskolonnen in Form einer thermischen Kopplung so, dass der absolute Kopfdruck 10 bis 50 mbar, bevorzugt 10 bis 40 mbar beträgt. Ebenfalls bevorzugt betreibt man dabei die Trennwandkolonne oder die Zusammenschaltung von zwei Destillationskolonnen in Form einer thermischen Kopplung so, dass der absolute Sumpfdruck 5 bis 200 mbar, bevorzugt 10 bis 100 und besonders bevorzugt 20 bis 50 mbar beträgt.

Das Rücklaufverhältnis kann bei der Durchführung des Verfahrens zur destillativen Trennung von Geranial und Neral in breiten Grenzen variiert werden und liegt üblicherweise bei etwa 5 zu 1 bis etwa 2000 zu 1, bevorzugt etwa 20 zu 1 bis 1000 zu 1. Vorteilhaft ist auch eine Dephlegmator-Fahrweise, d.h. es wird im Kopfkondensator der Kolonne nur der Rücklauf kondensiert und wieder der Kolonne zugeführt. In einem solchen energetisch günstigen Fall der Teilkondensation fällt das auszuschleusende Kopfprodukt ausschließlich im Nachkühler an, der bei niedrigerer Temperatur betrieben werden kann.

Unter dem Begriff "Neral in angereicherter Form" sind Neral-haltige Stoffgemische zu verstehen, die einen höheren Gehalt an Neral aufweisen als das jeweils erfindungsgemäß eingesetzte Neral oder Geranial enthaltende Stoffgemisch. Bevorzugt ist unter dem Begriff Neral in angereicherter Form solches Neral zu verstehen, das eine Reinheit, d.h. einen Neral-Gehalt von 80 bis 95 Gew.-%, bevorzugt von 85 bis 95 Gew.-% und ganz besonders bevorzugt von 90 bis 95 Gew.-% aufweist. Das erfindungsgemäße Verfahren ermöglicht auch die Herstellung von Neral (cis-Citral) in reiner Form. Unter dem Begriff "Neral in reiner Form" ist Neral mit einem Gehalt von größer oder gleich 95, 96 oder 97 Gew.-%, bevorzugt größer oder gleich 98 Gew.-% und besonders bevorzugt 98 bis 99,5 Gew.-% zu verstehen. Insbesondere bevorzugt ist unter dem Begriff "Neral in reiner Form" solches Neral zu verstehen, das einen Geranialgehalt von bis zu 1 Gew.-% bevorzugt von 0,05 bis 0,5 Gew.-% und besonders bevorzugt von 0,1 bis 0,3 Gew.-% aufweist. Ebenfalls bevorzugt weist das erfindungsgemäß zugängliche Neral in reiner Form einen Gehalt an iso-Citralen der Formeln (IV), (V) und (VI) 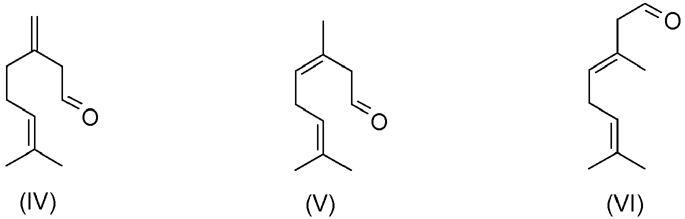 von bis zu 2 Gew.-%, bevorzugt von 0,1 bis 1 Gew.-% auf, wobei sich alle Angaben im Rahmen der vorliegenden Erfindung auf die Gesamtmenge der jeweiligen Stoffgemische beziehen.

Der Zulauf, d.h. das einzusetzende Stoffgemisch kann flüssig oder gasförmig in die Trennwandkolonne bzw. die Zusammenschaltung von zwei Destillationskolonnen in Form einer thermischen Kopplung, bevorzugt in die Trennwandkolonne geführt und dort in eine Kopf- und Sumpffraktion sowie einen oder mehrere, bevorzugt in mehrere Seitenabläufe wie vorstehend beschrieben aufgetrennt werden. In einem Seitenablauf fällt das Wertprodukt Neral in der gewünschten Reinheit an. In einer besonderen Ausführungsform ist dem Kopfkondensator der Kolonne ein Nachkondensator nachgeschaltet, der mit Kühlflüssigkeit (beispielsweise Sole) gekühlt ist und in dem noch eine Neral-arme Leichtsiederfraktion anfällt.

Für die kontinuierliche destillative Zerlegung von Mehrstoffgemischen sind nach dem Stand der Technik verschiedene Verfahrensvarianten gebräuchlich. Im einfachsten Fall wird das Zulaufgemisch in zwei Fraktionen, eine leichtsiedende Kopffraktion und eine schwersiedende Sumpffraktion, zerlegt. Bei der Auftrennung von Zulaufgemischen in mehr als zwei Fraktionen müssen nach dieser Verfahrensvariante mehrere Destillationskolonnen eingesetzt werden. Um den apparativen Aufwand zu begrenzen, setzt man bei der Auftrennung von Vielstoffgemischen nach Möglichkeit Kolonnen mit flüssigen oder dampfförmigen Seitenabzügen ein. Die Anwendungsmöglichkeit von Destillationskolonnen mit Seitenabzügen ist jedoch dadurch stark eingeschränkt, dass die an den Seitenabzugsstellen entnommenen Produkte nie völlig rein sind. Bei Seitenentnahmen im Verstärkungsteil, die üblicherweise in flüssiger Form erfolgen, enthält das Seitenprodukt noch Anteile an leichtsiedenden Komponenten, die über Kopf abgetrennt werden sollen. Entsprechendes gilt für Seitenentnahmen im Abtriebsteil, die meist dampfförmig erfolgen, bei denen das Seitenprodukt noch Hochsiederanteile aufweist. Die Verwendung von konventionellen Seitenabzugskolonnen ist daher auf Fälle begrenzt, in denen verunreinigte Seitenprodukte zulässig sind.

Eine Abhilfemöglichkeit bieten Trennwandkolonnen. Dieser Kolonnentyp ist beispielsweise beschrieben in US 2,471,134; US 4,230,533; EP 0 122 367; EP 0 126 288; EP 0 133 510; Chem. Eng. Technol. 10 (1987) 92 - 98; Chem.-Ing.-Tech. 61 (1989) Nr.1, 16 - 25; Gas Separation and Purification 4 (1990) 109 - 114; Process Engineering 2 (1993) 33 - 34; Trans IChemE 72 (1994) Part A 639 - 644 und Chemical Engineering 7 (1997) 72 - 76.

Bei dieser Bauart ist es möglich, Seitenprodukte ebenfalls in reiner Form zu entnehmen. Im mittleren Bereich oberhalb und unterhalb der Zulaufstelle und der Seitenentnahme ist eine Trennwand angebracht, die den Zulaufteil gegenüber dem Entnahmeteil abdichtet und in diesem Kolonnenteil eine Quervermischung von Flüssigkeits- und Brüdenströmen unterbindet. Hierdurch verringert sich bei der Auftrennung von Vielstoffgemischen die Zahl der insgesamt benötigten Destillationskolonnen. Da dieser Kolonnentyp eine apparative Vereinfachung von thermisch gekoppelten Destillationskolonnen darstellt, weist er darüber hinaus auch einen besonders niedrigen Energieverbrauch auf. Eine Beschreibung von thermisch gekoppelten Destillationskolonnen, die in verschiedener apparativer Ausgestaltung ausgeführt sein können, findet sich ebenfalls in den oben genannten Stellen in der Fachliteratur. Trennwandkolonnen und thermisch gekoppelte Kolonnen bieten gegenüber der Anordnung von konventionellen Destillationskolonnen sowohl hinsichtlich des Energiebedarfs als auch der Investitionskosten Vorteile und werden daher zunehmend industriell eingesetzt.

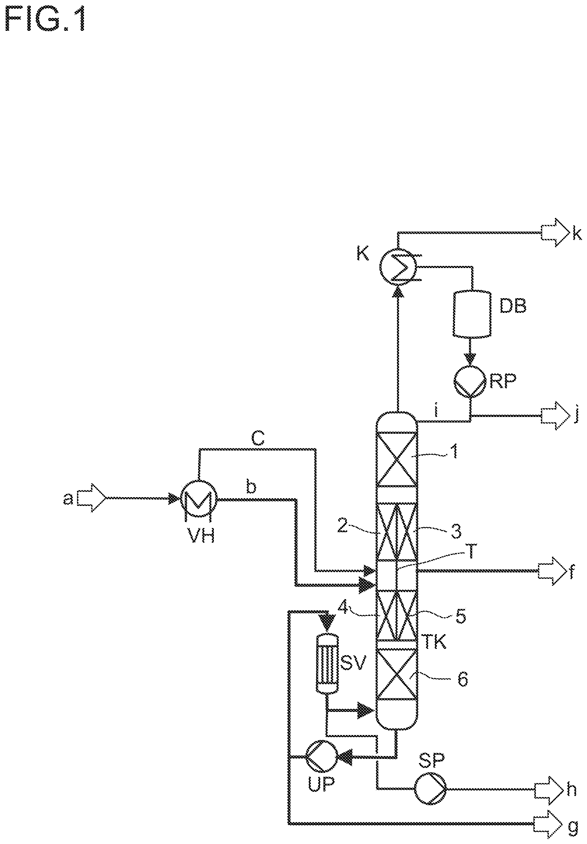

Figur 1 zeigt schematisch eine bevorzugte Ausführungsform der erfindungsgemäß bevorzugten, optional durchzuführenden Auftrennung des einzusetzenden Neral und Geranial enthaltenden Stoffgemisches in eine Neral-arme Kopffraktion (j), eine Neralreiche Seitenfraktion (f) und eine Neral-arme Sumpffraktion (g). Der Neral- und Geranial-haltige Zulauf zur Trennwandkolonne kann flüssig (b) gasförmig (c), bzw. gasförmig und flüssig erfolgen.

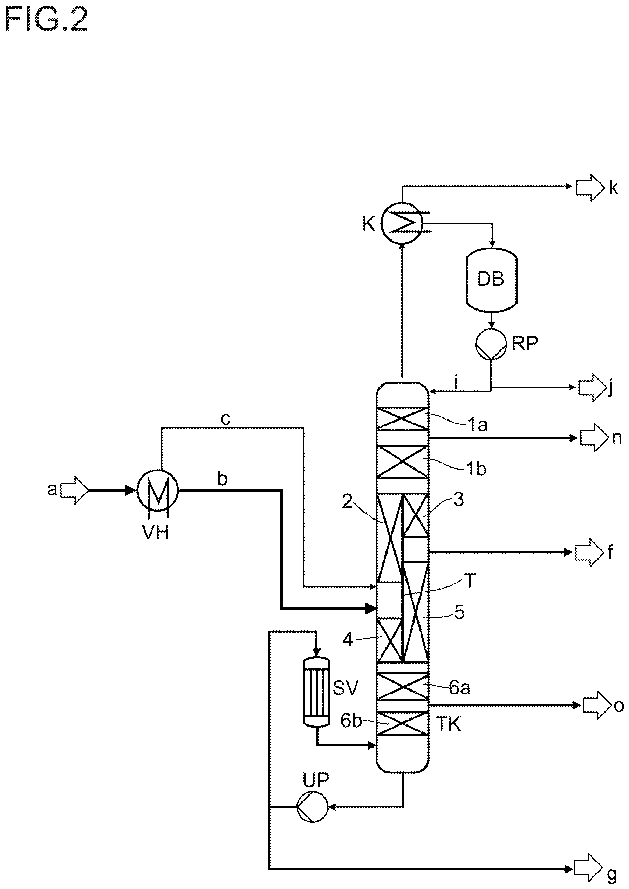

Figur 2 zeigt schematisch eine besonders bevorzugte Ausführungsform des erfindungsgemäßen Verfahrens zur Herstellung von Neral in reiner oder angereicherter Form, bei der zusätzlich zu den unter Figur 1 genannten Merkmalen einschließlich des Seitenabzugs (f) die Seitenabzugsstellen (n) und (o) vorgesehen sind.

Das erfindungsgemäß bevorzugt durchzuführende Verfahren zur destillativen Trennung von Geranial- und Neral-haltigen Stoffgemischen wird kontinuierlich durchgeführt. Demzufolge werden die als Ausgangsstoff einzusetzenden Neral und Geranial enthaltenden Stoffgemische der Trennwandkolonne oder der Zusammenschaltung von zwei Destillationskolonnen in Form einer thermischen Kopplung kontinuierlich zugeführt und die erfindungsgemäß erhaltenen Produkte (Fraktionen) bzw. Nebenprodukte kontinuierlich ausgetragen.

Der Kolonne wird üblicherweise ein weiterer Kondensator nachgeschaltet, dessen Arbeitstemperatur 10 bis 40 K, bevorzugt 20 bis 30 K unter der Arbeitstemperatur des Kopfkondensators der Trennwandkolonne liegt. Mit dessen Hilfe kann ein Großteil der noch im Kopfstrom (k) enthaltenen Leichtsieder niedergeschlagen werden.

Trennwandkolonnen können auch durch jeweils zwei thermisch gekoppelte Kolonnen ersetzt werden. Dies ist vor allem dann günstig, wenn die Kolonnen schon vorhanden sind oder die Kolonnen bei verschiedenen Drücken betrieben werden sollen. Bei thermisch gekoppelten Kolonnen kann es von Vorteil sein, den Sumpfstrom der ersten Kolonne in einem zusätzlichen Verdampfer teilweise oder vollständig zu verdampfen und danach der zweiten Kolonne zuzuführen. Diese Vorverdampfung bietet sich insbesondere dann an, wenn der Sumpfstrom der ersten Kolonne größere Mengen an Mittelsieder enthält. In diesem Fall kann die Vorverdampfung auf einem niedrigeren Temperaturniveau erfolgen und der Verdampfer der zweiten Kolonne entlastet werden. Weiterhin kann durch diese Maßnahme der Abtriebsteil der zweiten Kolonne wesentlich entlastet werden. Der vorverdampfte Strom kann dabei der zweiten Kolonne zweiphasig oder in Form von zwei separaten Strömen zugeführt werden.

Darüber hinaus kann es sowohl bei Trennwandkolonnen als auch bei thermisch gekoppelten Kolonnen von Vorteil sein, den Zulaufstrom einer Vorverdampfung zu unterziehen und anschließend zweiphasig oder in Form von zwei Strömen der Kolonne zuzuführen. Diese Vorverdampfung bietet sich besonders dann an, wenn der Zulaufstrom größere Mengen an Leichtsiedern enthält. Durch die Vorverdampfung kann der Abtriebsteil der Kolonne wesentlich entlastet werden.

Trennwandkolonnen und thermisch gekoppelte Kolonnen können sowohl als Packungskolonnen mit Füllkörpern oder geordneten Packungen oder als Bodenkolonnen ausgeführt werden. Bei dem erfindungsgemäßen Verfahren zur Herstellung von Neral in reiner oder angereicherter Form setzt man bevorzugt Packungskolonnen ein. Dabei sind geordnete Blech- oder Gewebepackungen mit einer spezifischen Oberfläche von etwa 100 bis 750 m²/m³, bevorzugt etwa 350 bis 500 m²/m³ besonders geeignet.

Falls, wie im Fall der vorliegenden Erfindung, besonders hohe Anforderungen an die Reinheiten der Produkte gestellt werden, ist es günstig, die Trennwand mit einer thermischen Isolierung auszustatten. Eine Beschreibung der verschiedenen Möglichkeiten der thermischen Isolierung der Trennwand findet sich in EP-A 0 640 367. Eine doppelwandige Ausführung mit einem zwischenliegenden engen Gasraum ist besonders günstig.

Für die Regelung von Trennwandkolonnen und thermisch gekoppelten Kolonne wurden verschiedene Regelungsstrategien beschrieben. Beschreibungen finden sich in US 4,230,533; DE 35 22 234 ; EP 0 780 147; Process Engineering 2 (1993) 33 - 34 und Ind. Eng. Chem. Res. 34 (1995), 2094 - 2103.

Bei der Trennung von Mehrstoffgemischen in eine Leichtsieder-, Mittelsieder- und Hochsiederfraktion existieren üblicherweise Spezifikationen über den maximal zulässigen Anteil an Leichtsiedern und Hochsiedern in der Mittelsiederfraktion. Hierbei werden entweder einzelne für das Trennproblem kritische Komponenten, so genannte Schlüsselkomponenten, oder die Summe von mehreren Schlüsselkomponenten spezifiziert. Diese Schlüsselkomponenten sind im Rahmen dieses optionalen Verfahrensschritts der vorliegenden Erfindung Geranial als hochsiedende Nebenkomponente und iso-Citral bzw. ein Gemisch von isomeren iso-Citralen als tiefsiedende Nebenkomponente.

Die Einhaltung der Spezifikation für die Hochsieder in der Mittelsiederfraktion kann beispielsweise über das Aufteilungsverhältnis der Flüssigkeit am oberen Ende der Trennwand geregelt werden. Dabei wird das Aufteilungsverhältnis der Flüssigkeit am oberen Ende der Trennwand vorzugsweise so eingestellt, dass die Konzentration der Schlüsselkomponenten für die Hochsiederfraktion in der Flüssigkeit am oberen Ende der Trennwand 10 bis 80%, bevorzugt 30 bis 50%, des Wertes ausmacht, der im Seitenabzugsprodukt erzielt werden soll. Die Flüssigkeitsaufteilung wird vorzugsweise dahingehend eingestellt, dass bei höheren Gehalten an Schlüsselkomponenten der Hochsiederfraktion mehr und bei niedrigeren Gehalten an Schlüsselkomponenten der Hochsiederfraktion weniger Flüssigkeit auf den Zulaufteil geleitet wird.

Entsprechend kann die Spezifikation für die Leichtsieder in der Mittelsiederfraktion durch die Heizleistung geregelt werden. Hierbei wird beispielsweise die Heizleistung im Verdampfer so eingestellt, dass die Konzentration an Schlüsselkomponenten der Leichtsiederfraktion in der Flüssigkeit am unteren Ende der Trennwand 10 bis 80, bevorzugt 30 bis 50% des Wertes ausmacht, der im Seitenabzugsprodukt erzielt werden soll. Die Heizleistung wird vorzugsweise dahingehend eingestellt, dass bei höherem Gehalt an Schlüsselkomponenten der Leichtsiederfraktion die Heizleistung erhöht und bei niedrigerem Gehalt an Schlüsselkomponenten der Leichtsiederfraktion die Heizleistung verringert wird.

Zur Kompensation von Störungen der Zulaufmenge oder der Zulaufkonzentration erwies es sich zudem als vorteilhaft, durch einen entsprechenden Regelmechanismus (z.B. durch Regelvorschriften im Prozessleitsystem) sicherzustellen, dass die Mengenströme der Flüssigkeiten, die auf die Kolonnenteile (2), d.h. den Verstärkungsteil des Zulaufteils, und (5), d.h. den Abtriebsteil des Entnahmeteils, nicht unter 30 % ihres Normalwertes sinken können.

Zur Entnahme und Aufteilung der Flüssigkeiten am oberen Ende der Trennwand und an der bzw. den Seitenentnahmestelle(n) eignen sich sowohl innen liegende als auch außerhalb der Kolonne angeordnete Auffangräume für die Flüssigkeit, welche die Funktion einer Pumpenvorlage übernehmen oder für eine ausreichend hohe statische Flüssigkeitshöhe sorgen, die eine durch Stellorgane, beispielsweise Ventile, geregelte Flüssigkeitsweiterleitung ermöglichen. Bei der Verwendung von gepackten Kolonnen wird die Flüssigkeit zunächst in Sammlern gefasst und von dort aus in einen innenliegenden oder außenliegenden Auffangraum geleitet.

Anstelle einer Trennwandkolonne - die bei einem Neubau hinsichtlich der Investitionskosten zu bevorzugen ist - ist es auch möglich, zwei Destillationskolonnen nach Art einer thermischen Kopplung so zu verschalten, dass sie hinsichtlich des Energiebedarfs einer Trennwandkolonne entsprechen. Sie können bei Verfügbarkeit von bestehenden Kolonnen eine sinnvolle Alternative zu Trennwandkolonnen sein. Je nach der Trennstufenzahl der vorhandenen Kolonnen können die geeigneten Formen der Zusammenschaltung ausgewählt werden.

Setzt man im Rahmen dieser Ausführungsform des gewünschtenfalls durchzuführenden Verfahrensschritts 0) zur Trennung von Geranial- und Neral-haltigen Stoffgemischen zwei Destillationskolonnen in einer Zusammenschaltung in Form einer thermischen Kopplung ein, hat es sich als vorteilhaft erwiesen, beide auf diese Weise thermisch gekoppelten Destillationskolonnen jeweils mit einem eigenen Verdampfer und Kondensator auszustatten. Daneben können die beiden thermisch gekoppelten Kolonnen bei verschiedenen Drücken betrieben werden und in den Verbindungsströmen zwischen den beiden Kolonnen nur Flüssigkeiten gefördert werden. Im Rahmen einer bevorzugten Ausführungsform wird der Sumpfstrom der ersten Kolonne in einem zusätzlichen Verdampfer teilweise oder vollständig verdampft und anschließend der zweiten Kolonne zweiphasig oder in Form eines gasförmigen und eines flüssigen Stromes zugeführt wird.

Im Rahmen einer besonders bevorzugten Ausführungsform führt man das erfindungsgemäß bevorzugte Trennverfahren gemäß dem optionalen Verfahrtensschritt 0) in einer Anlage durch, wie sie schematisch in Figur 1 gezeigt ist. Die bevorzugte Ausführungsform zeichnet sich dadurch aus, dass man eine Trennwandkolonne (TK) einsetzt, die eine Trennwand (T) in Kolonnenlängsrichtung unter Ausbildung eines oberen gemeinsamen Kolonnenbereichs (1), eines unteren gemeinsamen Kolonnenbereichs (6), eines Zulaufteils (2, 4) mit Verstärkungsteil (2) und Abtriebsteil (4) sowie eines Entnahmeteils (3, 5) mit Abtriebsteil (3) und Verstärkungsteil (5) aufweist.

Das als Einsatzstoff dienende Neral und Geranial enthaltende Stoffgemisch (a) wird erfindungsgemäß vorzugsweise in den mittleren Bereich des Zulaufteils (2, 4) zugeführt, das Neral in reiner oder angereicherter Form als Seitenabzug (f) aus dem mittleren Bereich des Entnahmeteils (3, 5) gewonnen und eine oder mehrere Leichtsiederfraktionen aus dem oberen gemeinsamen Kolonnenbereich (1) und eine oder mehrere Hochsiederfraktionen aus dem unteren gemeinsamen Kolonnenbereich (6) abgeführt.

Der Zulaufstrom (a) kann über einen Vorheizer (VH) als flüssiger (b), gasförmiger (c) oder teilweise flüssig und gasförmiger Strom in die Kolonne (TK) eingeleitet werden. Der Kopfstrom der Kolonne wird im Kondensator (K) ganz oder teilweise kondensiert. Bei einer teilweisen Kondensation (Dephlegmator-Betrieb) enthält der Abgasstrom (k) des Kopfkondensators (K) üblicherweise noch merkliche Mengen an kondensierbaren Leichtsiedern, die dann in einem bei niedriger Temperatur betriebenen Nachkondensator niedergeschlagen werden können.

Das im Kondensator (K) niedergeschlagene Kopfprodukt kann in dem Destillatbehälter (DB) gepuffert und über die Rücklaufpumpe (RP) als Kolonnenrücklauf (i) der Kolonne wieder zugeführt werden. Bei Bedarf lässt sich hieraus auch eine Destillatfraktion (j) gewinnen. Bei Intergration des Kondensators in den Kolonnenkopf kann auf den Destillatbehälter (DB) und die Rücklaufpumpe verzichtet (RP) werden.

Der Sumpfstrom wird vorteilhaft über die Umlaufpumpe (UP) dem Sumpfverdampfer (SV) zugeführt, der bevorzugt als Fallfilmverdampfer ausgeführt ist. Diesem Umpumpstrom kann auch der Sumpfaustrag (g) der Kolonne (TK) entnommen werden.

Das Wertprodukt Neral in reiner oder angereicherter Form wird bevorzugt als flüssiger Seitenabzug, Strom (f), aus dem Entnahmeteil der Trennwandkolonne (TK) abgezogen. Es ist auch möglich, bei Bedarf den Wertprodukt-Strom (f) als gasförmigen Abzug zu entnehmen, dann wird aber üblicherweise ein weiterer Kondensator benötigt.

Der obere gemeinsame Teilbereich (1) der Kolonne weist üblicherweise 5 bis 50%, der Verstärkungsteil (2) des Zulaufteils der Kolonne 5 bis 50%, der Abtriebsteil (4) des Zulaufteils der Kolonne 2 bis 50%, der Abtriebsteil (2) des Entnahmeteils der Kolonne 5 bis 50%, der Verstärkungsteil (5) des Entnahmeteils 2 bis 50%, und der gemeinsame untere Teil (6) der Kolonne 5 bis 50% der Gesamtzahl der theoretischen Trennstufen der Kolonne auf, wobei sich die gewählten Prozentangaben zu 100% addieren.

Bevorzugt weist der obere gemeinsame Teilbereich (1) der Kolonne 10 bis 25%, der Verstärkungsteil (2) des Zulaufteils der Kolonne 15 bis 30%, der Abtriebsteil (4) des Zulaufteils der Kolonne 5 bis 20%, der Abtriebsteil (3) des Entnahmeteils der Kolonne 15 bis 30%, der Verstärkungsteil (5) des Entnahmeteils 5 bis 20% und der gemeinsame untere Teil (6) der Kolonne 10 bis 25% der Gesamtzahl der theoretischen Trennstufen der Kolonne auf, wobei sich die gewählten Prozentangaben zu 100% addieren.

Die Summe der Zahl der theoretischen Trennstufen der Teilbereiche (2) und (4) im Zulaufteil beträgt bevorzugt 80 bis 110%, besonders bevorzugt 95 bis 105% der Summe der Zahl der Trennstufen der Teilbereiche (3) und (5) im Entnahmeteil.

Vorteilhafterweise sind die Zulaufstelle und die Seitenabzugsstelle hinsichtlich der Lage der theoretischen Trennstufen auf unterschiedlicher Höhe in der Kolonne angeordnet, indem die Zulaufstelle um 1 bis 50, bevorzugt um 30 bis 45 theoretische Trennstufen höher oder niedriger anordnet ist als die Seitenabzugsstelle.

Es hat sich darüber hinaus als vorteilhaft erwiesen, wenn der durch die Trennwand unterteilte Teilbereich der Kolonne bestehend aus den Teilbereichen (2), (3), (4) und (5) oder Teilen davon mit geordneten Packungen oder Füllkörpern (beispielsweise Gewebepackungen wie Montz A3-500, Sulzer BX oder CY) bestückt ist. Weiterhin hat es sich als vorteilhaft erwiesen, wenn die Trennwand in diesen Teilbereichen wärmeisolierend ausgeführt ist.

Der Brüdenstrom am unteren Ende der Trennwand kann durch die Wahl und/oder Dimensionierung der Trenneinbauten und/oder den Einbau druckverlusterzeugender Vorrichtungen, beispielsweise von Blenden, so eingestellt werden, dass das Verhältnis des Brüdenstroms im Zulaufteil zu dem des Entnahmeteils 0,8 bis 1,2, bevorzugt 0,9 bis 1,1 beträgt.

Die aus dem oberen gemeinsamen Teilbereich (1) der Kolonne ablaufende Flüssigkeit wird vorteilhaft in einem in der Kolonne oder außerhalb der Kolonne angeordneten Auffangraum gesammelt und gezielt durch eine Festeinstellung oder Regelung am oberen Ende der Trennwand so aufgeteilt, dass das Verhältnis des Flüssigkeitsstroms zum Zulaufteil zu dem zum Entnahmeteil 0,1 bis 2,0 bei größtenteils flüssigen Zulauf und 1,0 bis 2 bei gasförmigem Zulauf beträgt. Dabei ist der flüssige Zulauf erfindungsgemäß bevorzugt.

Die aus dem oberen gemeinsamen Teilbereich (1) auf den Zulaufteil ablaufende Flüssigkeit kann über eine Pumpe gefördert oder über eine statische Zulaufhöhe von mindestens 1 m mengengeregelt aufgegeben werden, bevorzugt über eine Kaskadenregelung in Verbindung mit der Flüssigkeitsstandregelung des Auffangraums. Die Regelung wird bevorzugt so eingestellt, dass die auf den Zulaufteil aufgegebene Flüssigkeitsmenge nicht unter 30 % des gewünschten Normalwertes sinken kann. Darüber hinaus wird die Aufteilung der aus dem Teilbereich (3) im Entnahmeteil der Kolonne ablaufenden Flüssigkeit auf den Seitenabzug und auf den Teilbereich (5) im Entnahmeteil der Kolonne durch eine Regelung vorteilhaft so eingestellt, dass die auf den Teilbereich (5) aufgegebene Flüssigkeitsmenge nicht unter eine Höhe von 30% des gewünschten Normalwertes sinken kann. Als Normalwert sind dabei vorteilhaft die zwei- bis vierfache Menge, bezogen auf die Zulaufmenge an Geranial/Neral-Gemisch, anzunehmen.

Die im Rahmen des optionalen Verfahrensschritts 0) bevorzugt einzusetzende Trennwandkolonne weist vorzugsweise am oberen und unteren Ende der Trennwand Probenahmemöglichkeiten auf, aus der der Kolonne kontinuierlich oder in zeitlichen Abständen flüssig oder gasförmig Proben entnommen und hinsichtlich ihrer Zusammensetzung, bevorzugt gaschromatographisch, untersucht werden können.

Das Aufteilungsverhältnis der Flüssigkeit am oberen Ende der Trennwand wird vorzugsweise so eingestellt, dass die Konzentration an denjenigen Komponenten der Hochsiederfraktion, für die im Seitenabzug ein bestimmter Grenzwert für die Konzentration erzielt werden soll (speziell Geranial), in der Flüssigkeit am oberen Ende der Trennwand 10 bis 80%, bevorzugt 30 bis 50% des Wertes ausmacht, der im Seitenabzugsprodukt erzielt werden soll. Die Flüssigkeitsaufteilung sollte bevorzugt dahingehend eingestellt werden, dass bei höheren Gehalten an Komponenten der Hochsiederfraktion mehr und bei niedrigeren Gehalten an Komponenten der Hochsiederfraktion weniger Flüssigkeit auf den Zulaufteil geleitet wird.

Die Heizleistung im Verdampfer (SV) stellt man bevorzugt so ein, dass die Konzentration an denjenigen Komponenten der Leichtsiederfraktion, für die im Seitenabzug ein bestimmter Grenzwert für die Konzentration erzielt werden soll (speziell Isocitrale), am unteren Ende der Trennwand 10 bis 80%, bevorzugt 30 bis 50% des Wertes ausmacht, der im Seitenabzugsprodukt erzielt werden soll. Die Heizleistung stellt man vorteilhaft dahingehend ein, dass bei höherem Gehalt an Komponenten der Leichtsiederfraktion die Heizleistung erhöht und bei niedrigerem Gehalt an Komponenten der Leichtsiederfraktion die Heizleistung verringert wird.

Die Destillatentnahme, d.h. die Entnahme der tiefsiedenden Nebenprodukte erfolgt bevorzugt temperaturgeregelt. Als Regeltemperatur verwendet man mit Vorteil eine Messstelle im Teilbereich (1) der Kolonne, die um 3 bis 8, bevorzugt 4 bis 6 theoretische Trennstufen unterhalb des oberen Endes der Kolonne angeordnet ist.

Die Entnahme des Sumpfprodukts erfolgt bevorzugt mengengeregelt, vorzugsweise in Abhängigkeit von der Zulaufmenge.

Die Entnahme des als Seitenprodukt gewonnenen Nerals in reiner oder angereicherter Form erfolgt bevorzugt standgeregelt, wobei als Regelgröße vorzugsweise der Flüssigkeitsstand im Kolonnensumpf verwendet wird.

Der Zulaufstrom (a) wird vorzugsweise teilweise oder vollständig vorverdampft und der Kolonne zweiphasig oder in Form eines gasförmigen und eines flüssigen Stromes zugeführt.

Im Rahmen einer bevorzugten Ausführungsform setzt man eine Trennwandkolonne ein, deren Trennwand nicht in die Kolonne eingeschweißt ist, sondern in Form von lose gesteckten und adäquat abgedichteten Teilsegmenten gestaltet ist.

Die Flüssigkeitsverteilung in den einzelnen Teilbereichen der Kolonne kann bevorzugt gezielt ungleichmäßig eingestellt werden, wobei insbesondere in den Teilbereichen (2) und (5) die Flüssigkeit verstärkt im Wandbereich aufgegeben wird und in den Teilbereichen (3) und (4) die Flüssigkeit reduziert im Wandbereich aufgegeben wird.

Das Verteilungsverhältnis der rücklaufenden Flüssigkeit zwischen Abnahme- und Zulaufseite der Trennwand beträgt vorzugsweise etwa 1 zu 1 bis etwa 3 zu 1, bevorzugt etwa 1 zu 1 bis etwa 1,5 zu 1.

Die Lage der Trennwand in den einzelnen Teilbereichen der Kolonne kann vorteilhaft so angepasst werden, dass die Querschnitte von Zulauf- und Entnahmeteil verschiedene Flächen aufweisen.

Eine besonders bevorzugte Ausführungsform des im Rahmen des erfindungsgemäßen Verfahrens gemäß dem optionalen Verfahrensschritt 0) bevorzugt durchzuführenden Verfahrens zur Herstellung von reinem oder angereichertem Neral zeichnet sich dadurch aus, dass mindestens eine Leichtsiederfraktion als flüssiger oder gasförmiger, bevorzugt als flüssiger Seitenabzug (n) im oberen Abschnitt (1) der Kolonne, bevorzugt 4 bis 10 theoretische Stufen unter dem Kolonnenkopf, gewonnen wird (siehe Figur 2). In diesem Fall ist es zweckmäßig, den oberen Kolonnenabschnitt (1) in zwei Abschnitte ((1a) und (1 b)) aufzuteilen. Zwischen diesen Abschnitten kann durch einen geeigneten Sammler die aus dem Abschnitt (1a) ablaufende Flüssigkeit aufgefangen und wieder auf den unterhalb liegenden Abschnitt (1 b) verteilt werden (siehe Figur 2). Aus dem Sammler kann eine Leichtsieder- arme und Neral-arme Fraktion abgezogen werden, die vor allem isomere Citrale enthält.

Diese durch den zusätzliche Seitenabzug (n) zugängliche iso-Citral-reiche Nebenproduktfraktion kann in geeigneter Weise weiterverwertet werden, beispielsweise kann sie einer durchgreifenden Hydrierung oder einer partiellen Hydrierung zum Tetrahydrogeraniol unterworfen werden, wodurch zu entsorgende Abfall- bzw. Nebenprodukte vermieden werden können.

Im Rahmen einer erfindungsgemäß besonders bevorzugten Ausführungsform betrifft der optional durchzuführende Verfahrensschritt 0) daher ein kontinuierliches Verfahren zur Herstellung von Neral der Formel (I) in reiner oder angereicherter Form durch destillative Abtrennung von Neral aus Stoffgemischen enthaltend Neral und Geranial der Formel (II)
- wobei man die destillative Abtrennung in einer Trennwandkolonne (TK), die eine Trennwand (T) in Kolonnenlängsrichtung unter Ausbildung eines oberen gemeinsamen Kolonnenbereichs (1), eines unteren gemeinsamen Kolonnenbereichs (6), eines Zulaufteils (2, 4) mit Verstärkungsteil (2) und Abtriebsteil (4) sowie eines Entnahmeteils (3, 5) mit Abtriebsteil (3) und Verstärkungsteil (5) aufweist, mit 80 bis 200 theoretischen Trennstufen und mehrerer, bevorzugt 2 bis 4, besonders bevorzugt 2 oder 3 Seitenabzugsstellen bei einem absoluten Betriebsdruck von 5 bis 200 mbar durchführt und
- wobei man Neral in reiner oder angereicherter Form als Seitenabzug (f) aus dem mittleren Bereich des Entnahmeteils (3, 5) gewinnt und
- wobei man eine Leichtsiederfraktion (n) als flüssigen oder gasförmigen, bevorzugt als flüssigen Seitenabzug aus dem oberen gemeinsamen Kolonnenbereich (1) gewinnt.

Eine weitere bevorzugte Ausführungsform des erfindungsgemäßen einsetzbaren Trennverfahrens zeichnet sich dadurch aus, dass mindestens eine Hochsiederfraktion als gasförmiger Seitenabzug (o) im unteren gemeinsamen Teilbereich der Kolonne (6), bevorzugt 1 bis 5 theoretische Stufen über dem Kolonnensumpf, gewonnen wird (siehe Figur 2). Dadurch kann ein besonders hochsiederarmes Geranial-reiches Produkt erhalten werden. In diesem Fall kann es zweckmäßig sein, den unteren Kolonnenabschnitt (6) in zwei Abschnitte (6a und 6b) aufzuteilen. Zwischen diesen Abschnitten kann durch einen geeigneten Sammler die aus dem Abschnitt (6a) ablaufende Flüssigkeit aufgefangen und wieder auf den unterhalb liegenden Abschnitt (6b) verteilt (siehe Figur 2) sowie der Gasstrom für den Seitenabzug entnommen werden.

Als Sumpfverdampfer (SV) für die Trennwandkolonne kann vorteilhaft ein Dünnschichtapparat, beispielsweise ein Fallfilmverdampfer, verwendet werden.

Der Kopfkondensator (K) kann beispielsweise als Plattenapparat ausgeführt und in den Kolonnenmantel integriert sein.

Das durch das dargestellte destillative Trennverfahren gemäß optionalem Verfahrensschritt 0) zugängliche Neral in reiner oder angereicherter Form wird über den Seitenabzug, bzw. im Fall, dass weitere Seitenabzüge vorgesehen sind, über den mittleren Seitenabzug (f) kontinuierlich gewonnen und weist im Rahmen einer bevorzugten Ausführungsform einen Neral-Gehalt von über 98 Gew.-%, bevorzugt von 98,5 bis 99,5 Gew.-%, einen Geranial-Gehalt von weniger als 0,3 Gew.-% und einen Gehalt an sonstigen Isomeren (Citral-Isomere der Formeln (IV), (V) und (VI)) von weniger als 1 Gew.-% (jeweils bezogen auf die Gesamtmenge des erhaltenen Gemischs), gegebenenfalls neben geringen Mengen weiterer Verunreinigungen, auf.

Wird ein wie vorstehend beschriebener oberer Seitenabzug (n) vorgesehen, kann dort ein Nebenproduktgemisch erhalten werden, das üblicherweise einen Neral-Gehalt von weniger als 80 Gew.-%, einen Geranial-Gehalt von weniger als 0,1 Gew.-% und einen Gehalt an sonstigen Isomeren, insbesondere der Citral-Isomere der Formeln (IV),(V) und/oder (VI) von über 20 Gew.-%, bevorzugt von über 30 Gew.-% aufweist. Daneben kann in einem gewünschtenfalls vorgesehenen unteren Seitenabzug (o), genauso wie in der Sumpffraktion (g), ein Produktgemisch mit einem Neral-Gehalt von weniger als 20 Gew.-% und einem Geranial-Gehalt von mehr als 70 Gew.-% erhalten werden. Die Kopffraktion (j) weist üblicherweise einen Neral-Gehalt von weniger 30 Gew.-% auf. Die davon abgetrennte Leichtsiederfraktion (k) weist üblicherweise einen Neral-Gehalt von weniger als 5 Gew.-% neben iso-Citralen als Hauptkomponenten auf.

Ein weiterer Aspekt des optionalen Teilschrittes 0) der vorliegenden Erfindung betrifft die Verwendung einer wie vorstehend beschriebenen Trennwandkolonne oder einer Zusammenschaltung von zwei Destillationskolonnen in Form einer thermischen Kopplung, bevorzugt einer Trennwandkolonne mit 80 bis 200 theoretischen Trennstufen und einer oder mehrerer Seitenabzugsstellen zur kontinuierlichen Herstellung von Neral der Formel (I) in reiner oder angereicherter Form durch destillative Abtrennung von Neral aus Stoffgemischen enthaltend Neral und Geranial der Formel (II) bzw. die Verwendung derselben zur Isolierung von Neral. Ein weiterer Aspekt des optionalen Teilschritte 0) der vorliegenden Erfindung betrifft eine wie vorstehend beschriebene Trennwandkolonne oder eine Zusammenschaltung von zwei Destillationskolonnen in Form einer thermischen Kopplung, bevorzugt eine Trennwandkolonne mit 80 bis 200 theoretischen Trennstufen und einer oder mehrerer Seitenabzugsstellen, die sich zur kontinuierlichen Herstellung von Neral der Formel (I) in reiner oder angereicherter Form durch destillative Abtrennung von Neral aus Stoffgemischen enthaltend Neral und Geranial der Formel (II) eignet.

Verfahrensschritt a): Katalytische Hydrierung von Neral und/oder Geranial zu Citronellal

Gemäß Stufe a) des erfindungsgemäßen Verfahrens zur Herstellung von Menthol, führt man eine katalytische Hydrierung von Neral und/oder Geranial zu Citronellal der Formel (XIII) 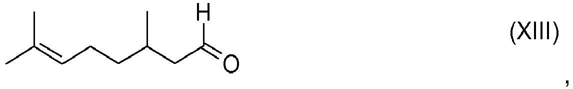 bevorzugt eine katalytische Hydrierung von wie vorstehend beschriebenem bzw. von wie vorstehend beschrieben durch destillative Trennung von Geranial- und Neralhaltigen Stoffgemischen hergestelltem Neral in reiner oder angereicherter Form durch.

Katalytische Hydrierverfahren der genannten Ausgangsstoffe zu racemischem Citronellal sind dem Fachmann bekannt und beispielsweise in W. J. Houlihan, J. Org. Chem. 1958, 23, 689-690; R. Giannandrea, P. Mastrorilli, G. Zaccaria, C. F. Nobile, J. Mol. Cat. A. 1996, 109, 113-117; U. K. Singh, M. A. Vannice, J. Catal. 2000, 191, 165-180; WO 2004/007414 A1 beschrieben. Daneben ist aus der EP 0 000 315 auch ein enantioselektives Verfahren zur Herstellung von optisch aktivem Citronellal durch Hydrierung von Geranial oder Neral in Gegenwart eines Rhodium-Phosphin-Katalysators bekannt.

Ein verbessertes und im Rahmen der vorliegenden Erfindung bevorzugt einsetzbares Verfahren zur Herstellung optisch aktiver Carbonylverbindungen durch asymmetrische Hydrierung α,β-ungesättigter Carbonylverbindungen in Gegenwart von im Reaktionsgemisch löslichen, optisch aktiven Übergangsmetallkatalysatoren, die mindestens einen Kohlenmonoxid-Liganden aufweisen ist aus der WO 2006/040096 bekannt, auf die hiermit vollumfänglich Bezug genommen wird und die, einschließlich aller bevorzugten Ausführungsformen, als Bestandteil der vorliegenden Offenbarung zu betrachten ist. Auch die dabei vorteilhaft einzusetzenden Katalysatoren bzw. geeignete Liganden zur Herstellung derselben können der genannten Offenbarung entnommen werden. Das genannte Hydrierverfahren ist dadurch gekennzeichnet, dass man den Katalysator mit einem Kohlenmonoxid und Wasserstoff enthaltenden Gasgemisch vorbehandelt und/oder die asymmetrische Hydrierung in Gegenwart von dem Reaktionsgemisch zusätzlich zugeführtem Kohlenmonoxid durchführt.

Im Rahmen einer besonders bevorzugten Ausführungsform des im Rahmen von Verfahrensschritt a) bevorzugt durchführbaren Hydrierverfahrens setzt man Neral oder Geranial, bevorzugt Neral, wiederum bevorzugt solches, das bis zu etwa 5 mol-%, besonders bevorzugt bis zu etwa 2 mol-% des jeweiligen anderen Doppelbindungsisomeren enthält, zu optisch aktivem Citronellal um.

Zur Bildung des im Rahmen der von Verfahrensschritt a) bevorzugt durchzuführenden asymmetrischen katalytischen Hydrierung einzusetzenden Katalysators setzt man bevorzugt eine im Reaktionsgemisch lösliche Verbindung des Rhodiums, insbesondere Rh(OAc)₃, [Rh(cod)Cl]₂, Rh(CO)₂acac, [Rh(cod)OH]₂, [Rh(cod)OMe]₂, Rh₄(CO)₁₂ oder Rh₆(CO)₁₆ und als chiralen Liganden (R,R)-Chiraphos der Formel (R-VII) bzw. (S,S)-Chiraphos der Formel S-VII)

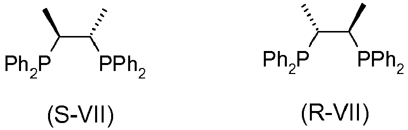

((2R, 3R)-(+)-2,3-Bis(diphenylphosphino)butan bzw. (2S, 3S)-(-)-2,3-Bis(diphenylphosphino)butan) im molaren Verhältnis von etwa 1:1 bis etwa 1:4 ein. In einer insbesondere bevorzugten Ausführungsform des erfindungsgemäßen Verfahren setzt man Neral, das bis zu etwa 5 mol-%, bevorzugt bis zu etwa 2 mol-% Geranial enthält, in Gegenwart von Rh(OAc)₃, [Rh(cod)Cl]₂, Rh(CO)₂acac, [Rh(cod)OH]₂, [Rh(cod)OMe]₂, Rh₄(CO)₁₂ oder Rh₆(CO)₁₆ und (R,R)-Chiraphos zum D-Citronellal der Formel (R-XIII) 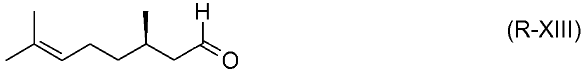 um.

Neben den genannten chiralen Liganden eignen sich zur Durchführung des im Rahmen von Verfahrensschritt a) des erfindungsgemäßen Verfahrens bevorzugt durchzuführenden Hydrierverfahrens auch die in der WO 2006/040096 genannten Liganden, insbesondere die Liganden der allgemeinen Formeln (VIII), (IX) und (X), bevorzugt solche der allgemeinen Formel (VIII), 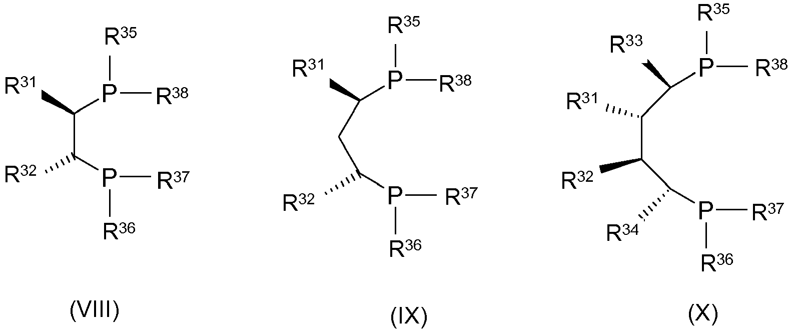 in denen
- R³¹, R³²:: jeweils unabhängig voneinander für einen unverzweigten, verzweigten oder cyclischen Alkylrest mit 1 bis 20 Kohlenstoffatomen, der gegebenenfalls eine oder mehrere, in der Regel 1 bis etwa 4, ethylenische Doppelbindungen und/oder einen oder mehrere, in der Regel 1 bis etwa 4, gleiche oder verschiedene Substituenten ausgewählt aus der Gruppe der Substituenten OR³⁹, NR⁴⁰R⁴¹, Halogen, C₆-C₁₀-Aryl und C₃-C₉-Hetaryl tragen kann und R³¹ und R³² gemeinsam einen 4 bis 20-gliedrigen Ring bilden können, der eines oder mehrere, in der Regel 1 oder 2 O-Atome beinhalten kann, stehen und
- R³³ R³⁴:: jeweils unabhängig voneinander Wasserstoff oder geradkettiges oder verzweigtes C₁- bis C₄-Alkyl bedeuten und
- R³⁵, R³⁶, R³⁷, R³⁸:: jeweils für C₆- bis C₁₀-Aryl, das gegebenenfalls einen oder mehrere, in der Regel 1 bis 8, bevorzugt 1 bis 4 Substituenten ausgewählt aus der Gruppe der Substituenten C₁- bis C₄-Alkyl, C₆- bis C₁₀-Aryl, C₁- bis C₄-Alkoxy und Amino tragen kann, stehen und
- R³⁹, R⁴⁰, R⁴¹:: jeweils unabhängig voneinander Wasserstoff, C₁-C₄-Alkyl, C₆-C₁₀-Aryl, C₇-C₁₂-Aralkyl oder C₇-C₁₂-Alkylaryl bedeuten, wobei
- R⁴⁰, R⁴¹:: gemeinsam auch eine Alkylenkette mit 2 bis 5 Kohlenstoffatomen, die durch N oder O unterbrochen sein kann, bedeuten können.

Dabei können die genannten Reste die in der WO 2006/040096 beispielhaft genannten Bedeutungen zukommen.

Bevorzugt präformiert man den zur Hydrierung einzusetzenden Katalysator unter den in WO 2006/040096 genannten Bedingungen und führt anschließend die asymmetrische Hydrierung in Gegenwart von Wasserstoff durch, der etwa 600 bis etwa 3000 ppm Kohlenmonoxid enthält. Im Rahmen der bevorzugten Ausführungsform verzichtet man vorteilhaft auf den Zusatz von Lösemitteln und führt die genannten Umsetzungen im umzusetzenden Substrat bzw. dem Produkt und gegebenenfalls in hochsiedenden Nebenprodukten als Lösemedium durch. Insbesondere bevorzugt ist die kontinuierliche Reaktionsführung unter Wiederverwendung bzw. Rückführung des erfindungsgemäß stabilisierten homogenen Katalysators.

Eine im Rahmen der Hydrierung gemäß Verfahrensschritt a) des erfindungsgemäßen Verfahrens zur Herstellung von Menthol weiterhin bevorzugten Ausführungsform betrifft ein Verfahren zur Herstellung optisch aktiver Carbonylverbindungen durch asymmetrische Hydrierung α,β-ungesättigter Carbonylverbindungen in Gegenwart von im Reaktionsgemisch löslichen, optisch aktiven Übergangsmetall-Katalysatoren, die mindestens einen Kohlenmonoxid-Liganden aufweisen, wobei man zur Herstellung des jeweils einzusetzenden optisch aktiven, mindestens einen Kohlenmonoxid-Liganden aufweisenden Katalysators eine Katalysator-Vorstufe mit einem Kohlenmonoxid und Wasserstoff enthaltenden Gasgemisch vorbehandelt und die asymmetrische Hydrierung in Gegenwart von dem Reaktionsgemisch zusätzlich zugeführtem Kohlenmonoxid durchführt, das dadurch gekennzeichnet ist, dass man
i) die Vorbehandlung der Katalysator-Vorstufe mit einem Gasgemisch umfassend 20 bis 90 Vol.-% Kohlenmonoxid, 10 bis 80 Vol-% Wasserstoff und 0 bis 5 Vol.-% weiterer Gase, wobei sich die genannten Volumenanteile zu 100 Vol.-% ergänzen, bei einem Druck von 5 bis 100 bar durchführt,
ii) von dem so erhaltenen Katalysator vor Einsatz in der asymmetrischen Hydrierung überschüssiges Kohlenmonoxid abtrennt und
iii) die asymmetrische Hydrierung in Gegenwart von Wasserstoff mit einem Kohlenmonoxidgehalt von 100 bis 1200 ppm durchführt.

Im Anschluss an die wie in der WO 2006/040096 beschrieben durchführbaren Präformierung des einzusetzenden Übergangsmetall-Katalysators bzw. dessen Vorstufe gemäß Schritt i) trennt man gemäß Schritt ii) des bevorzugten Hydrierverfahrens von dem durch Präformierung bzw. Vorbehandlung mit dem genannten Gasgemisch erhaltenen Katalysator vor Einsatz in der asymmetrischen Hydrierung überschüssiges Kohlenmonoxid ab.

Unter dem Begriff überschüssiges Kohlenmonoxid ist dabei solches Kohlenmonoxid zu verstehen, das in dem gemäß Schritt i) durch Präformierung erhaltenen Reaktionsgemisch in gasförmiger oder gelöster Form enthalten ist und nicht an den Übergangsmetall-Katalysator bzw. dessen Vorstufe gebunden ist. Demgemäß entfernt man das überschüssige, nicht an den Katalysator gebundene Kohlenmonoxid zumindest weitgehend, d.h. in einem Ausmaß, dass sich eventuelle Restmengen gelösten Kohlenmonoxids in der folgenden Hydrierung nicht störend bemerkbar machen. Dies ist üblicherweise gewährleistet, wenn etwa 90%, bevorzugt etwa 95% oder mehr des zur Präformierung eingesetzten Kohlenmonoxids gemäß Schritt ii) dieser bevorzugten Ausführungsform des Hydrierverfahrens abgetrennt werden. Bevorzugt entfernt man gemäß Schritt ii) überschüssiges Kohlenmonoxid vollständig von dem durch Präformierung erhaltenen Katalysator.

Die Abtrennung des überschüssigen Kohlenmonoxids vom gemäß Schritt i) erhaltenen Katalysator bzw. vom den Katalysator enthaltenden Reaktionsgemisch gemäß Schritt ii) der bevorzugten Ausführungsform des Hydrierverfahrens kann auf verschiedenen Wegen erfolgen. Bevorzugt entspannt man den Katalysator bzw. das durch Präformierung gemäß Schritt i) erhaltene, den Katalysator enthaltende Gemisch auf einen Druck von bis zu etwa 5 bar (absolut), bevorzugt auf einen Druck im Bereich von etwa 1 bar bis etwa 5 bar, so dass gasförmiges, nicht gebundenes Kohlenmonoxid aus dem Produkt der Präformierung entweicht.

Die vorstehend genannte Entspannung des präformierten Katalysators kann beispielsweise unter Einsatz eines Hochdruckabscheiders, wie er dem Fachmann an sich bekannt ist, erfolgen. Derartige Abscheider, bei denen die Flüssigkeit in der kontinuierlichen Phase ist, sind beispielsweise beschrieben in: Perry's Chemical Engineers' Handbook, 1997, 7. Aufl., McGraw-Hill, S. 14.95 und 14.96; die Verhinderung eines möglichen Tropfenmitrisses ist auf den Seiten 14.87 bis 14.90 beschrieben. Die Entspannung des präformierten Katalysators kann einstufig oder zweistufig bis zum Erreichen des gewünschten Druckes im Bereich von 1 bar bis etwa 5 bar erfolgen, wobei die Temperatur üblicherweise auf 10 bis 40°C sinkt.

Alternativ kann die Abtrennung überschüssigen Kohlenmonoxids gemäß Schritt ii) auch durch sogenanntes Strippen des Katalysators bzw. des den Katalysator enthaltenden Gemischs mit einem Gas, vorteilhaft mit einem unter den Reaktionsbedingungen inerten Gas, erreicht werden. Unter dem Begriff Strippen versteht der Fachmann dabei das Einleiten eines Gases in den Katalysator bzw. das den Katalysator enthaltende Reaktionsgemisch wie beispielsweise in W. R. A. Vauck, H. A. Müller, Grundoperationen chemischer Verfahrenstechnik, Deutscher Verlag für Grundstoffchemie Leipzig, Stuttgart, 10. Auflage, 1984, Seite 800 beschrieben. Als geeignete inerte Gase seien hierfür beispielhaft genannt: Wasserstoff, Helium, Neon, Argon, Xenon, Stickstoff und/oder CO₂, bevorzugt Wasserstoff, Stickstoff, Argon.

Im Anschluss an die Präformierung gemäß Schritt i) und die Befreiung des Katalysators von überschüssigem Kohlenmonoxid gemäß Schritt ii) führt man gemäß Schritt iii) dieser bevorzugten Ausführungsform des erfindungsgemäß einsetzbaren Hydrierverfahrens die asymmetrische Hydrierung des gewählten Substrats in Gegenwart von Wasserstoff mit einem Kohlenmonoxidgehalt von 100 bis 1200 ppm durch.

Die Zugabe von zusätzlichem Kohlenmonoxid zum Reaktionsgemisch der asymmetrischen Hydrierung kann auf verschiedene Weise vorgenommen werden: So kann das Kohlenmonoxid beispielsweise dem zur asymmetrischen Hydrierung eingesetzten Wasserstoff beigemischt oder auch direkt gasförmig in die Reaktionslösung eindosiert werden. Eine weitere Möglichkeit besteht beispielsweise darin, dem Reaktionsgemisch Verbindungen zuzusetzen, die leicht Kohlenmonoxid freisetzen, wie beispielsweise Formiate oder Oxalyl-Verbindungen.

Der Anteil an Kohlenmonoxid im eingesetzten Wasserstoff beträgt im Rahmen einer bevorzugten Ausführungsform des erfindungsgemäßen Verfahrens etwa 300 bis 1000 ppm, besonders bevorzugt 400 bis 800 ppm.

Die vorstehend beschriebene asymmetrische Hydrierung nimmt man vorteilhaft bei einem Druck von etwa 1 bis etwa 300 bar, bevorzugt von etwa 10 bis etwa 100 bar, insbesondere bei etwa 50 bis etwa 100 bar und einer Temperatur von in der Regel etwa 0°C bis etwa 100°C, bevorzugt etwa 0°C bis etwa 30°C, insbesondere bei etwa 10°C bis etwa 30°C vor.

Die Wahl des zur Durchführung der asymmetrischen Hydrierung einzusetzenden Lösemittels ist nicht kritisch. Geeignete Lösemittel sind beispielsweise jene zur Durchführung der erfindungsgemäßen Präformierung genannten. Mit besonderem Vorteil führt man die asymmetrische Hydrierung im gleichen Lösemittel durch wie die gegebenenfalls zuvor durchgeführte Präformierung.

Als Reaktionsgefäße zur Durchführung der vorstehend beschriebenen asymmetrischen Hydrierung sind prinzipiell all jene geeignet, die Umsetzungen unter den genannten Bedingungen, insbesondere in den genannten Druck- und Temperaturbereichen erlauben und für Hydrierreaktionen geeignet sind, wie beispielsweise Autoklaven, Rohrreaktoren, Blasensäulen und dergleichen mehr.

Führt man die Hydrierung gemäß Schritt iii) des im Rahmen des erfindungsgemäßen Verfahrens bevorzugten Hydrierverfahrens unter Einsatz von hochsiedenden, in der Regel viskosen Lösungsmitteln durch, wie sie beispielsweise in der WO 2006/040096 zum Einsatz im Rahmen der Vorbehandlung des Katalysators gemäß Schritt i) des erfindungsgemäßen Verfahrens beschrieben sind (etwa die genannten Lösungsmittel Octadecanol, Biphenylether, Texanol, Marlotherm®, Oxoöl 9N oder führt man die Hydrierung ohne zusätzlichen Einsatz von Lösemittel jedoch unter Aufpegelung der als Nebenprodukte in geringem Ausmaß entstehenden Hochsieder (wie beispielsweise Dimere oder Trimere, die durch Reaktionen der Edukte bzw. Produkte und anschließenden Folgereaktionen entstehen) durch, kann es vorteilhaft sein, für guten Gaseintrag und gute Durchmischung von Gasphase und kondensierter Phase zu sorgen. Dies gelingt beispielsweise dadurch, dass man den Hydrierschritt des Verfahrens in einem Gasumlaufreaktor durchführt. Gasumlaufreaktoren sind dem Fachmann an sich bekannt und beispielsweise in P. Trambouze, J.-P. Euzen, Chemical Reactors, Ed. Technip, 2004, S. 280-283 und P. Zehner, R. Benfer, Chem. Eng. Sci. 1996, 51, 1735-1744 sowie z. B. in der EP 1 140 349 beschrieben.

Bei Einsatz eines wie vorstehend genannten Gasumlaufreaktors hat es sich als besonders vorteilhaft erwiesen, das einzusetzende Gas bzw. Gasgemisch (den Kohlenmonoxid enthaltenden Wasserstoff) parallel zu den in den Reaktor eingetragenen Edukten und/oder dem umlaufenden Reaktionsgemisch bzw. dem Katalysator mittels einer einfachen Düse oder einer Zweistoffdüse in den Gasumlaufreaktor einzutragen. Dabei zeichnet sich die Zweistoffdüse dadurch aus, dass in den Reaktor einzubringende Flüssigkeit und Gas durch zwei getrennte ineinander liegenden Röhren unter Druck bis zum Düsenmund gelangen und dort miteinander vereint werden.

Das erfindungsgemäß bevorzugte Hydrierverfahren kann mit gutem Erfolg mit und ohne Zugabe von tertiären Aminen durchgeführt werden. Bevorzugt führt man das erfindungsgemäße Verfahren in Abwesenheit, d.h. ohne Zugabe von zusätzlichen tertiären Aminen oder in Anwesenheit nur katalytischer Mengen von zusätzlichen tertiären Aminen durch. Die verwendete Aminmenge kann dabei zwischen 0.5 und 500 molÄquivalenten bezogen auf die eingesetzte Menge an Metall, bevorzugt aber 1 bis 100 mol-Äquivalenten bezogen auf die eingesetzte Metallmenge betragen. Die Wahl des tertiären Amins ist nicht kritisch. Neben kurzkettigen Alkylaminen, wie beispielsweise Triethylamin können auch langkettige Alkylamine, wie zum Beispiel Tridodecylamin, verwendet werden. Im Rahmen einer bevorzugten Ausführungsform führt man das erfindungsgemäße Hydrierverfahren in Gegenwart eines tertiären Amins, bevorzugt Tridodecylamin, in einer Menge von etwa 2 bis 30 mol-Äquivalenten, bevorzugt etwa 5 bis 20 mol-Äquivalenten und besonders bevorzugt 5 bis 15 mol-Äquivalenten bezogen auf die Menge an eingesetztem Übergangsmetall ein.

Das erfindungsgemäß bevorzugt durchzuführende asymmetrische Hydrierverfahren zeichnet sich dadurch aus, dass die eingesetzten homogenen Katalysatoren durch das zusätzlich ins Reaktionssystem eingebrachte Kohlenmonoxid stabilisiert werden, wodurch zum einen die Standzeit der Katalysatoren deutlich erhöht wird und zum anderen die Wiederverwendbarkeit der homogenen Katalysatoren ermöglicht wird.

So lässt sich beispielsweise das erhaltene Reaktionsprodukt durch dem Fachmann an sich bekannte Verfahren, wie z.B. durch Destillation, beispielsweise mittels eines Feinfilmverdampfers, Sambays oder dergleichen mehr aus dem Reaktionsgemisch entfernen und der zurückbleibende Katalysator, gegebenenfalls nach abermaliger, wie vorstehend beschriebener Präformierung, im Rahmen weiterer Umsetzungen nutzen.

Das erfindungsgemäß im Rahmen von Verfahrenschritt a) bevorzugt einzusetzende Hydrierverfahren kann demgemäß sowohl diskontinuierlich bzw. semikontinuierlich als auch kontinuierlich betrieben werden und eignet sich insbesondere für Umsetzungen in technischem Maßstab. Bevorzugt führt man das Verfahren kontinuierlich durch.

Die im Rahmen der erfindungsgemäß bevorzugten asymmetrischen Hydrierung durchzuführende Vorbehandlung der Katalysatorvorstufe (Präformierung) gemäß Schritt i) und die eigentliche asymmetrische Hydrierung gemäß Schritt iii) werden vorteilhaft in getrennten Reaktionsgefäßen durchgeführt. Bei der Überführung des präformierten Katalysators in den eigentlichen Hydrierrektor, bevorzugt den wie vorstehend beschriebenen Gasumlaufreaktor, kann dann das überschüssige Kohlenmonoxid vom Katalysator, beispielsweise durch Entspannung des zur Präformierung angewendeten Druckes, entfernt werden.

Auch die Hydrierung kann in mehreren, vorzugsweise in zwei oder drei, besonders bevorzugt in zwei, hintereinander geschalteten Hydrierreaktoren erfolgen. Dabei können verschiedenartige oder gleiche Reaktortypen zum Einsatz kommen. In einer bevorzugten Ausführungsform führt man die asymmetrische Hydrierung beispielsweise in einer Kaskade von zwei Gasumlaufreaktoren durch, wobei einer als Hauptreaktor und der zweite als Nachreaktor fungiert. Zur Überführung des Reaktionsgemisches vom Hauptreaktor in den Nachreaktor kann dabei beispielsweise ein gewünschtenfalls so einzustellendes Druckgefälle genutzt werden.

Das auf diese Weise zugängliche racemische oder optisch aktive Citronellal fällt üblicherweise in hoher Ausbeute und insbesondere hoher chemischer und optischer Reinheit an. Je nach den an die chemische Reinheit des erhaltenen und im Rahmen des weiteren Verfahrensschritts b) weiter umzusetzenden Citronellals, bevorzugt D-Citronellals, gestellten Anforderungen kann dieses noch durch dem Fachmann an sich bekannte Trenn- bzw. Reinigungsverfahren, wie beispielsweise chromotografische oder destillative Verfahren, weiter aufgereinigt werden. Es hat sich als vorteilhaft erwiesen, das erhaltene Citronellal durch Destillation weiter aufzureinigen, wobei im Prinzip alle dem Fachmann geeeignet erscheinenden Destillationsverfahren bzw. - vorrichtungen wie beispielsweise Destillationskolonnen (gepackt oder ungepackt), Fallfilmverdampfer, Dünschichtverdampfer und dergleichen mehr eingesetzt werden können. Die genannten Verfahren können auch in Kombination miteinander, d.h. hintereinander durchgeführt werden. So hat sich eine Vorreinigung des durch die dargestellte Hydrierung erhaltenen, Citronellal-haltigen Produktgemisches mittels eines Fallfilmverdampfers und anschließende Feindestillation des Citronellals als vorteilhaft erwiesen.

Zur destillativen Reinigung bzw. Abtrennung des erhaltenen optisch aktiven oder racemischen Citronellals führt man bevorzugt eine Destillation mittels einer Trennwandkolonne durch wie sie beispielsweise in der DE 103 30 934 A1 offenbart ist. Unter Einsatz einer Trennwandkolonne mit etwa 30 bis etwa 100, bevorzugt etwa 45 bis etwa 85 theoretischen Trennstufen gelingt es, bei geeigneter Wahl von Druck und Temperatur der Destillation üblicherweise Citronellal mit hoher Reinheit, oft mit einer Reinheit von 98 Gew.-% und darüber, bevorzugt von 99 Gew.-% und darüber zu isolieren.

### Verfahrensschritt b): Cyclisierung von Citronellal zu Isopulegol

Gemäß Verfahrensschritt b) des erfindungsgemäßen Verfahrens führt man eine Cyclisierung von Citronellal, das nach dem vorstehend beschriebenen Verfahrenschritt a) durch katalytische Hydrierung von Neral- und/oder Geranial enthaltenden Stoffgemischen erhalten wurde, zu Isopulegol in Gegenwart eines sauren Katalysators durch.

Die Cyclisierung von Citronellal zu Isopulegol unter sauren Bedingungen ist altbekannt. Eine Übersicht über die zur Verfügung stehenden sauren bzw. Lewis-sauren Reagenzien bzw. Katalysatoren findet sich beispielsweise unter E. J. Lenardao, G. V. Botteselle, F. de Azambuja, G. Perin, R. G. Jacob Tetrahedron 2007, 63, 6671-6712.

Als gebräuchliche Katalysatoren bzw. Reagenzien sind eine breite Vielfalt von Systemen bekannt, wie beispielsweise: Kieselgel oder Aluminumoxid oder Gemische derselbe, wie z.B. in der WO 2004/089299 offenbart, Zeolithe, wie z.B. für den Fall borhaltiger Zeolithe in der WO 2004/101480 beschrieben. Weitere gebrächliche saure bzw. Lewis-saure Katalysatoren sind beispielsweise Zinkbromid, wie z.B. in Synthesis 1978, 147-148 und in der EP 1053974 A1 oder auch Wolfram-haltige Säuren wie in der BR 2005002489 A beschrieben.

Die EP-A 1 225 163 beschreibt darüber hinaus die Cyclisierung von Citronellal zu Isopulegol in Gegenwart von Tris(2,6-diphenylphenol)-aluminium-Katalysatoren. Tris(2,6-diphenylphenol)-aluminium ist literaturbekannt und als Katalysator für selektive 1,4-Funktionalisierungen von α,β-ungesättigten Carbonylverbindungen und für spezielle Claisen-Umlagerungen beschrieben, beispielsweise in Angew. Chem. Int. Ed. 2004, 43, 994. Das genannte Katalysatorsystem eignet sich auch zum Einsatz im Rahmen von Verfahrensschritt b) des erfindungsgemäßen Verfahrens.

Die WO 2007/039342 und die WO 2007/039366 offenbaren ebenfalls Aluminiumhaltige homogene Katalysatoren, und zwar solche, die einen oder mehrere Siloxid-Liganden am Aluminium aufweisen. Die offenbarten Aluminium-Siloxid-Verbindungen eignen sich als Katalysatoren für intramolekulare Prins-Reaktionene, darunter die Cyclisierung von Citronellal zu Isopulegol.

Erfindungsgemäß bevorzugt führt man Cyclisierung von Citronellal zu Isopulegol gemäß Verfahrensschritt b) in Gegenwart eines Aluminium-haltigen, speziell in Gegenwart eines Lewis-sauren Aluminium-haltigen Katalysators durch.

Ein im Rahmen von Verfahrensschritt b) des erfindungsgemäßen Verfahrens besonders bevorzugtes Verfahren zur Cyclisierung von Citronellal zu Isopulegol ist in der WO 2006/092433 beschrieben, auf die hiermit vollumfänglich Bezug genommen wird und deren Offenbarung einschließlich aller Bevorzugungen und Ausführungsformen als Bestandteil der vorliegenden Offenbarung zu betrachten ist. Die genannte Patentanmeldung offenbart spezielle Diarylphenoxy-Aluminium-Verbindungen, die erhältlich sind durch Umsetzung eines Bis(diarylphenol)-Liganden der Formel (I) 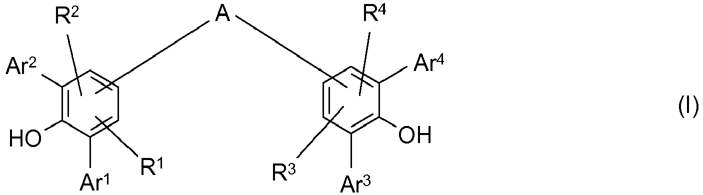 wobei
- Ar¹, Ar², Ar³, Ar⁴: gleich oder verschieden sind und jeweils unabhängig voneinander einen Arylrest mit 6 bis15 Kohlenstoffatomen oder einen Heteroarylrest mit 2 bis 15 Kohlenstoffatomen, die gegebenenfalls jeweils 1 bis 7 gleiche oder verschiedene Substituenten, ausgewählt aus der Gruppe der Substituenten C₁- bis C₆-Alkyl, C₁- bis C₆-Perfluoralkyl, C₁- bis C₆-Alkoxy, C₇- bis C₁₂-Aralkyl, Halogen, -SiR^{5a}R^{6a}R^{7a}, substituiertes oder unsubstituiertes C₆- bis C₁₀-Aryl, -NR^{8a}R^{9a}, -SR^{10a} und-NO₂ tragen können, bedeuten,
- R¹, R², R³, R⁴: gleich oder verschieden sind und jeweils unabhängig voneinander Wasserstoff, C₁- bis C₆-Alkyl, C₁- bis C₆-Perfluoralkyl, C₁- bis C₆-Alkoxy, C₇- bis C₁₂-Aralkyl, Halogen, -SiR^{5b}R^{6b}R^{7b}, substituiertes oder unsubstituiertes C₆- bis C₁₀-Aryl, -NR^{8b}R^{9b}, -SR10^{b} und/oder -NO₂ bedeuten und R¹ oder R² und/oder R³ oder R⁴ gemeinsam mit A einen aromatischen oder nicht-aromatischen Cyclus bilden kann, bedeuten und
- A: (1) für einen geradkettigen oder verzweigten und/oder cyclischen Kohlenwasserstoffrest mit 1 bis 25 Kohlenstoffatomen steht, der gesättigt oder einoder mehrfach ungesättigt und/oder teilweise aromatisch sein kann und gegebenenfalls eines oder mehrere gleiche oder verschiedene Heteroatome, ausgewählt aus der Gruppe der Heteroatome O, S und NR¹¹ und/oder eine oder mehrere gleiche oder verschiedene funktionelle Gruppen, ausgewählt aus der Gruppe der funktionellen Gruppen C(O), S(O) und S(O)₂, aufweisen kann und gegebenenfalls einen oder mehrere gleiche oder verschiedene Substituenten, ausgewählt aus der Gruppe der Substituenten C₁- bis C₆-Alkyl, C₁- bis C₆-Perfluoralkyl, C₁- bis C₆-Alkoxy, C₁- bis C₁₀- Acyloxy, C₇- bis C₁₂-Aralkyl, Halogen, -SiR^{5c}R^{6c}R^{7c}, substituiertes oder unsubstituiertes C₆- bis C₁₀-Aryl, substituiertes oder unsubstituiertes C₂- bis C₁₀-Hetaryl, -NR^{8c}R^{9c}, -SR^{10c}, -NO₂, C₁-bis C₁₂-Acyl und C₁- bis C₁₀- Carboxyl tragen kann, oder
(2) für einen Arylrest mit 6 bis15 Kohlenstoffatomen oder einen Heteroarylrest mit 2 bis 15 Kohlenstoffatomen steht, die gegebenenfalls jeweils 1 bis 5 Substituenten, ausgewählt aus der Gruppe der Substituenten C₁- bis C₆-Alkyl, C₁- bis C₆-Perfluoralkyl, C₁- bis C₆-Alkoxy, C₇- bis C₁₂-Aralkyl, Halogen,-SiR^{5d}R^{6d}R^{7d}, substituiertes oder unsubstituiertes C₆- bis C₁₀-Aryl, -NR^{8d}R^{9d}, SR^{10d} und NO₂ tragen können, oder
(3) für eine funktionelle Gruppe oder ein Heteroatom ausgewählt aus der Gruppe -O-, -S-, -N(R¹¹)-, -S(O)-, -C(O)-, -S(O)₂-, -P(R¹¹)-, -(R¹¹)P(O)- und-Si(R¹²R¹³) steht,
wobei die Reste R^{5a}, R^{6a}, R^{7a}, R^{8a}, R^{9a}, R^{10a} bis R^{5d}, R^{6d}, R^{7d}, R^{8d}, R^{9d}, R^{10d} und R¹¹ bis R¹³ jeweils unabhängig voneinander für C₁- bis C₆-Alkyl, C₇- bis C₁₂-Aralkyl und/oder substituiertes oder unsubstituiertes C₆- bis C₁₀-Aryl stehen, und die Reste R^{8a} und R^{9a}, R^{8b} und R^{9b}, R^{8c} und R^{9c}, R^{8d} und R^{9d} unabhängig voneinander jeweils gemeinsam auch einen cyclischen Kohlenwasserstoffrest mit 2 bis 8 Kohlenstoffatomen bilden können, der eines oder mehrere gleiche oder verschiedene Heteroatome ausgewählt aus der Gruppe O, S und NR^{11a} aufweisen kann und R^{11a} die für R¹¹ angegebenen Bedeutungen haben kann,
mit einer Aluminium-Verbindung der Formel (XIV)

(R¹⁴)₃₋ₚAlHₚ (XIV),

wobei
- Al: Aluminium bedeutet und
- R¹⁴: einen verzweigten oder unverzweigten Alkylrest mit 1 bis 5 Kohlenstoffatomen und
- p: 0 oder eine ganze Zahl von 1 bis 3 bedeutet,
und/oder
mit einer Aluminium-Verbindung der Formel (XV)

MAIH₄ (XV),

wobei
- Al: Aluminium bedeutet und
- M: Lithium, Natrium oder Kalium bedeutet.

Die zur Herstellung der im Rahmen von Verfahensschritt b) des erfindungsgemäßen Verfahrens bevorzugt einzusetzenden Diarylphenoxy-Aluminium-Verbindungen einzusetzenden Bis(diarylphenol)-Liganden der Formel (I) weisen zwei Phenolsysteme auf, die jeweils in beiden ortho-Positionen zur phenolischen Hydroxy-Gruppe durch Aromaten bzw. Heteroaromaten (Ar¹ bis Ar⁴) substituiert sind und über ein Strukturelement A miteinander verknüpft sind und gegebenenfalls noch weitere Substituenten (R¹ bis R⁴) tragen können. Die genannten Diarylphenoxy-Aluminium-Verbindungen werden als Reaktionsprodukte bzw. Produktgemische der Umsetzung der vorstehend genannten Bis(diarylphenol)-Liganden der Formel (I) mit den Aluminium-Verbindungen (XIV) oder (XV) erhalten.

Im Rahmen des erfindungsgemäßen Verfahrens bevorzugt einzusetzende Diarylphenoxy-Aluminium-Verbindungen sind solche, die erhältlich sind durch wie oben genannte Umsetzung von Liganden der allgemeinen Formeln (la)

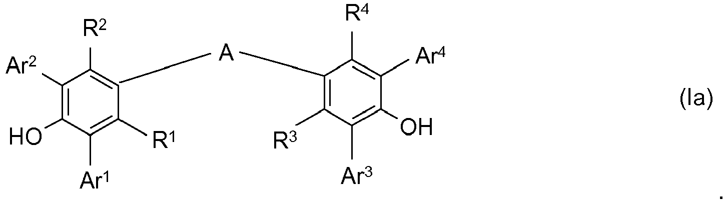

Die Liganden der Formel (la) weisen ebenfalls zwei Phenolsysteme auf, die jeweils in beiden ortho-Positionen zur phenolischen Hydroxy-Gruppe durch Aromaten bzw. Heteroaromaten (Ar¹ bis Ar⁴) substituiert sind und über ein Strukturelement A miteinander verknüpft sind und gegebenenfalls noch weitere Substituenten (R¹ bis R⁴) tragen können, wobei das Strukturelement A jeweils in para-Position zur phenolischen Hydroxy-Gruppe mit den beiden Phenolsystemen verknüpft ist. Dabei können den Resten Ar¹, Ar², Ar³, Ar⁴, den Resten R¹, R², R³, R⁴ und dem Strukturelement A die gleichen Bedeutungen zukommen wie vorstehend für Formel (I) genannt.

Insbesonders bevorzugte Bis(diarylphenol)-Liganden zur Herstellung der erfindungsgemäß im Rahmen des Verfahrensschritts b) bevorzugt einzusetztenden Diarylphenoxy-Aluminium-Verbindung sind solche, die durch Umsetzung von Liganden der Formeln (Ia₁), (Ia₂) bzw. (Ia₃) wie in der WO 2006/092433 beschrieben erhältlich sind. Ein insbesondere im Rahmen von Schritt b) des erfindungsgemäßen Verfahrens einzusetzende Diarylphenoxy-Aluminium-Verbindung ist eine solche, die erhältlich ist durch Umsetzung eines Bis(diarylphenol)-Liganden der Formel (XI) 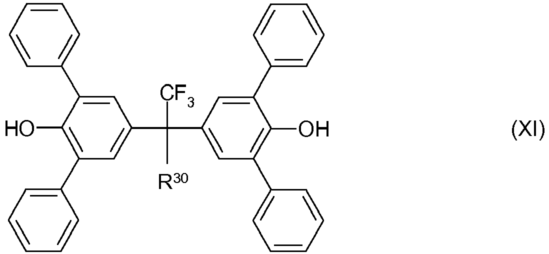 wobei der Rest
- R³⁰: C₁-bis C₆-Alkyl oder C₁- bis C₆-Perfluoralkyl
bedeutet,
mit einer Aluminium-Verbindung der Formel (XIV) und/oder (XV), bevorzugt mit Trimethyl- und/oder Triethylaluminium und besonders bevorzugt mit Triethylaluminium wie in der vorstehend genannten WO 2006/092433 beschrieben.

Dabei sind unter C₁- bis C₆-Alkyl geradkettige oder verzweigte Alkylreste mit 1 bis 6 Kohlenstoffatomen, wie beispielsweise Methyl, Ethyl, Propyl, Isopropyl, n-Butyl, sec-Butyl, tert-Butyl, n-Pentyl oder n-Hexyl, bevorzugt Methyl, Ethyl, Isopropyl zu verstehen.

Unter dem Begriff C₁- bis C₆-Perfluoralkyl sind dabei Alkylreste mit 1 bis 6 Kohlenstoffatomen zu verstehen, bei denen alle Wasserstoffatome durch Fluoratome substituiert sind, wie beispielsweise Trifluormethyl, Pentafluorethyl, Heptafluorpropyl, Heptafluorisopropyl, Nonafluorbutyl, bevorzugt Trifluormethyl.

Eine im Rahmen der vorliegenden Erfindung besonders bevorzugte Diarylphenoxy-Aluminium-Verbindung ist eine solche, die erhältlich ist durch Umsetzung des Bis(diarylphenol)-Liganden der Formel (Ia₂-3) 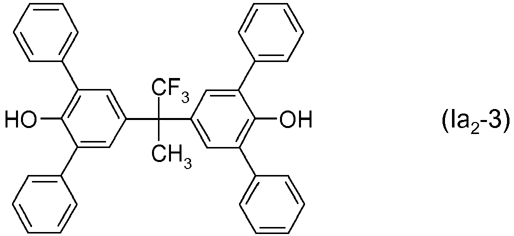 mit Trimethyl- oder Triethylaluminium, bevorzugt Triethylaluminium unter den in der vorstehend genannten WO 2006/092433 angegebenen Bedingungen einschließlich aller darin beschiebenen bevorzugten Ausführungsformen und deren Kombinationen.

Ein weiterer, erfindungsgemäß bevorzugter Bis(diarylphenol)-Ligand ist der zwei Trifluormethylgruppen aufweisende Ligand der Formel (Ia₂-1)

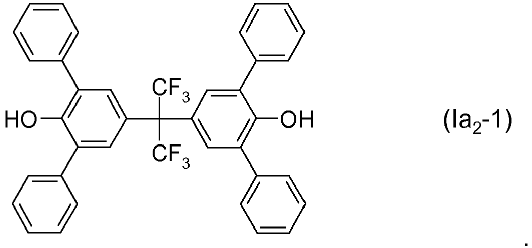

Im Rahmen einer bevorzugten Ausfürungsform führt man den erfindungsgemäß durchzuführenden Verfahrensschritt b) in Form eines Verfahrens zur Herstellung von Isopulegol der Formel (XII) 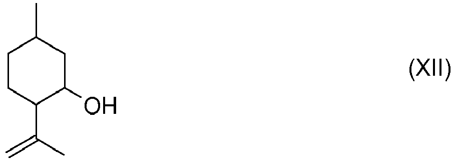 durch, umfassend die Cyclisierung von Citronellal der Formel (XIII) 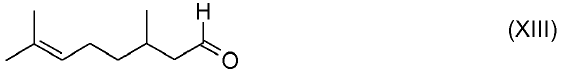 in Gegenwart eines Katalysators, der erhältlich ist durch Umsetzung eines Bis(diarylphenol)-Liganden der Formel (I) 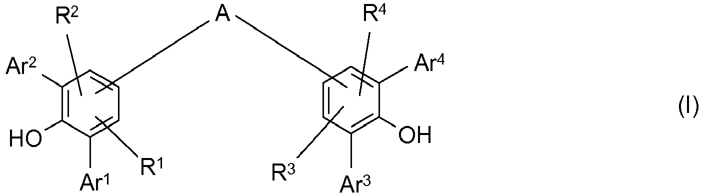 wobei
Ar¹, Ar², Ar³, Ar⁴, R¹, R², R³, R⁴ und A die vorstehend für Formel (I) angegebenen Bedeutungen besitzen
mit einer Aluminium-Verbindung der Formel (XIV)

(R¹⁴)₃₋ₚAlHₚ (XIV),

wobei
- Al: Aluminium bedeutet und
- R¹⁴: einen verzweigten oder unverzweigten Alkylrest mit 1 bis 5 Kohlenstoffatomen und
- p: 0 oder eine ganze Zahl von 1 bis 3 bedeutet,
und/oder
mit einer Aluminium-Verbindung der Formel (XV)

MAlH₄ (XV),

wobei
- Al: Aluminium bedeutet und
- M: Lithium, Natrium oder Kalium bedeutet.

Die Einzelheiten zur Durchführung dieser bevorzugten Ausführungsform des erfindungsgemäß durchzuführenden Cyclisierungsverfahrens sind der bereits genannten WO 2006/092433 zu entnehmen, auf die auch diesbezüglich umfassend Bezug genommen wird.

Die zur Cyclisierung gemäß Verfahrensschritt b) erfindungsgemäß bevorzugt verwendeten Bis(diarylphenoxy)-Aluminium-Verbindungen erhält man beispielsweise, indem man die vorstehend beschriebenen Bis(diarylphenol)-Liganden der Formeln (I) bzw. (la) mit einer Aluminium-Verbindung der Formel (XIV)

(R¹⁴)₃₋ₚAlHₚ (XIV),

umsetzt. Dabei steht R¹⁴ für einen verzweigten oder unverzweigten Alkylrest mit 1 bis 5 Kohlenstoffatomen wie beispielsweise Methyl, Ethyl, Propyl, Isopropyl, Butyl, Isobutyl, Pentyl, Isopentyl oder Neopentyl. Der Index p steht für 0 oder eine ganze Zahl von 1 bis 3. Bevorzugt steht der Index p für 1 oder 0, besonders bevorzugt für 0. Bevorzugte Verbindungen der Formel (XIV) sind beispielsweise Trimethylaluminium, Triethylaluminium, Diisobutylaluminiumhydrid, besonders bevorzugt Trimethylaluminium und Triethylaluminium.

Alternativ dazu erhält man die erfindungsgemäß bevorzugt zu verwendenden Bis(diarylphenoxy)-Aluminium-Verbindungen auch durch Umsetzung der wie vorstehend beschriebenen Bis(diarylphenol)-Liganden der Formeln (I) bzw. (la) mit einer Aluminium-Verbindung der Formel (XV)

MAlH₄ (XV),

wobei M Lithium, Natrium oder Kalium bedeutet. Demzufolge eignen sich zur Herstellung der erfindungsgemäß bevorzugt zu verwendenden Bis(diarylphenoxy)-Aluminium-Verbindungen durch Umsetzung der wie vorstehend beschriebenen Bis(diarylphenol)-Liganden der Formeln (I) bzw. (la) auch Lithiumaluminiumhydrid, Natriumaluminiumhydrid und Kaliumaluminiumhydrid sowie Gemische derselben. Darüber hinaus sind auch Gemische der genannten Verbindungen der Formeln (XIV) und (XV) zur Herstellung erfindungsgemäß verwendeten Bis(diarylphenoxy)-Aluminium-Verbindungen durch Umsetzung mit den wie vorstehend beschriebenen Bis(diarylphenol)-Liganden der Formeln (I) bzw. (Ia) geeignet.

Die Umsetzung wird vorteilhaft so durchgeführt, dass einer der wie vorstehend beschriebenen Bis(diarylphenol)-Liganden der Formeln (I) bzw. (la), insbesondere bevorzugt der Ligand der Formel (Ia₂-3) mit einer Verbindung der Formel (XIV) oder (XV) in Kontakt gebracht wird. Vorteilhaft führt man die Umsetzung in einem inerten organischen Lösungsmittel wie beispielsweise Toluol, Cyclohexan, Dichlormethan, Xylol, Ethylbenzol, Chlorbenzol, Tetrahydrofuran, Diethylether, Methyl-tert-Butylether, Essigsäureethylester, Pentan, Hexan, Dichlorethan, Dimethylformamid (DMF), Dimethylsulfoxid (DMSO) und dergleichen mehr durch, wobei der Einsatz vorgetrockneter bzw. wasserfreier Lösungsmittel als besonders vorteilhaft anzusehen ist. Üblicherweise erfolgt die Umsetzung bei Temperaturen im Bereich von etwa -100 °C bis etwa 100 °C, bevorzugt bei etwa -50 °C bis etwa 50 °C, besonders bevorzugt bei etwa -30 °C bis etwa 30 °C.

Bei der Herstellung der erfindungsgemäß im Rahmen von Verfahrensschritt b) bevorzugt einzusetzenden Bis(diarylphenoxy)-Aluminium-Verbindungen reagieren die phenolischen Hydroxy-Gruppen der eingesetzten Bis(diarylphenol)-Liganden der Formeln (I) bzw. (Ia) mit der bzw. den Verbindungen der Formeln (XIV) und (XV). Theoretisch kann jedes Aluminium-Atom mit 1 bis 3 phenolischen Hydroxy-Gruppen regieren. Aufgrund der sterischen Eigenschaften bzw. Anforderungen der eingesetzten Bis(diarylphenol)-Liganden der Formeln (I) bzw. (Ia) kann es dabei zur Ausbildung höhermolekularer Strukturen, wie linearen Strukturen oder Netzwerken, kommen.

Vorteilhaft wählt man dabei das molare Verhältnis der eingesetzten Bis(diarylphenol)-Liganden der Formeln (I) bzw. (Ia) zu den eingesetzten Verbindungen der Formel (XIV) und/oder (XV) so, dass die Menge an nicht abreagierten Verbindungen der Formeln (XIV) und/oder (XV) möglichst gering ist. Vorzugsweise wählt man das genannte Verhältnis so, dass nach dem Inkontaktbringen der Bis(diarylphenol)-Liganden der Formeln (I) bzw. (Ia) mit der bzw. den Verbindungen der Formeln (XIV) und (XV) keine nicht umgesetzte Verbindung der Formel (XIV) und/oder (XV) mehr vorliegt. Unter Berücksichtigung des wirtschaftlichen Aspekts ist es empfehlenswert, den Überschuss der eingesetzten Liganden der Formeln (I) bzw. (Ia) gering zu halten. Besonders bevorzugt setzt man Bis(diarylphenol)-Liganden der Formeln (I) bzw. (Ia) und die Verbindungen der Formeln (XIV) und/oder (XV) in einem molaren Verhältnis von etwa 4:1 bis etwa 1:1, ganz besonders bevorzugt von etwa 3:1 bis etwa 1,5:1 und am meisten bevorzugt im molaren Verhältnis von etwa 1,5:1 ein.

Zur Herstellung der erfindungsgemäß bevorzugt zu verwendenden Bis(diarylphenoxy)-Aluminium-Verbindungen geht man im Rahmen einer bevorzugten Ausführungsform der vorliegenden Erfindung so vor, dass man, je nach Löslichkeit, eine etwa 0,001 bis etwa 1 molare Lösung des gewählten Liganden der Formel (I) bzw. (Ia) in einem geeigneten organischen Lösungsmittel, beispielsweise Toluol, bei einer Temperatur von etwa -10 bis etwa 30°C vorlegt und eine Aluminiumverbindung der Formel (XIV) und/oder (XV), vorzugsweise in Form einer Lösung, beispielsweise eine Lösung von Trimethyl- oder Triethylaluminium in Toluol, zugibt.

Die Reaktion zwischen den eingesetzten Liganden der Formel (I) bzw. (Ia) und den Aluminiumverbindungen der Formeln (XIV) und/oder (XV) erfolgt in der Regel rasch und ist meist, in Abhängigkeit von den gewählten Reaktionsbedingungen, nach etwa 10 min bis etwa 2 h, oft nach etwa 1 h, abgeschlossen. Bei Einsatz reaktionsträgerer Reaktanden kann es vorteilhaft sein, die Temperatur des Reaktionsgemisches kurzzeitig zu erhöhen.

In Abhängigkeit von den gewählten Reaktionsbedingungen, insbesondere im Hinblick auf die Löslichkeit der umzusetzenden Liganden der Formel (I) bzw. (Ia) und der Aluminiumverbindung der Formel (XIV) und/oder (XV) in den gewählten Lösungsmitteln, den Konzentrationen sowie den Reaktionstemperaturen, erhält man die erfindungsgemäß im Rahmen von Verfarensschritt b) bevorzugt einzusetzenden Bis(diarylphenoxy)-Aluminium-Verbindungen in Form eines Feststoffes, einer Suspension oder einer Lösung im eingesetzten Lösungsmittel bzw. Lösungsmittelgemisch. Die so erhaltenen, erfindungsgemäß bevorzugt verwendeten Bis(diarylphenoxy)-Aluminium-Verbindungen können in der jeweils erhaltenen Form weiterverwendet oder abgetrennt und von den eingesetzten Lösungsmitteln befreit werden.

Die Isolierung kann dabei nach dem Fachmann bekannten und vorteilhaft erscheinenden Methoden erfolgen. Vorzugsweise führt man die Isolierung, Lagerung bzw. Weiterbehandlung der erfindungsgemäß bevorzugt zu verwendenden Bis(diarylphenoxy)-Aluminium-Verbindungen unter weitgehendem Ausschluss von Sauerstoff und Feuchtigkeit durch.

Zur Durchführung des erfindungsgemäß bevorzugten Verfahrens zur Herstellung von Isopulegol geht man vorteilhaft so vor, dass man zunächst eine Lösung der erfindungsgemäß verwendeten Bis(diarylphenoxy)-Aluminium-Verbindungen in einem geeigneten Lösungsmittel, wie vorstehend beschrieben, bereitstellt. Dieser Lösung setzt man dann erfindungsgemäß das zu cyclisierende racemische oder nicht-racemische Citronellal zu. Das Citronellal kann dabei als solches oder in Form einer Lösung, vorteilhaft in einem der vorstehend genannten geeigneten Lösungsmittel zugegeben werden. Im Rahmen einer bevorzugten Ausführungsform des erfindungsgemäßen Verfahrens bereitet man zunächst eine Lösung des gewählten Liganden der Formeln (I) bzw. (la) in Toluol und setzt dann, vorteilhaft unter Rühren, die gewählte Aluminium-Verbindung der Formel (XIV) und/oder (XV), bevorzugt Trimethyl- oder Triethylaluminium in toluolischer, Lösung zu.

Als Ausgangsstoff zur Durchführung des erfindungsgemäß bevorzugten Cyclisierungsverfahrens eignet sich prinzipiell Citronellal, das nach jedwedem Verfahren hergestellt sein kann. Im Rahmen der vorliegenden Erfindung setzt man bevorzugt allerdings optisch aktives Citronellal ein, wie es gemäß vorstehend beschriebenem Verfahrens schritt a) durch asymmetrische Hydrierung von Geranial und/oder Neral erhalten werden kann. Bevorzugt setzt man Citronellal ein, das eine Reinheit von etwa 90 bis etwa 99,9 Gew.-%, besonders bevorzugt von etwa 95 bis etwa 99,9 Gew.-%, aufweist.

Die Zugabe des zu cyclisierenden Citronellals erfolgt vorteilhaft bei Temperaturen im Bereich von etwa -40 °C bis etwa 40 °C, bevorzugt im Bereich von etwa -20 °C bis etwa 20 °C. Dazu wird vorteilhaft die bereitete Lösung der erfindungsgemäß verwendeten Bis(diarylphenoxy)-Aluminium-Verbindung auf eine Temperatur in diesem Bereich, z. B. auf eine Temperatur im Bereich von -10 °C bis 10 °C abgekühlt und vorgekühltes Citronellal bzw. eine vorgekühlte Lösung von Citronellal zugegeben.

Die Zugabe des Citronellals bzw. der Lösung davon kann so vorgenommen werden, dass man entweder die gesamte Menge auf einmal zusetzt oder sie portionsweise oder auch kontinuierlich zur bereiteten Katalysatorlösung gibt. Als Lösungsmittel eignen sich wiederum die vorstehend genannten Lösungsmittel, insbesondere Toluol. Bevorzugt setzt man das zu cyclisierende Citronellal als solches, d. h. ohne weiteren Zusatz von Lösungsmitteln, ein. Bei Einsatz eines Lösungsmittels wird die gesamte Lösungsmittelmenge (für Katalysatorherstellung und zur Durchführung der Cyclisierungsreaktion) vorteilhaft so gewählt, dass das volumenbezogene Verhältnis von umzusetzendem Citronellal zum Lösungsmittel etwa 2:1 bis etwa 1:20, bevorzugt von etwa 1,5:1 bis etwa 1:10 beträgt.

Das Mengenverhältnis zwischen dem umzusetzenden Citronellal und der eingesetzten Menge der erfindungsgemäß verwendeten Bis(diarylphenoxy)-Aluminium-Verbindung wird durch die Menge der zur Herstellung derselben eingesetzten Verbindungen der Formel (I) bzw. (la) und der Formel (XIV) und/oder (XV), also durch das Mengenverhältnis von eingesetztem Ligand zu eingesetzter Aluminium-Verbindung der Formel (XIV) und/oder (XV) bestimmt.

Erfindungsgemäß wählt man die Menge von umzusetzendem Citronellal zur eingesetzten Menge an Aluminium-Verbindung der Formel (XIV) und/oder (XV) so, dass das molare Verhältnis etwa 5:1 bis etwa 1000:1, bevorzugt etwa 10:1 bis etwa 500:1, besonders bevorzugt etwa 50:1 bis etwa 200:1 beträgt.

Unabhängig davon kann das Verhältnis zwischen eingesetztem Ligand der Formel (I) bzw. (la) und der eingesetzten Aluminium-Verbindung der Formel (XIV) und/oder (XV) in den vorstehend zur Herstellung der erfindungsgemäß bevorzugt zu verwendenden Bis(diarylphenoxy)-Aluminium-Verbindung genannten Grenzen variiert werden.

Die Cyclisierung von Citronallal zu Isopulegol erfolgt je nach Wahl der Reaktionspartner und Reaktionsbedingungen in der Regel rasch und ist üblicherweise nach etwa 0,5 bis etwa 10 h, oft nach etwa 5 h, weitgehend abgeschlossen. Der Reaktionsfortschritt kann durch dem Fachmann an sich bekannte Methoden, beispielsweise durch chromatographische, speziell gaschromatographische Methoden oder auch HPLC-Methoden leicht verfolgt werden.

Im Rahmen einer bevorzugten Ausführungsform des erfindungsgemäß bevorzugten Cyclisierungsverfahrens führt man die Cyclisierung von Citronellal zu Isopulegol in Gegenwart eines Hilfsstoffs (iv), beispielsweise einer Säure, vorzugsweise einer organischen Säure durch. Beispielhaft seien als vorteilhaft einsetzbare organische Säuren genannt: Essigsäure, Propionsäure, Benzoesäure, Toluolsulfonsäure, Methansulfonsäure, bevorzugt Essigsäure. Die genannten Säuren werden vorteilhaft in einer Menge von etwa 0,5 bis etwa 10 Gew.-%, bezogen auf die Menge an umzusetzendem Citronellal, eingesetzt. Vorteilhaft werden sie zusammen mit dem Citronellal, z. B. in Form eines Gemisches, dem Reaktionsgemisch zugesetzt.

In einer besonders bevorzugten Ausführungsform führt man das erfindungsgemäß bevorzugte Verfahren zur Herstellung von Isopulegol durch Cyclisierung von Citronellal in Gegenwart mindestens eines Hilfsstoffs (iv) durch, der ausgewählt ist unter Carbonsäureanhydriden, Aldehyden, Ketonen und Vinylethern.

Die Hilfsstoffe (iv) der genannten Substanzklassen können jeweils einzeln oder in Form von Gemischen untereinander eingesetzt werden. Bevorzugt setzt man im Fall von Gemischen solche ein, die aus Verbindungen einer Substanzklasse bestehen. Besonders bevorzugt setzt man einzelne Verbindungen ein. Unter wie nachfolgend beschriebenem Einsatz der genannten Verbindungen gelingt es in der Regel, die Bildung unerwünschter Nebenprodukte weitgehend zu unterdrücken.

Im Rahmen einer bevorzugten Ausführungsform führt man die vorstehend beschriebene Cyclisierung von Citronellal in Gegenwart von Bis(diarylphenoxy)-Aluminium-Verbindungen in Gegenwart eines Carbonsäureanhydrids der Formel (XVI) 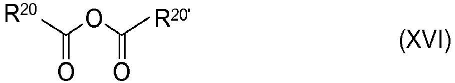 durch wobei die Reste R²⁰ und R^{20'} gleich oder verschieden, bevorzugt gleich, sein können und einen verzweigten oder unverzweigten C₁-C₁₂-Alkylrest oder C₇-C₁₂-Aralkylrest oder einen C₆-C₁₀-Arylrest bedeuten, wobei die genannten Reste jeweils einen oder mehrere, in der Regel 1 bis etwa 3, gleiche oder verschiedene Substituenten, ausgewählt aus der Gruppe OR^{10e}, SR^{10f}, NR^{8e}R^{9e} und Halogen aufweisen können und wobei R²⁰ und R^{20'} gemeinsam auch einen 5- bis 8-gliedrigen Ring bilden können, der eine oder mehrere ethylenische Doppelbindungen und ein oder mehrere gleiche oder verschiedene Heteroatome, ausgewählt aus der Gruppe O, S und NR^{11b} aufweisen kann und wobei R^{10e}, R^{10f}, R^{8e}, R^{9e} und R^{11b} die vorstehend für R¹¹ angegebenen Bedeutungen haben können.

Im Rahmen einer weiteren bevorzugten Ausführungsform führt man die Cyclisierung von Citronellal in Gegenwart (eines von Citonellal verschiedenen) Aldehyds der Formel (XVII) 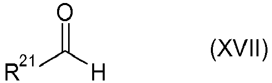 durch, wobei der Rest R²¹ einen verzweigten oder unverzweigten C₁-C₁₂-Alkylrest oder C₇-C₁₂-Aralkylrest oder einen C₆-C₁₀-Arylrest bedeutet, wobei die genannten Reste jeweils einen oder mehrere, bevorzugt 1 bis 3, gleiche oder verschiedene Substituenten, ausgewählt aus der Gruppe OR^{10e}, SR^{10f} NR^{8e}R^{9e} und Halogen aufweisen können und wobei R^{10e}, R^{10f} R^{8e} und R^{9e} die vorstehend für R¹¹ angegebenen Bedeutungen haben können.

Im Rahmen einer weiteren bevorzugten Ausführungsform führt man Cyclisierung von Citronellal in Gegenwart eines Ketons der Formel (XVIII) 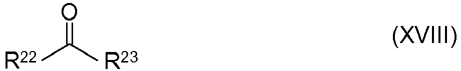 durch, wobei die Reste R²² und R²³ jeweils gleich oder verschieden sein können und einen verzweigten oder unverzweigten C₁-C₁₂-Alkylrest oder C₇-C₁₂-Aralkylrest oder einen C₆-C₁₀-Arylrest oder einen C₁-C₆-Alkoxycarbonylrest bedeuten, wobei die genannten Reste jeweils einen oder mehrere, bevorzugt 1 bis 3, gleiche oder verschiedene Substituenten, ausgewählt aus der Gruppe OR^{10e}, SR^{10f}, NR^{8e}R^{9e} und Halogen aufweisen können, und wobei R²² und R²³ gemeinsam auch einen 5- bis 8-gliedrigen Ring bilden können, der eine oder mehrere ethylenische Doppelbindungen und ein oder mehrere gleiche oder verschiedene Heteroatome, ausgewählt aus der Gruppe O, S, NR^{11b} aufweisen kann und wobei R^{10e}, R^{10f}, R^{8e}, R^{9e} und R^{11b} die vorstehend für R¹¹ angegebenen Bedeutungen haben können.

Alternativ zu den zuvor genannten Carbonylverbindungen lassen sich auch Vinylether der allgemeinen Formel (XIX) 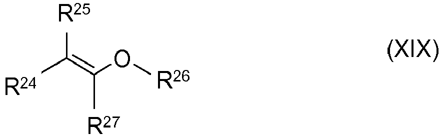 im Rahmen des erfindungsgemäß bevorzugten Cyclisierungsverfahrens einsetzen, wobei die Reste R²⁴, R²⁵, R²⁶ und R²⁷ unabhängig voneinander jeweils gleich oder verschieden sein können und einen verzweigten oder unverzweigten C₁-C₁₂-Alkylrest oder C₇-C₁₂-Aralkylrest oder einen C₆-C₁₀-Arylrest bedeuten, wobei die genannten Reste jeweils einen oder mehrere, bevorzugt 1 bis 3, gleiche oder verschiedene Substituenten, ausgewählt unter Oxo, OR^{10e}, SR^{10f}, NR^{8e}R^{9e} und Halogen aufweisen können und wobei R²⁵ und R²⁶ gemeinsam auch einen 5- bis 8-gliedrigen Ring bilden können, der eine oder mehrere ethylenische Doppelbindungen und ein oder mehrere, üblicherweise 1 oder 2, gleiche oder verschiedene Heteroatome, ausgewählt aus der Gruppe O, S, NR^{11b} aufweisen kann und wobei R^{10e}, R^{10f}, R^{8e}, R^{9e} und R^{11b} die vorstehend für R¹¹ angegebenen Bedeutungen haben können.

C₁-C₁₂-Alkyl steht dabei für wie vorstehend beschriebenes C₁-C₆-Alkyl und darüber hinaus beispielsweise für Heptyl, Octyl, Nonyl, Decyl, Undecyl oder Dodecyl. In den Fällen, in denen zwei Alkylreste gemeinsam einen Ring ausbilden, sind unter Alkylresten auch Alkylenylreste zu verstehen. C₇-C₁₂-Aralkylresten und C₆-C₁₀-Arylresten können beispielhaft die vorstehend genannten Bedeutungen zukommen. Beispielhaft seien als C₁-C₆-Alkoxycarbonylrest genannt: Methoxycarbonyl, Ethoxycarbonyl, Propoxycarbonyl und Isopropoxycarbonyl, bevorzugt Methoxycarbonyl und Ethoxycarbonyl.

Im Rahmen einer bevorzugten Ausführungsform des erfindungsgemäß bevorzugten Cyclisierungsverfahrens führt man die Cyclisierung von Citronellal in Gegenwart eines Carbonsäureanhydrids der Formel (XVI) durch, wobei die Reste R²⁰ und R^{20'} gleich sind und einen verzweigten oder unverzweigten C₁-C₁₂-Alkylrest oder C₇-C₁₂-Aralkylrest oder einen C₆-C₁₀-Arylrest bedeuten, und wobei R²⁰ und R^{20'} gemeinsam auch einen 5- bis 8-gliedrigen Ring bilden können, der eine oder mehrere ethylenische Doppelbindungen und ein oder mehrere gleiche oder verschiedene Heteroatome, ausgewählt aus der Gruppe OR^{10e}, SR^{10f}, NR^{11b} aufweisen kann und R^{10e}, R^{10f} und R^{11b} unabhängig voneinander die vorstehend für R¹¹ angegebenen Bedeutungen haben kann.

Insbesondere bevorzugt setzt man solche Carbonsäureanhydride ein, bei denen die Reste R²⁰ und R^{20'} gleich sind und einen verzweigten oder unverzweigten C₁-C₁₂-Alkylrest oder einen C₆-C₁₀-Arylrest bedeuten. Beispielhaft seien als erfindungsgemäß besonders bevorzugt einzusetzende Carbonsäureanhydride genannt: Essigsäureanhydrid, Propionsäureanhydrid, Pivalinsäureanhydrid und Benzoesäureanhydrid.

Als erfindungsgemäß ebenfalls bevorzugt einsetzbare Aldehyde der Formel (XVII) seien beispielhaft Acetaldeyhd, Propionaldehyd und Chloral (Trichloracetaldehyd) genannt.

Führt man die erfindungsgemäß bevorzugte Cyclisierung von Citronellal im Rahmen einer weiteren bevorzugten Ausführungsform in Gegenwart eines Ketons der Formel (XVIII) durch, setzt man mit Vorteil solche mit einer aktivierten, d. h. elektronenarmen, Carbonylfunktion ein. Beispielhaft seien die folgenden Ketone genannten, die sich in besonderem Maße zum Einsatz im Rahmen des erfindungsgemäßen Verfahrens eignen: 1,1,1-Trifluoraceton, 1,1,1-Trifluoracetophenon, Hexafluoraceton, Brenztraubensäuremethylester und Brenztraubensäureethylester.

Als erfindungsgemäß im Rahmen dieser Ausführungsform von Verfahrensschritt b) ebenfalls bevorzugt einsetzbare Vinylether der Formel (XIX) seien beispielhaft genannt: Methylvinylether, Ethylvinylether, Isobutylvinylether und 3,4-Dihydro-2H-pyran.

Die genannten Verbindungsklassen können gleichermaßen mit gutem Erfolg im Rahmen dieser bevorzugten Ausführungsform des erfindungsgemäßen Verfahrens eingesetzt werden. Im Hinblick auf praktische Gesichtspunkte wie beispielsweise eine höhere Reaktionsgeschwindigkeit hat sich der Einsatz von Aldehyden und/oder elektronenarmen Ketonen als vorteilhaft erwiesen.

Die Menge an bevorzugt zu verwendendem Carbonsäureanhydrid, Aldehyd, Keton und/oder Vinylether kann in breiten Grenzen variiert werden und richtet sich nach Art des eingesetzten Stoffes und dem Reinheitsgrad bzw. dem Vorhandensein noch nicht näher identifizierter Verunreinigungen. Üblicherweise setzt man die genannten Verbindungen bzw. deren Gemische in einer Menge von etwa 0,01 mol-% bis etwa 5 mol-%, bevorzugt von etwa 0,1 mol-% bis etwa 2 mol-%, bezogen auf die eingesetzte Menge an Citronellal, ein.

An die Art und Weise der Reaktionsführung, beispielsweise die Ausgestaltung von Reaktoren oder die Reihenfolge der Zugabe einzelner Reaktionspartner, sind keine besonderen Anforderungen zu stellen, solange eine Reaktionsführung unter weitgehendem Ausschluss von Sauerstoff und Wasser gewährleistet ist.

Zur Durchführung dieser bevorzugten Ausführungsform des erfindungsgemäß im Rahmen von Verfahrensschritt b) durchzuführenden Cyclisierungsverfahrens geht man vorteilhaft so vor, dass man zunächst eine Lösung der erfindungsgemäß einzusetzenden Bis(diarylphenoxy)-Aluminium-Verbindung in einem geeigneten Lösungsmittel wie vorstehend beschrieben bereitstellt. Dieser Lösung setzt man dann bevorzugt ein Gemisch des zu cyclisierenden racemischen oder nicht-racemischen, bevorzugt nichtracemischen, d.h. optisch aktiven Citronellals mit dem gewählten Carbonsäureanhydrid, dem Aldehyd, dem aktivierten Keton und/oder dem Vinylether zu. Alternativ dazu kann man beispielsweise auch die Lösung der erfindungsgemäß bevorzugt einzusetzenden Bis(diarylphenoxy)-Aluminium-Verbindung zunächst mit dem gegebenenfalls jeweils gewählten Carbonsäureanhydrid, dem Aldehyd, dem Keton und/oder dem Vinylether versetzen und im Anschluss daran das zu cyclisierende racemische oder optisch aktive Citronellal zugeben.

Es sich als vorteilhaft erwiesen, das Citronellal bzw. das Gemisch von Citronellal mit der gewählten Verbindung innerhalb eines Zeitraums von etwa 30 min bis etwa 6 h, bevorzugt innerhalb von etwa 2 h bis etwa 4 h, zur Katalysatorlösung bzw. dem Reaktionsgemisch zuzudosieren. Das Citronellal kann dabei als solches oder in Form einer Lösung, vorteilhaft in einem der vorstehend genannten geeigneten Lösungsmittel, zugegeben werden. Im Rahmen einer wiederum bevorzugten Ausführungsform des erfindungsgemäß bevorzugten Verfahrens bereitet man zunächst eine Lösung des gewählten Liganden der Formeln (I) bzw. (la) in Toluol und setzt dann, zweckmäßigerweise unter Rühren, die gewählte Aluminium-Verbindung der Formel (XIV) und/oder (XV), bevorzugt Trimethyl- oder Triethylaluminium in toluolischer Lösung zu.

Die Zugabe des zu cyclisierenden Citronellals bzw. des Gemischs von Citronellal mit dem gewählten Carbonsäureanhydrid, Aldehyd, aktivierten Keton und/oder dem Vinyl-ether erfolgt im Rahmen dieser Ausführungsform vorteilhaft bei Temperaturen im Bereich von etwa -40°C bis etwa 40°C, bevorzugt im Bereich von etwa -20°C bis etwa 20°C. Dazu wird vorteilhaft die bereitete Lösung oder Suspension der erfindungsgemäß bevorzugt einzusetzenden Bis(diarylphenoxy)-Aluminium-Verbindung auf eine Temperatur in diesem Bereich, z. B. auf eine Temperatur im Bereich von -10°C bis 10°C, abgekühlt und die weiteren Reaktanden in vorgekühlter Form zugegeben.

Die Zugabe des Gemischs von Citronellal und der gewählten weiteren Verbindung kann so vorgenommen werden, dass man entweder die gesamte Menge Citronellal auf einmal zusetzt oder sie portionsweise oder auch kontinuierlich zur bereiteten Katalysatorlösung gibt. Als Lösungsmittel eignen sich wiederum bevorzugt die vorstehend genannten Lösungsmittel, insbesondere Toluol. Bevorzugt setzt man das zu cyclisierende Citronellal in Form eines Gemisches mit dem gewählten Carbonsäureanhydrid, Aldehyd, aktivierten Keton und/oder Vinylether ohne weiteren Zusatz von Lösungsmitteln ein. Bei Einsatz eines Lösungsmittels wählt man die gesamte Lösungsmittelmenge vorteilhaft so, dass das volumenbezogene Verhältnis von umzusetzendem Citronellal zum Lösungsmittel etwa 1 : 1 bis etwa 1 : 20, bevorzugt von etwa 1 : 1 bis etwa 1 : 10 beträgt.

Es wurde gefunden, dass üblicherweise im Rahmen des vorstehend beschriebenen bevorzugten Cyclisierungsverfahrens während der Reaktion ein Teil des Katalysatorkomplexes deaktiviert wird. Dies ist unter anderem auf Ligandenaustausch-Prozesse zwischen den jeweils eingesetzten Bis(diarylphenol)-Liganden der Formel der eingesetzten Bis(diarylphenoxy)-Aluminium-Verbindungen und dem durch Cyclisierung entstehenden Isopulegol zurückzuführen. Die deaktivierte Form des Katalysators ist, in Abhängigkeit von der Wahl der eingesetzten Lösemittel, üblicherweise im Gegensatz zum aktiven polymeren Katalysator in der Reaktionsmischung löslich.

In einer bevorzugten Ausführungsform kann durch einfache physikalische Trennmethoden (z. B. durch Abfiltration oder Zentrifugation des noch aktiven Katalysators) der deaktiverte Teil des Katalysators zusammen mit der übrigen Reaktionsmischung abgetrennt werden. Der zurückgehaltene, immer noch aktive Teil des Katalysators kann gewünschtenfalls mit frischem Katalysator ergänzt werden und ohne nennenswerten Aktivitätsverlust wieder eingesetzt werden, vorzugsweise im Rahmen einer weiteren erfindungsgemäßen Cyclisierungsreaktion von Citronellal zu Isopulegol.

Alternativ kann die eingesetzte Katalysatormenge so gewählt werden, dass der gesamte eingesetzte Katalysatorkomplex im Laufe bzw. nach Beendigung der erfindungsgemäßen Cyclisierungsreaktion deaktiviert und somit löslich ist, was an einer klaren Reaktionsmischung zu erkennen ist. Dabei macht sich vorteilhaft bemerkbar, dass in diesem Falle aufgrund der vorstehend beschriebenen Ligandenaustausch-Prozesse der jeweils eingesetzte Bis(diarylphenol)-Ligand der Formel (I) ohne gesondert durchzuführende Hydrolyse freigesetzt wird.

Überraschenderweise wurde gefunden, dass Isopulegol aus den Aluminium-haltigen Reaktionsprodukten der Cyclisierung von Citronellal ohne vorherige Hydrolyse der jeweils als Katalysator eingesetzten Diarylphenoxy-Aluminium-Verbindungen (gegebenenfalls nach der destillativen Entfernung eines eingesetzten Lösungsmittels und/oder zusätzlich eingesetzter Hilfsstoffe) in hohen Reinheiten abdestilliert werden kann. Dabei bilden sich im Destillationssumpf in der Regel keine erkennbaren unerwünschten oder störenden Nebenprodukte. In einer speziellen Ausführung erfolgt vor oder während der destillativen Auftrennung in Schritt I) die Zugabe eines geeigneten, inerten, hochsiedenden Lösungsmittels. Dann erhält man im Destillationssumpf eine Lösung des Liganden der Formel (I) in dem erwärmten, jeweils eingesetzten Hochsieder.

Das erfindungsgemäß im Rahmen von Verfahrensschritt b) bevorzugt durchzuführende Cyclisierungsverfahren eignet sich, wie bereits erwähnt, in gleichem Maße zur Cyclisierung von racemischem wie auch nicht-racemischem, d. h. optisch aktivem Citronellal zu racemischem wie nicht-racemischem Isopulegol.

Daher dient das erfindungsgemäße Verfahren in einer bevorzugten Ausführungsform zur Herstellung von optisch aktivem Isopulegol durch Cyclisierung von aktivem Citronellal.

Insbesondere dient das erfindungsgemäß bevorzugte Cyclisierungsverfahren zur Herstellung von L-(-)-Ispulegol ausgehend von D-(+)-Citronellal.

Die genannten Bis(diarylphenol)-Liganden lassen sich nach erfolgtem Einsatz der daraus erhältlichen Diarylphenoxy-Aluminium-Verbindungen als Katalysatoren zur Cyclisierung von Citronellal zu Isopulegol aufgrund ihres guten Kristallisationsverhaltens in besonders vorteilhafter Weise diskontinuierlich oder bevorzugt halb- bzw. vollkontinuierlich zurückgewinnen und so für weitere Umsetzungen nutzbar machen. Eine besonders bevorzugte Ausführungsform der erfindungsgemäß im Rahmen von Verfahrensschritt b) bevorzugt durchzuführenden Cyclisierung umfasst daher ein Verfahren zur Aufarbeitung eines Aluminium-haltigen Reaktionsprodukts aus der Herstellung von Isopulegol durch Cyclisierung von Citronellal, enthaltend
i) Isopulegol,
ii) wenigstens einen Liganden der Formel (I), 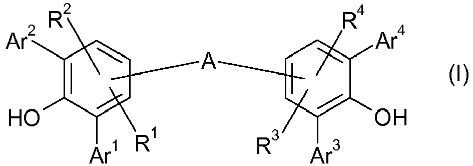 wobei die Reste Ar¹, Ar², Ar³, Ar⁴, R¹, R², R³, R⁴ und A die vorstehend genannten Bedeutungen haben können,
in freier und/oder komplexgebundener Form,
bei dem man
I) das Aluminium-haltige Reaktionsprodukt einer destillativen Auftrennung unter Erhalt eines an Isopulegol angereicherten Kopfprodukts und eines an Isopulegol abgereicherten Sumpfprodukts unterzieht,
II) das an Isopulegol abgereicherte Sumpfprodukt mit einer wässrigen Base unter Erhalt einer Aluminium-haltigen wässrigen Phase und einer die Hauptmenge der Liganden der Formel (I) enthaltenden organischen Phase in innigen Kontakt bringt,
III) den Liganden der Formel (I) aus der organischen Phase abtrennt.

In einer bevorzugten Ausführungsform der erfindungsgemäß bevorzugten, im Rahmen von Verfahrensschritt b) durchzuführenden Cyclisierung in Gegenwart der vorstehend genannten Diarylphenoxy-Aluminium-Verbindungen bevorzugt durchzuführenden Aufarbeitung erfolgt das Abtrennen des Liganden der Formel (I) aus der organischen Phase durch Kristallisation.

Die nach dem erfindungsgemäß bevorzugten Cyclisierungsverfahren erhaltenen Bis(diarylphenol)-Liganden der Formel (I) können üblicherweise ohne weitere Aufreinigungsschritte im Rahmen einer neuen Charge mit den entsprechenden Aluminiumverbindungen der Formeln (XIV) bzw. (XV), wie im Folgenden definiert, zum reaktiven Katalysatorkomplex umgesetzt werden, wobei bei derartig wiederhergestellten Katalysatorkomplexen keine bzw. keine nennenswerte Abschwächung der Reaktivität festzustellen ist.

Im Rahmen der erfindungsgemäß im Rahmen von Verfahrensschritt b) bevorzugt durchzuführenden Cyclisierung in Gegenwart der genannten DiarylphenoxyAluminium-Verbindungen und deren bevorzugter Aufarbeitung umfasst der Begriff "Ligand in freier oder komplexgebundener Form" sowohl die freie Form des Liganden als auch sämtliche denkbaren Formen, die unter den Verfahrensbedingungen in die freie Form überführbar sind. Beispielhaft hierfür seien Alkoholate des Liganden genannt, die durch die basische Hydrolyse in die freie Form des Liganden überführt werden.

Im Rahmen der vorliegenden Erfindung umfasst der Ausdruck "wässrige Base" allgemein wässrige Lösungen, deren pH-Wert größer als 7 ist. Insbesondere handelt es sich um wässrige Lösungen von Alkali- und Erdalkalimetallhydroxiden, speziell wässrige Lösungen von KOH und NaOH.

Der Ausdruck "Aluminium-haltiges Reaktionsprodukt" beschreibt im Rahmen des erfindungsgemäß bevorzugten Cyclisierungs- bzw. Aufarbeitungsverfahrens ein Reaktionsprodukt, das wenigstens eine Verbindung umfasst, die Aluminium ionisch, kovalent oder komplexgebunden enthält. Dabei handelt es sich um Verbindungen des Aluminiums, wie sie unter den Bedingungen des erfindungsgemäßen Verfahrens aus den bei der Cyclisierung von Citronellal eingesetzten Verbindungen der Formel (R¹⁴)₃₋ₚAlHₚ (XIV) oder MAlH₄ (XV), wie vorstehend definiert, resultieren.

Unter dem Ausdruck "Hauptmenge" soll im Rahmen des vorliegenden erfindungsgemäß bevorzugten Cyclisierungs- bzw Aufarbeitungsverfahrens ein prozentualer Mengenanteil an der vorhandenen Gesamtmenge einer Verbindung verstanden werden, der größer 50%, bevorzugt größer 80% und insbesondere bevorzugt größer 90% ist.

### Schritt I):

In Schritt I) des erfindungsgemäß bevorzugten Aufarbeitungsverfahrens wird das Aluminium-haltige Reaktionsprodukt aus der Herstellung von Isopulegol durch Cyclisierung von Citronellal einer destillativen Auftrennung unter Erhalt eines an Isopulegol angereicherten Kopfprodukts und eines an Isopulegol abgereicherten Sumpfprodukts unterzogen.

In einer speziellen Ausführungsform wird in Schritt I) ein höher als das Isopulegol siedendes Lösungsmittel eingesetzt. Somit kann eine unerwünschte thermische Beanspruchung des Sumpfinhalts vermieden werden. Insbesondere liegen die darin enthaltenen Liganden der Formel (I) während der Abtrennung des Isopulegols nicht frei von Lösungsmittel vor. Das höher siedende Lösungsmittel kann dem Aluminium-haltigen Reaktionsprodukt vor und/oder während der destillativen Auftrennung hinzugefügt werden. Vorzugsweise wird ein höher siedendes Lösungsmittel eingesetzt, dessen Siedepunkt unter den Bedingungen der Destillation über dem Siedepunkt des Isopulegols liegt. Bevorzugt liegt der Siedepunkt des zugeführten Lösungsmittels unter den Bedingungen der Destillation wenigstens 5°C, bevorzugt wenigstens 10°C und insbesondere wenigstens 20°C, über dem Siedepunkt des Isopulegols.

Bevorzugte höher siedende Lösungsmittel, die einen solchen Siedepunkt aufweisen, sind beispielsweise Kohlenwasserstoffe, wie Phenylcyclohexan, Benzyltoluol, Dibenzyltoluol, 1-Methylnaphthalin und Tridecan, 1-Decanol, 1,2-Propylencarbonat, Ether, wie Diethylenglycoldibutylether, Tetraethylenglycoldimethylether und Dibenzylether, sowie technische Gemische dieser Lösungsmittel. Insbesondere bevorzugt sind Gemische, die als Hauptbestandteil Phenylcyclohexan enthalten.

Bei Einsatz wenigstens eines höher siedenden Lösungsmittels wird als an Isopulegol abgereichertes Sumpfprodukt in Schritt I) eine organische Phase erhalten, umfassend das höher siedende Lösungsmittel, die Hauptmenge der Liganden der Formel (I) sowie gegebenenfalls wenigstens eine Aluminium-haltige Verbindung.

Vorzugsweise erfolgt die destillative Abtrennung von Isopulegol in Schritt I) bei einer Sumpftemperatur von vorzugsweise höchstens 250°C, bevorzugt höchstens 150°C und besonders bevorzugt höchstens 100°C. Die untere Sumpftemperatur ist in der Regel unkritisch und beträgt im Allgemeinen wenigstens 0°C, vorzugsweise wenigstens 20°C. Zur Einhaltung dieser Maximaltemperaturen kann die Destillation gewünschtenfalls unter einem geeigneten Vakuum durchgeführt werden.

Der Druck in Schritt I) des erfindungsgemäß bevorzugten Aufarbeitungsverfahren liegt unabhängig von der speziellen Ausführungsform im Allgemeinen in einem Bereich von 0,1 bis 1500 mbar, bevorzugt in einem Bereich von 1 bis 500 mbar und besonders bevorzugt in einem Bereich von 5 bis 100 mbar.

Unabhängig von der Zusammensetzung des Aluminium-haltigen Reaktionsprodukts aus der Cyclisierung von Citronellal und vom Einsatz eines höher siedenden Lösungs-mittels kann die destillative Abtrennung des Isopulegols kontinuierlich oder diskontinuierlich, vorzugsweise kontinuierlich erfolgen. In einer geeigneten Vorgehensweise wird dem Reaktionsprodukt aus der Cyclisierung von Citronellal das höher siedende Lösungsmittel vor der destillativen Auftrennung zugegeben und im Verlauf der Destillation die im Sumpf vorhandene Menge an hoch siedendem Lösungsmittel im Weiteren konstant gehalten.

Zur destillativen Auftrennung in Schritt I) können die üblichen dem Fachmann bekannten Vorrichtungen eingesetzt werden (siehe z. B. Sattler, Thermische Trennverfahren, 2. Auflage 1995, Weinheim, S. 135ff; Perry's Chemical Engineers Handbook, 7. Auflage 1997, New York, Section 13). Dazu zählen Destillationssäulen und -kolonnen, die mit Packungen, Einbauten etc. versehen sein können. Die eingesetzten Destillationssäulen können trennwirksame Einbauten enthalten, wie Trennböden, z. B. Lochböden, Glockenböden oder Ventilböden, geordnete Packungen, z. B. Blech- oder Gewebepackungen, oder regellose Schüttungen von Füllkörpern. Die in der/den eingesetzten Kolonne(n) notwendige Stufenzahl und das Rücklaufverhältnis richten sich im Wesentlichen nach den Reinheitsanforderungen und der relativen Siedelage der Bestandteile des Aluminium-haltigen Reaktionsprodukts aus der Herstellung von Isopulegol durch Cyclisierung von Citronellal und des höhersiedenden Lösungsmittels, wobei der Fachmann die konkreten Auslegungs- und Betriebsdaten nach bekannten Methoden ermitteln kann. Die destillative Auftrennung kann z. B. in einer oder mehreren miteinander gekoppelten Destillationskolonnen erfolgen.

Zur destillativen Auftrennung in Schritt I) des erfindungsgemäß bevorzugten Aufarbeitungsverfahrens eignen sich ebenfalls übliche Verdampfer, vorzugsweise Verdampfer mit Zwangsumlauf, besonders bevorzugt Fallfilmverdampfer.

In Abhängigkeit gegebenenfalls enthaltener zusätzlicher Komponenten im Aluminiumhaltigen Reaktionsprodukt aus der Cyclisierung von Citronellal kann die Zusammensetzung des bei der destillativen Auftrennung erhaltenen Kopfproduktes es erforderlich machen, dieses gegebenenfalls einem weiteren Aufarbeitungsschritt zu unterziehen.

In einer speziellen Ausführungsform des erfindungsgemäß im Rahmen von Verfahrensschritt b) bevorzugten Verfahrens zur Aufbereitung eines Aluminium-haltigen Reaktionsprodukts aus der Herstellung von Isopulegol durch Cyclisierung von Citronellal, enthält das Reaktionsprodukt zusätzlich ein niedriger siedendes Lösungsmittel (iii).

Der Ausdruck "niedriger siedendes Lösungsmittel (iii)" bezieht sich im Rahmen der vorliegenden Erfindung auf den Siedepunkt des Isopulegols. Insbesondere eignen sich hierfür solche Lösungsmittel oder Lösungsmittelgemische, die unter den Bedingungen der destillativen Auftrennung einen Siedepunkt aufweisen, der wenigstens 5°C, bevorzugt 10°C und insbesondere 20°C, unter dem des Isopulegols bei den jeweiligen Bedingungen liegt.

Bevorzugte Lösemittel mit einem solchen Siedepunkt sind im Rahmen des bevorzugten Aufarbeitungsverfahrens inerte organische Lösungsmittel oder Mischungen davon, wie beispielsweise aromatische Lösungsmittel, z. B. Toluol, Ethylbenzol oder Xylol, halogenierte Lösungsmittel, z. B. Dichlormethan , Dichlorethan oder Chlorbenzol, aliphatische Lösungsmittel, z. B. Pentan, Hexan oder Cyclohexan, Ether, z. B. Tetrahydrofuran, Diethylether, Methyl-tert-Butylether, Ester, z. B. Essigsäureethylester, oder Dimethylformamid (DMF), Dimethylsulfoxid (DMSO) und dergleichen mehr. Besonders bevorzugt handelt es sich um Toluol.

Enthält das aufzuarbeitende Aluminium-haltige Reaktionsprodukt ein solches niedriger siedendes Lösungsmittel, so wird dieses in einer geeigneten Ausführungsform vor der destillativen Abtrennung des Isopulegols zumindest teilweise aus dem Reaktionsprodukt entfernt. Die Abtrennung des niedriger siedenden Lösungsmittels erfolgt vorzugsweise ebenfalls destillativ. In Abhängigkeit vom Siedepunkt des niedriger siedenden Lösungsmittels können die üblichen zuvor genannten Destillationsvorrichtungen eingesetzt werden.

In einer weiteren geeigneten Ausführungsform erfolgt die destillative Auftrennung des Aluminium-haltigen Reaktionsprodukts in Schritt I) unter Erhalt eines an Isopulegol angereicherten Kopfprodukts, das gleichzeitig zumindest einen Teil, vorzugsweise die Hauptmenge des niedriger siedenden Lösungsmittels, enthält. In diesem Fall kann das Kopfprodukt einer weiteren Auftrennung, bevorzugt ebenfalls destillativ, unterzogen werden.

Das abgetrennte niedriger siedende Lösungsmittel wird mit Vorteil in die Cyclisierung des Citronellals zurückgeführt, in dem es als Lösungsmittel eingesetzt wird. Auf diese Weise erfordert das erfindungsgemäß bevorzugte Aufarbeitungsverfahren - bis auf Ergänzungen, die durch unvermeidliche Verluste erforderlich werden - die lediglich einmalige Bereitstellung einer Menge des niedriger siedenden Lösungsmittels.

In einer speziellen Ausführungsform des erfindungsgemäß bevorzugt durchzuführenden Verfahrens zur Cyclisierung von Citronellal zu Isopulegol sowie zur Aufbereitung eines Aluminium-haltigen Reaktionsprodukts aus der Herstellung von Isopulegol durch Cyclisierung von Citronellal, enthält das Reaktionsprodukt zusätzlich einen Hilfsstoff (iv).

Der Begriff "Hilfsstoff (iv)" bezieht sich im Rahmen von Verfahrensschritt b) der vorliegenden Erfindung auf Verbindungen, die bei der Cyclisierung von Citronellal zugesetzt werden, um unerwünschte Nebenreaktionen zu unterdrücken. Bevorzugt sind die Hilfsstoffe (iv) ausgewählt unter organischen Säuren, Carbonsäureanhydriden, Aldehyden (mit Ausnahme von Citronellal), Ketonen und Vinylethern, wie sie in der WO 2006/092433, auf die auch diesbezüglich in vollem Umfang Bezug genommen wird, sowie vorstehend beschrieben sind.

In einer weiteren speziellen Ausführungsform des vorliegenden erfindungsgemäß bevorzugten Cyclisierungs- bzw. Aufarbeitungsverfahrens sind die Hilfsstoffe (iv), wie vorstehend beschrieben, ausgewählt unter Carbonsäureanhydriden, Aldehyden (mit Ausnahme von Citronellal), Ketonen und Vinylethern.

Die Hilfsstoffe (iv) der genannten Substanzklassen können jeweils einzeln oder in Form von Gemischen in dem aufzuarbeitenden Reaktionsprodukt vorliegen. Bevorzugte Gemische sind solche, die aus Verbindungen einer Substanzklasse bestehen. Besonders bevorzugt enthält das Reaktionsprodukt einen einzelnen Hilfsstoff.

Vorzugsweise werden die im Reaktionsprodukt aus der Cyclisierung von Citronellal enthaltenen Hilfsstoffe (iv) ebenfalls zumindest teilweise entfernt und soweit wie möglich in die Cyclisierung von Citronellal zurückgeführt.

Weisen die Hilfsstoffe (iv) unter den Bedingungen der Destillation einen Siedepunkt auf, der unterhalb oder nur geringfügig, d. h. weniger als 30°C, über dem Siedepunkt des Isopulegols liegt, können diese durch Destillation aus der ausreagierten Mischung weitgehend und in dem Maße, in dem sie nicht gegebenenfalls selbst umgesetzt wurden, zurück gewonnen werden. In Abhängigkeit vom Siedepunkt des Hilfsstoffs können die üblichen zuvor genannten Destillationsvorrichtungen eingesetzt werden.

Weisen die Hilfsstoffe (iv) unter den Bedingungen der Destillation einen Siedepunkt auf, der deutlich oberhalb, d. h. wenigstens 30°C, über dem Siedepunkt des Isopulegols liegt, verbleiben diese im Sumpfprodukt und werden gegebenenfalls in Schritt II) des erfindungsgemäß bevorzugten Aufarbeitungsverfahrens entfernt, wenn ihre physikalischen Eigenschaften dies erlauben.

In einer weiteren geeigneten Ausführungsform erfolgt die destillative Auftrennung des Reaktionsprodukts in Schritt I) unter Erhalt eines an Isopulegol angereicherten Kopfprodukts, das gleichzeitig zumindest einen Teil, vorzugsweise die Hauptmenge des Hilfsstoffs (iv), enthält. Gegebenenfalls kann dieses Hauptprodukt ein niedriger siedendes Lösungsmittel, wie zuvor ausgeführt, enthalten. In diesem Fall kann das Kopfprodukt einer weiteren Auftrennung, bevorzugt ebenfalls destillativ, unterzogen werden. Der abgetrennte Hilfsstoff (iv) wird, gegebenenfalls gemeinsam mit dem niedriger siedenden Lösungsmittel, mit Vorteil in die Cyclisierung des Citronellals zurückgeführt, in der er z. B. zur Unterdrückung unerwünschter Nebenreaktionen eingesetzt wird. Auf diese Weise erfordert das erfindungsgemäß bevorzugte Aufarbeitungsverfahren - bis auf Ergänzungen, die durch unvermeidliche Verluste erforderlich werden - die lediglich einmalige Bereitstellung einer Menge des Hilfsstoffs (iv).

Die Isopulegolabtrennung, die Zufuhr des höhersiedenden Lösungsmittels und gegebenenfalls die Leichtsiederabtrennung, d. h. die Abtrennung von gegebenenfalls vorhandenen Lösungsmitteln und flüchtigen Hilfsstoffen aus der Cyclisierung von Citronellal, können auf unterschiedliche Weisen kombiniert werden:
In einer geeigneten Ausführungsform verwendet man zur Destillation eine so genannte Trennwandkolonne, d. h. Zulaufstelle und ein Seitenabzug befinden sich auf entgegen gesetzten Seiten einer Trennwand, die sich über einen Abschnitt der Längsausdehnung der Kolonne erstreckt. Derartige Destillationskolonnen, die eine Trennwand enthalten, sind dem Fachmann an sich bekannt. Sofern sich Seitenabzug und Zulauf im Bereich der Trennwand befinden, entsteht eine zu einer Brugma- oder Petlyuk-Schaltung analoge Schaltung. Derartige Destillationen unter Verwendung von Trennwandkolonnen sind in der DE-A-33 02 525 und EP-A-0 804 951 beschrieben, worauf hier in vollem Umfang Bezug genommen wird. In diesem Fall kann z. B. als Kopfprodukt eine an Leichtsiedern angereicherte Fraktion und als Seitenabzug einen den Hauptteil an Isopulegol enthaltenden Strom abgezogen werden. Das höher siedende Lösungsmittel wird unterhalb der Zulaufstelle, bevorzugt in den Sumpf der Kolonne oder kurz oberhalb des Sumpfes, zugefahren. Eine Lösung der Hauptmenge des Liganden der Formel (I) in dem höhersiedenden Lösungsmittel fällt als Sumpfprodukt an. In einer alternativen Ausführungsform verwendet man zur Destillation gekoppelte Kolonnen. Diese Ausführungsform kann von Vorteil sein, wenn das Reaktionsprodukt der Cyclisierung von Citronellal ein Lösungsmittel und/oder einen flüchtigen Hilfsstoff enthält, wie im Folgenden näher ausgeführt.

In diesem Fall können Gemische aus Isopulegol und niedriger oder geringfügig höher siedenden Lösungsmitteln und/oder Hilfsstoff (iv) das Kopfprodukt der ersten Kolonne bilden und in der zweiten Kolonne einer Auftrennung unter Erhalt eines zumindest die Hauptmenge des Isopulegols enthaltenden Stroms und eines an Isopulegol abgereicherten, die niedriger siedenden Lösungsmittel und/oder Hilfsstoffe der Cyclisierung enthaltenden Stroms, unterzogen werden.

Ströme, die niedriger siedende Lösungsmittel (iii) und Hilfsstoff (iv) der erfindungsgemäß bevorzugten Cyclisierung enthalten, können in der Regel ohne weitere Auftrennung in die Cyclisierung zurückgeführt werden.

Die Liganden der Formel (I) fallen, gegebenenfalls in Form ihrer Komplexe oder anderer Derivate, als Sumpfprodukt der ersten Kolonne an.

### Schritt II):

In Schritt II) des im Rahmen von Verfahrensschritt b) erfindungsgemäß bevorzugt durchzuführenden Aufarbeitungsverfahrens wird das an Isopulegol abgereicherte Sumpfprodukt mit einer wässrigen Base unter Erhalt einer Aluminium-haltigen wässrigen Phase und einer die Hauptmenge der Liganden der Formel (I) enthaltenden organischen Phase in innigen Kontakt gebracht. Bevorzugte wässrige Basen sind die zuvor genannten.

Das in Schritt I) erhaltene, an Isopulegol abgereicherte Sumpfprodukt kann neben dem Liganden der Formel (I) in freier oder komplexgebundener Form wenigstens eine weitere schwerflüchtige Komponente enthalten. Dazu zählen z. B. in Schritt I) zugesetzte höher siedende Lösungsmittel, die Umsetzungsprodukte der zur Cyclisierung von Citronellal zu Isopulegol bevorzugt eingesetzten Aluminium-haltigen Verbindungen sowie gegebenenfalls in Schritt I) nicht abgetrennte Hilfsstoffe (iv). Da sich Aluminiumhaltige Komponenten und/oder die Hilfsstoffe (iv) insbesondere bei einem kontinuierlichen Verfahren anreichern und sich speziell auf die Ausbeute und Reinheit des Abtrennens in Schritt III) negativ auswirken, ist es vorteilhaft, diese Verbindungen möglichst vollständig zu entfernen. Dies gilt speziell für die Aluminium-haltigen Verbindungen.

Das Inkontaktbringen in Schritt II) erfolgt vorzugsweise durch Extraktion. Die Zahl der Extraktionsstufen liegt vorzugsweise in einem Bereich von 1 bis 20 Stufen.

Als Extraktionsmittel dienen die zuvor genannten wässrigen Basen. Daher werden diese Ausdrücke im Rahmen der vorliegenden Erfindung synonym verwendet.

Zur Extraktion bringt man das an Isopulegol abgereicherte Sumpfprodukt aus Schritt I) mit einer wässrigen Base innig in Kontakt. Nach Trennung der Phasen erhält man eine die Hauptmenge des Liganden der Formel (I) enthaltende Phase und eine an Aluminium-haltigen Verbindungen angereicherte wässrige Phase. Anschließend entfernt man die wässrige Phase. Das Inkontaktbringen kann kontinuierlich oder diskontinuierlich erfolgen.

Zur diskontinuierlichen Durchführung bringt man unter mechanischer Bewegung, z. B. durch Rühren, das an Isopulegol abgereicherte Sumpfprodukt aus Schritt I) und das wässrige Extraktionsmittel in einem geeigneten Gefäß in Kontakt, lässt das Gemisch zur Phasentrennung ruhen und entfernt eine der Phasen, indem man zweckmäßigerweise die dichtere Phase am Boden des Gefäßes abzieht.

Mehrere diskontinuierliche Trennoperationen können kaskadenartig hintereinander durchgeführt werden, wobei die von der wässrigen Phase abgetrennte, die Hauptmenge des Liganden der Formel (I) enthaltende Phase jeweils mit einer frischen Portion des wässrigen Extraktionsmittels in Kontakt gebracht wird und/oder das wässrige Extraktionsmittel im Gegenstrom geführt wird.

Vorzugsweise erfolgt die Extraktion kontinuierlich. Zur kontinuierlichen Durchführung der Extraktion führt man das wässrige Extraktionsmittel und den Strom des an Isopulegol abgereicherten Sumpfprodukts aus Schritt I) geeigneten Apparaturen in analoger Weise zur diskontinuierlichen Variante kontinuierlich zu. Gleichzeitig wird der Apparatur, in der die Trennung der Phasen stattfindet, ein Austrag der die Hauptmenge des Liganden der Formel (I) enthaltenden Phase und ein Austrag der an Aluminiumhaltigen Verbindungen angereicherten wässrigen Phase kontinuierlich entnommen.

Die Extraktion erfolgt mindestens einstufig, z. B. in einer Mischer-Abscheider-Kombination. Geeignete Mischer sind sowohl dynamische als auch statische Mischer. Eine Extraktion in mehreren Stufen erfolgt beispielsweise in mehreren Mischer-Abscheidern oder Extraktionskolonnen.

In einer geeigneten Ausführungsform wird zur Verbesserung der Phasentrennung wenigstens eine Koalesziervorrichtung eingesetzt. Diese ist vorzugsweise ausgewählt unter Koaleszierfiltern, Elektrokoaleszern und Kombinationen davon. Beim Einsatz von Mischer-Abscheider-Vorrichtungen zur Extraktion hat sich der Einsatz von Koaleszierfiltern, wie Kerzen- oder Sandfiltern, als vorteilhaft zur Verbesserung der Phasentrennung herausgestellt. Der Filter kann dabei direkt nach dem Mischer (Rührbehälter) und/oder im organischen Ablauf des Abscheiders installiert werden. Des Weiteren bevorzugt zur Verbesserung der Phasentrennung ist der Einsatz von Elektrokoaleszern. Diese haben sich zur Abtrennung wässriger Fremdphasen von bis zu 5 Massen-% bewährt. Der Einsatz von Koalesziervorrichtungen eignet sich in dem erfindungsgemäß bevorzugten Aufarbeitungsverfahren auch vorteilhaft zur Abscheidung von feindispergierter wässriger Phase aus dem die Hauptmenge des Liganden der Formel (I) enthaltenden organischen Austrag einer Extraktionskolonne.

In einer geeigneten Ausgestaltung erfolgt die Extraktion in wenigstens einer Mischer-Abscheider-Kombination für die Extraktion Aluminium-haltiger Komponenten aus dem an Isopulegol abgereicherten Sumpfprodukt aus Schritt I). Die Verwendung einer weiteren Mischer-Abscheider-Kombination ist insbesondere vorteilhaft, um Anteile des Liganden der Formel (I) oder gegebenenfalls des höhersiedenden Lösungsmittels, die gegebenenfalls mit den abzutrennenden Aluminium-haltigen Verbindungen teilweise in das Extraktionsmittel übergehen, nachträglich zu re-extrahieren und somit in das Verfahren zurückzuführen.

Unter bestimmten Umständen kann es vorteilhaft sein, die die Hauptmenge an Liganden der Formel (I) enthaltende organischen Phase vor dem Abtrennen des Liganden in Schritt III) oder im Anschluss an das Abtrennen einem Trocknungsschritt zu unterziehen. Geeignete Trocknungsverfahren sind die üblichen, dem Fachmann bekannten, insbesondere die Adsorption an Wasser entziehenden Mitteln, z. B. unter Verwendung eines zeolithischen Molekularsiebs.

In einer alternativen Ausführungsform des erfindungsgemäß im Rahmen von Verfahrensschritt b) bevorzugten Aufarbeitungsverfahrens wird, nach dem Inkontaktbringen des an Isopulegol abgereicherten Sumpfprodukts mit der wässrigen Base, das Wasser vollständig oder wenigstens teilweise destillativ entfernt.

Um ein frühzeitiges Abtrennen, speziell durch Kristallisation, des Liganden der Formel (I) zu verhindern, sollte zu keinem Zeitpunkt während Schritt II) die Löslichkeit des Liganden in der organischen Phase überschritten werden. Dies kann durch geeignete Wahl der Temperatur und/oder der Menge und Art gegebenenfalls zugegebener Lösungsmittel erfolgen.

Demzufolge wird in einer bevorzugten Ausführungsform des erfindungsgemäß bevorzugten Aufarbeitungsverfahrens ein Austrag des erwärmten Sumpfprodukts aus Schritt I) mit einer erwärmten wässrigen Base in innigen Kontakt gebracht.

Der Ausdruck "erwärmt" bezeichnet im Rahmen des vorliegenden Aufarbeitungsverfahrens eine Temperatur oberhalb der Raumtemperatur und unterhalb der jeweiligen Siedepunktstemperaturen der wässrigen oder organischen Lösungen bei den jeweiligen Reaktionsbedingungen. Insbesondere bezeichnet der Ausdruck "erwärmt" eine Temperatur im Bereich von 25°C bis 150°C, speziell im Bereich von 70°C bis 100°C.

In Abhängigkeit der gegebenenfalls bei der erfindungsgemäß bevorzugten Cyclisierung von Citronellal in Gegenwart von Diarylphenoxy-Aluminium-Verbindungen verwendeten Hilfsstoffe kann das an Isopulegol abgereicherte Sumpfprodukt gegebenenfalls weitere in Schritt I) nicht abgetrennte Komponenten enthalten. Diese werden vorzugsweise in Schritt II) abgetrennt. In diesem Fall kann man die erhaltene wässrige Phase einem geeigneten Trennverfahren unterziehen, um diese Komponenten, z. B. einen Hilfsstoff (iv), zurückzugewinnen.

### Schritt III):

In Schritt III) des erfindungsgemäß im Rahmen von Verfahrensschritt b) bevorzugten Aufarbeitungsverfahrens wird der Ligand der Formel (I) aus der in Schritt II) erhaltenen, die Hauptmenge des Liganden enthaltenden organischen Phase durch Kristallisation abgetrennt, wobei Schritt III) kontinuierlich oder diskontinuierlich ausgeführt werden kann. Geeignete Ausführungsformen dieses Schritts sind beispielsweise Kristallisation und/oder vollständiges oder wenigstens teilweises destillatives Entfernen volatiler Bestandteile.

In einer bevorzugten Ausführungsform des erfindungsgemäß bevorzugten Cyclisierungs- bzw. Aufarbeitungsverfahrens erfolgt das Abtrennen des Liganden der Formel (I) durch Kristallisation.

Zur Kristallisation des Liganden der Formel (I) muss zunächst die Löslichkeit des Liganden der Formel (I) in der organischen Phase aus Schritt II) überschritten werden. Dies kann beispielsweise durch einen Abkühlungsprozess der organischen Phase oder durch (teilweises) destillatives Abtrennen des Lösungsmittels erfolgen. Verfahren hierzu sind dem Fachmann bekannt. Zur technischen Ausgestaltung der erfindungsgemäß im Rahmen des bevorzugten Aufarbeitungsverfahrens bevorzugten Kristallisation sind beispielsweise übliche Kühlungskristallisatoren, Verdampfungskristallisatoren, Vakuumkristallisatoren, Kristallisierrinnen oder Sprühkristallisatoren geeignet.

In einer bevorzugten Ausführungsform erfolgt die bevorzugt durchzuführende Kristallisation des Liganden der Formel (I) durch Abkühlen der organischen Phase aus Schritt II) des Verfahrens. Im Allgemeinen erfolgt die Kristallisation bei einer Temperatur im Bereich von -50°C bis 100°C, bevorzugt im Bereich von -20°C bis 50°C und speziell in einem Bereich von 10°C bis 40°C.

Dieser Prozess kann durch Hinzufügen von Impfkristallen beschleunigt werden.

Der kristalline Ligand der Formel (I) kann beispielsweise durch Filtration, Flotation, Zentrifugation oder Siebung aus der Lösung isoliert werden.

Der so zurückerhaltene Ligand der Formel (I) kann gegebenenfalls durch geeignete Trocknungsverfahren getrocknet werden. Verfahren hierfür sind dem Fachmann bekannt. Beispielsweise sind zur technischen Ausgestaltung der Trocknung übliche Walzentrockner, Tellertrockner, Kammertrockner, Fließbetttrockner oder Strahlungstrockner geeignet.

Die an Ligand der Formel (I) abgereicherte organische Phase kann dem Verfahren erneut vor oder während Schritt I) zugeführt werden.

In einer geeigneten Ausführungsform des erfindungsgemäß bevorzugten Aufarbeitungsverfahrens erfolgt die Kristallisation bei Abkühlen auf Raumtemperatur aus einer erwärmten, gesättigten, in Schritt II) erhaltenen organischen Phase.

Das Verfahren eignet sich in besonderem Maße zur Herstellung von racemischem Isopulegol ausgehend von racemischem Citronellal wie auch zur Herstellung von optisch aktivem Isopulegol, bevorzugt L-Isopulegol durch Cyclisierung von entsprechendem optisch aktivem Citronellal. Die Cyclsierung unter den genannten Bedingungen in Gegenwart der im Rahmen des erfindungsgemäßen Verfahrens bevorzugten, wie vorstehend beschriebenen Diarylphenoxy-Aluminium-Verbindungen verläuft in der Regel hoch diastereoselektiv und unter weitgehendem Erhalt der stereochemischen Information, d.h. des Enantiomerenüberschusses des eingesetzten Citronellals, bevorzugt des eingesetzten D-Citronellals.

Das so durch die wie vorstehend dargestellte Cyclisierung von Citronellal zugängliche Isopulegol kann im Anschluss durch geeignete Trenn- bzw. Reinigungsverfahren insbesondere durch Destillation weiter aufgereinigt und von unerwünschten Verunreinigungen oder Nebenprodukten zumindest weitgehend befreit werden. Zur Durchführung einer derartigen destillativen Aufreinigung eignet sich in besonderem Maße eine Trennwandkolonne oder eine Zusammenschaltung zweier thermisch gekoppelter Kolonnen mit Seitenabzug, wobei das Isopulegol in aufgereinigter bzw. angereicherter Form am Seitenabzug in flüssiger Form gewonnen werden kann. Unter Einsatz einer Trennwandkolonne mit einer Gesamtanzahl von etwa 30 bis etwa 200, bevorzugt etwa 45 bis etwa 90 theoretischen Trennstufen und einer oder mehrerer, bevorzugt einer oder zwei Seitenabzugsstellen gelingt es, bei geeigneter Wahl von Druck und Temperatur der Destillation üblicherweise Isopulegol mit hoher Reinheit, oft mit einer Reinheit von 97 Gew.-% und darüber, bevorzugt von 98 Gew.-% und darüber zu isolieren. Vorteilhaft wird dabei bei absoluten Drücken in der Kolonne von 10 bis 500 mbar, bevorzugt bei 50 bis 200 mbar gearbeitet. Im Rahmen einer bevorzugten Ausführungsform reingt man das durch Cylisierung von Citronellal erhaltene Isopulegol destillativ auf, wobei man die Aufreinigung in einer Trennwandkolonne mit 30 bis 200 theoretischen Trennstufen und einer oder mehrerer Seitenabzugsstellen bei einem absoluten Betriebsdruck von 10 bis 500 mbar durchführt.

Spezielle Ausgestaltungsformen derartiger Stofftrennungen durch Destillation mittels einer Trennwandkolonne sind der vorstehenden Beschreibung der destillativen Trennung von Geranial- und Neral-haltigen Stoffgemischen sowie der nachstehend beschriebenen Feindestillation von Menthol zu entnehmen. Aufgrund des Festpunktes von Isopulegol unterhalb 14°C (entspricht in etwa dem Festpunkt von reinem L-Isopulegol) sind gegebenenfalls dem Fachmann bekannte Maßnahmen zu ergreifen, um ein unerwünschtes Festwerden der Austräge der Trennwandkolonne bei tieferen Umgebungstemperaturen, beispielsweise in der kälteren Jahreszeit zu vermeiden.

### Verfahrensschritt c): Aufreinigung von Isopulegol durch Kristallisation

Gemäß Verfahrensschritt c) des erfindungsgemäßen Verfahrens führt man eine Aufreinigung von wie vorstehend beschrieben gemäß Schritt b) des erfindungsgemäßen Verfahrens zugänglichem Isopulegol durch Kristallisation durch.

Die Kristallisation von Isopulegol ist dem Fachmann bekannt und beispielsweise in der US 5,663,460 offenbart. Das Schutzrecht beschreibt die Aufreinigung von (-)-n-Isopulegol durch Kristallisation aus Petrolether oder vorteilhaft aus Aceton bei Temperaturen von -20°C bis -60°C. Dabei kann auch eine Steigerung der optischen Reinheit erreicht werden.

Daneben ist aus der US 3,218,361 ein Verfahren zur Kristallisation von Isopulegol aus Isopulegol und Diastereomere des Isopulegols enthaltenden Stoffgemischen bekannt. Die Kristallisation wird dabei bei Temperaturen unterhalb von 0°C, vorzugsweise unterhalb von -30°C und beispielhaft bei -65°C durchgeführt und kann sowohl aus der Lösung als auch aus der Schmelze durchgeführt werden.

Die WO 2007/023109, auf die hiermit vollumfänglich Bezug genommen wird und deren Offenbarung einschließlich aller Bevorzugungen und Ausführungsformen als Bestandteil der vorliegenden Offenbarung zu betrachten ist, offenbart ein Verfahren zur Herstellung von angereichertem Isopulegol speziell von angreichertem L-Isopulegol der Formel (XX) 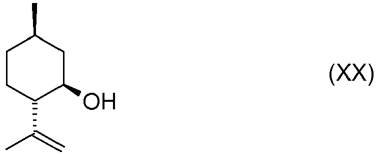 durch Kristallisation aus einer L-Isopulegol der Formel (XX) enthaltenden Schmelze.

Ein derartiges Verfahren zur Aufreinigung von Isopulegol, speziell von optisch aktivem L-Isopulegol durch Schmelzekristallisation stellt ein bevorzugtes Verfahren zur Aufreinigung von Isopulegol durch Kristallisation gemäß Verfahrensschritt c) des erfindungsgemäßen Verfahrens dar.

Als Ausgangsstoffe zur Durchführung der Kristallisation gemäß Verfahrensschritt c) eignen sich Schmelzen, die racemisches oder optisch aktives, bevorzugt optisch aktives Isopulegol der Formel (XX) bevorzugt in Form seines L-Enantiomeren L-Isopulegol, wie es in Formel (XX) in seiner absoluten Konfiguration dargestellt ist, enthalten.

Der Verfahrensschritt c) der vorliegenden Erfindung betrifft demnach ein Verfahren zur Reinigung von Isopulegol durch Kristallisation aus einer Schmelze, die neben Isopulegol noch andere, unerwünschte Verunreinigungen oder Verbindungen, beispielsweise Nebenprodukte, die bei der Herstellung des eingesetzten Isopulegols angefallen sind, enthält, jedoch im wesentlichen frei von Lösungsmitteln ist.

Unter dem Begriff "angereichertes Isopulegol" ist im Rahmen der gemäß Verfahrensschritt c) der vorliegenden Erfindung durchzuführenden Kristallisation solches Isopulegol zu verstehen, das einen höheren Gehalt an L-Isopulegol aufweist, als das als Ausgangsmaterial zur Durchführung der Kristallisation, bevorzugt der Schmelzekristallisation dienende Material. Insbesondere ist im Rahmen von Verfahrensschritt c) unter dem Begriff "angereichertes Isopulegol" solches zu verstehen, das eine chemische Reinheit von mindestens etwa 90 Gew.-%, bevorzugt mindestens etwa 95 Gew.-% und besonders bevorzugt etwa 95 bis etwa 99,95 Gew.-% aufweist. Dabei kann Isopulegol der Formel (XX), im Folgenden auch als n-Isopulegol bezeichnet, im Gemisch mit einem oder mehreren der drei weiteren möglichen Diastereomere des Isopulegols, nämlich, Iso-Isopulegol der Formel (XXI), Neo-Isopulegol der Formel (XXII) und Neoiso-Isopulegol der Formel (XXIII) vorliegen.

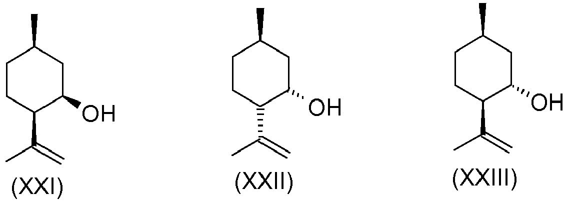

Als Ausgangsstoff zur Durchführung des erfindungsgemäßen Verfahrens eignet sich Isopulegol jeglichen Ursprungs, d.h. aus natürlichen Quellen isoliertes Isopulegol oder synthetisch hergestelltes Isopulegol, bevorzugt jedoch synthetisch hergestelltes Isopulegol, insbesondere solches wie es gemäß vorstehend beschriebenem Verfahrensschritt b) erhalten werden kann. Die erfindungsgemäß bevorzugt einzusetzende Schmelze besteht bevorzugt zu mindestens etwa 70 Gew.-%, besonders bevorzugt zu mindestens etwa 75 Gew.-%, ganz besonders bevorzugt zu etwa 80 bis etwa 100 Gew.-% und insbesondere bevorzugt zu etwa 85 bis etwa 100 Gew.-% aus Isopulegol und dessen Diastereomeren Iso-Isopulegol der Formel (XXI), Neo-Isopulegol der Formel (XXII) und Neoiso-Isopulegol der Formel (XXIII).

Eine bevorzugte Ausführungsform der Kristallisation gemäß Schritt c) des erfindungsgemäßen Verfahrens betrifft ein Verfahren zur Herstellung von enantio- und/oder diastereomerenangereichertem n-Isopulegol der Formel (XX) durch Kristallisation aus einer n-Isopulegol der Formel (XX) und gegebenenfalls weitere Diastereomere des Isopulegols enthaltenden Schmelze.

Das durch die Kristallisation gemäß Schritt c) des erfindungsgemäßen Verfahrens aus der Schmelze erhältliche n-Isopulegol der Formel (XX) fällt üblicherweise in diastereomerenangereicherter Form an. Unter dem Begriff diastereomerenangereichert ist dabei zu verstehen, dass die im Rahmen der Kristallisation erhältlichen Produkte einen höheren Gehalt des gewünschten Diastereomeren n-Isopulegol relativ zu den übrigen, vorstehend genannten Diastereomeren aufweisen, als die erfindungsgemäß bevorzugt eingesetzte Schmelze. Insbesondere ist unter dem Begriff diastereomerenangereichertes Isopulegol solches zu verstehen, das zu mindestens 80 bis 99,9 Gew.-%, bevorzugt 90 bis 99,8 Gew.-% und besonders bevorzugt 95 bis 99,5 Gew.-% n-Isopulegol der Formel (XX) neben zusammen bis zu 20 Gew.-%, bevorzugt bis zu 10 Gew.-%, besonders bevorzugt 5 bis 0,5 Gew.-% der weiteren Diastereomere der Formeln (XXI), (XXII) und/oder (XXIII) enthält.

Bei Einsatz optisch aktiver Ausgangsstoffe, d.h. Ausgangsstoffen, bei denen die beiden Enantiomere des n-Isopulegols nicht im gleichen Verhältnis vorliegen, erhält man im Rahmen einer bevorzugten Ausführungsform des Kristallisationsverfahrens gemäß Schritt c) enantiomerenangereichertes n-Isopulegol. Unter dem Begriff enantiomerenangereichert ist dabei zu verstehen, dass die erfindungsgemäß erhältlichen Produkte einen höheren Gehalt eines Enantiomeren des n-Isopulegols relativ zum anderen Enantiomeren, d.h. einen höheren Enantiomerenüberschuss (ee) aufweisen, als die erfindungsgemäß eingesetzte Schmelze.

Das erfindungsgemäß gemäß Schritt c) durchzuführende Kristallisationsverfahren ermöglicht demnach auch die Herstellung von enantio- und diastereomerenangereichertem n-Isopulegol durch Kristallisation aus einer optisch aktives n-Isopulegol mit geringerem Enantiomerenüberschuss enthaltenden Schmelze.

Erfindungsgemäß bevorzugte Ausgangsstoffe bzw. deren Schmelzen enthalten n-Isopulegol mit einem Enantiomerenüberschuss von mindestens etwa 75%, besonders bevorzugt von mindestens etwa 80% ee, ganz besonders bevorzugt von etwa 85 bis etwa 95% ee und insbesondere bevorzugt von 85 bis 90 % ee.

Bei Einsatz wie vorstehend beschriebener optisch aktiver Ausgangsstoffe erhält man im Rahmen des Kristallisationsverfahrens gemäß Schritt c) des erfindungsgemäßen Verfahrens bei geeigneter Wahl der Verfahrensparameter üblicherweise enantiomerenangereichertes n-Isopulegol der Formel (XX) mit einem Enatiomerenüberschuss von mindestens etwa 85%, bevorzugt etwa 90 bis etwa 100% ee, besonders bevorzugt etwa 95 bis etwa 99,9% ee und ganz besonders bevorzugt etwa 97 bis etwa 99,9% ee.

Daneben erhält man bei Einsatz von wie vorstehend beschriebenen optisch aktiven Ausgangsstoffen neben dem vorstehend beschriebenen enantiomerenangereicherten Isopulegol auch enantiomerenabgereichertes Isopulegol, wobei unter dem Begriff enantiomerenabgereichertes Isopulegol solches Isopulegol zu verstehen ist, das einen geringeren Enantiomerenüberschuss aufweist, als das in der Kristallisation gemäß Schritt c) eingesetzte Isopulegol. Das so erhaltene enantiomerenabgereicherte Isopulegol weist üblicherweise einen geringen Enantiomerenüberschuß von bis zu etwa 20% auf, entsprechend einem molaren Verhältnis der Enantiomere L-Isopulegol zu D-Isopulegol von etwa 60 zu 40. Bevorzugt weist das so erhaltene enantiomerenabgereicherte Isopulegol einen Enantiomerenüberschuss von bis zu 15% ee, bevorzugt von bis zu 10% ee, besonders bevorzugt von bis zu 7% ee und ganz besonders bevorzugt von bis zu 5% ee auf.

Die gemäß Verfahrensschritt c) des erfindungsgemäßen Verfahrens bevorzugt durchzuführende Schmelzekristallisation kann beispielsweise in Form einer Schichtkristallisation oder in Form einer Suspensionskristallisation durchgeführt werden. Zur Durchführung einer Schichtkristallisation bringt man üblicherweise eine gekühlte Fläche in die Schmelze des als Ausgangsstoff eingesetzten, gegebenenfalls optisch aktiven Isopulegols ein. Daraufhin bildet sich auf der eingebrachten, gekühlten Oberfläche eine Kristallschicht aus gegebenenfalls enantiomeren- und/oder diastereomerenangereichertem Isopulegol, die anschließend von der verbleibenden Mutterschmelze getrennt werden kann. Das so gewonnene kristalline angereicherte Isopulegol kann nach weiteren, hilfsstofffreien Reinigungsschritten (z.B. durch Waschen mit Reinprodukt, "Schwitzen" knapp unter dem Schmelzpunkt) wiederum aufgeschmolzen werden. Anschließend kann diese Operation zu Erhöhung der Reinheit und der Ausbeute am aufgeschmolzenen Kristallisat und an der Mutterschmelze beliebig oft wiederholt werden. Generell sind bei der im Rahmen von Verfahrensschritt c) des erfindungsgemäßen Verfahrens vorteilhaft durchzuführenden Schichtkristallisation die dynamischen von den statischen Verfahren zu unterscheiden. Bei den dynamischen Verfahren wird die Mutterphase, d.h. der geschmolzene Ausgangsstoff üblicherweise aktiv oder passiv am Kristallisat bzw. der Kühlfläche entlang bewegt. Im Rahmen der statischen Verfahren führt man die erfindungsgemäß bevorzugt durchzuführende Schmelzekristallisation in einer ruhenden Schmelze durch.

Die erfindungsgemäß bevorzugt durchzuführende Schmelzekristallisation gemäß Schritt c) lässt sich auch in Form einer dynamischen Schichtkristallisation durchführen. Im Rahmen einer wiederum bevorzugten Ausführungsform führt man diese Variante in Rohrbündelwärmetauschern durch, wie sie in G.F. Arkenbout, Melt Crystallization Technology, Lancater/PA, Technomic Publ. Co., 1995 (Kap. 6.2) beschrieben sind. Dabei werden Schmelze und Kältemittel z.B. in Form eines Rieselfilms an den Innenund Außenwänden der Wärmetauscher entlang geführt. Eine derartige Vorrichtung erlaubt eine erleichterte Abtrennung des erhaltenen kristallinen Isopulegols von der Mutterschmelze bzw. gegebenenfalls erhaltenen Schwitzfraktionen durch einfaches Ablaufen unter Schwerkrafteinfluss und bedarf, außer einer Umwälzpumpe, keiner weiteren Rührorgane.

Zur Durchführung einer dynamischen Schichtkristallisation füllt man das als Ausgangssubstanz dienende, gegebenenfalls optisch aktive Isopulegol üblicherweise mit einer aus dem Schmelzdiagramm ablesbaren Temperatur oberhalb seines Schmelzpunktes in den wie zuvor beschriebenen Schmelzekristallisator ein und führt es durch Umpumpen durch den gekühlten Rohrbündelwärmetauscher. Zur Erzielung eines vorteilhaften Kristallisationsergebnisses wird die Absenkung der Kälteträgertemperatur vorzugsweise so gewählt, dass sich innerhalb eines Zeitraumes von etwa 0,5 h bis etwa 10 h, bevorzugt innerhalb von etwa 1 h bis etwa 4 h eine Kristallschicht einer Stärke von etwa 1 mm bis etwa 50 mm, bevorzugt etwa 5 mm bis etwa 20 mm bildet. Die hierfür erforderlichen Kältemitteltemperaturen liegen in der Regel um etwa 1 K bis etwa 40 K, bevorzugt um etwa 5 K bis etwa 20 K unterhalb der jeweiligen Schmelzetemperatur.

Nach Durchführung der dynamischen Schichtkristallisation wird die verbleibende Mutterschmelze üblicherweise abgelassen. Durch Anheben der Temperatur des Heiz- bzw. Kühlmediums des Wärmetauschers können gegebenenfalls anhaftende Mutterschmelzenreste bzw. gegebenenfalls eingeschlossene Verunreinigungen aufgeschmolzen bzw. durch Drainage entfernt werden. Vorteilhafte Wärmeträgertemperaturen liegen im Bereich von etwa 15°C bis etwa 60°C, besonders vorteilhaft von etwa 20 bis etwa 30°C. Bei diesem als "Schwitzen" zu bezeichnenden Prozess können je nach Reinheitsanforderungen etwa 1 bis etwa 50 Gew.-%, oft etwa 5 bis etwa 20 Gew.-% des kristallisierten Isopulegols wieder aufgeschmolzen werden. Abschließend wird die verbleibende enantio- bzw. diastereomerenangereicherte Kristallschicht vorteilhaft abgeschmolzen und entweder ihrer weiteren Verwendung zugeführt oder nochmals zur weiteren Aufreinigung bzw. Steigerung des Enatiomeren- bzw. Diastereomerenüberschusses kristallisiert. Die wie beschrieben abgetrennte Mutterschmelze und die durch "Schwitzen" freigesetzte Fraktion können zur Erhöhung der Ausbeute in das erfindungsgemäße Verfahren zurückgeführt werden. Alternativ besteht die Möglichkeit, vor dem "Schwitzen" der Kristallschicht diese durch Inkontaktbringen mit aufgeschmolzenem Reinprodukt zu waschen, also von gegebenenfalls fest anhaftender Mutterlauge zu befreien. Aus der so zugänglichen Mutterlauge ist im Fall des Einsatzes von optisch aktiven Ausgangsstoffen enatiomerenabgereichertes oder racemisches Isopulegol zugänglich.

Die im Rahmen von Schritt c) des erfindungsgemäßen Verfahrens bevorzugte Kristallisation von Isopulegol bzw. n-Isopulegol aus der Schmelze wird vorteilhaft bei Temperaturen im Bereich von etwa -20°C bis etwa 15°C, bevorzugt im Bereich von etwa - 10°C bis etwa 15 °C und besonders bevorzugt im Bereich von etwa -5°C bis etwa 14°C durchgeführt. Die genaue Lage des Temperaturbereiches hängt dabei von der optischen und chemischen Eingangsreinheit des Ausgangsmaterials und der gewünschten Ausbeute ab und kann vom Fachmann aus dem Schmelzdiagramm des jeweils eingesetzten Isopulegols abgelesen werden.

Im Falle des im Rahmen von Schritt c) des erfindungsgemäßen durchzuführenden Herstellverfahrens für angereichertes, bevorzugt enantiomeren- bzw. diastereomerenangereichertes Isopulegol können alle genannten Methoden mit gutem Erfolg eingesetzt werden. Im Rahmen einer bevorzugten Ausführungsform des erfindungsgemäßen Verfahrens führt man die Kristallisation in Form einer statischen Schichtkristallisation, d.h. in einem statischen Schichtkristallisator mit internen Wärmetauscherflächen durch.

An die Anordnung der genannten Wärmetauscherflächen sind dabei keine besonderen Anforderungen zu stellen. Üblicherweise füllt man das als Ausgangssubstanz dienende Isopulegol mit einer aus dem Schmelzdiagramm ablesbaren Temperatur oberhalb seines Schmelzpunktes in den Schmelzekristallisator ein und kühlt den Inhalt des Kristallisators innerhalb eines Zeitraums von etwa 5 h bis etwa 30 h, bevorzugt von etwa 10 bis etwa 20 h, abhängig von der Reinheit des Ausgangsmaterials, auf Temperaturen von etwa -20°C bis etwa 15°C, bevorzugt von etwa -10°C bis etwa 15°C ab. Zur Erzielung eines vorteilhaften Kristallisationsergebnisses werden dabei bevorzugt Abkühlgeschwindigkeiten von etwa 0,1 K/h bis etwa 20 K/h, besonders bevorzugt von etwa 0,5 K/h bis etwa 5 K/h gewählt.

Nach Kristallisation der gewünschten Menge Ausagangsmaterials wird die verbleibende Mutterschmelze vorteilhaft abgelassen. Durch langsames Anheben der Temperatur des Heiz-/Kühlmediums des Wärmetauschers können gegebenenfalls anhaftende Mutterschmelzenreste bzw. gegebenenfalls eingeschlossene Verunreinigungen aufgeschmolzen bzw. durch Drainage entfernt werden. Vorteilhafte Aufheizgeschwindigkeiten liegen im Bereich zwischen etwa 0,1 und etwa 20 K/h, bevorzugt im Bereich von etwa 0,5 bis etwa 5 K/h. Bei diesem als "Schwitzen" zu bezeichnenden Prozess können je nach Reinheitsanforderungen etwa 3 bis etwa 60 Gew.-%, oft etwa 10 bis etwa 30 Gew.-% des kristallisierten Isopulegols wieder aufgeschmolzen werden. Abschlieβend kann die verbleibende enantiomerenangereicherte Kristallschicht vorteilhaft abgeschmolzen und entweder ihrer weiteren Verwendung zugeführt oder nochmals zur weiteren Aufreinigung bzw. Steigerung des Enatiomerenüberschusses kristallisiert werden. Die wie beschrieben abgetrennte Mutterschmelze und die durch Schwitzen freigesetzte Fraktion können zur Erhöhung der Ausbeute in das erfindungsgemäß bevorzugte Schmelzekristallisationsverfahren zurückgeführt werden.

Die erfindungsgemäß im Rahmen von Schritt c) bevorzugt durchzuführende Schmelzekristallisation kann alternativ dazu auch in Form einer Suspensionskristallisation durchgeführt werden. Dabei werden die Kristalle üblicherweise in suspendierter Form in ihrer Mutterschmelze erzeugt, ohne dass sich eine Kristallschicht bilden muss. Hierbei sind eine kontinuierliche Fahrweise bei konstanter Temperatur und eine diskontinuierliche Fahrweise mit sukzessive abgesenkter Temperatur möglich. Als Kühlfläche kommen hier beispielsweise mit einem wandgängigen Rührer ausgestattete Wände eines Rührbehälters in Frage, sogenannte Kratzkühler oder die gewischten Flächen in einem Kühlscheibenkristallisator. Alternativ kann auch durch Anlegen eines Vakuums und adiabatische Verdampfung des Wertstoffs (bzw. weniger bevorzugt, eines hilfsweise zugegebenen Lösemittels) die Schmelze abgekühlt werden. Die Abtrennung der suspendierten Kristalle kann dann auf dem Fachmann an sich bekannte Weise, z.B. mit einem beliebigen Filterorgan erfolgen, z.B. einer Nutsche, einer Zentrifuge oder einem Bandfilter. Wegen der prinzipiell erreichbaren, extrem hohen Reinigungswirkung kann die Abtrennung auch mittels einer Waschkolonne erfolgen, wobei der von oben an ein Filter herangeführten Suspension von unten aufgeschmolzenes Reinprodukt als Waschmittel entgegengeführt wird.

Auch das durch wie vorstehend beschriebene Kristallisation gemäß Schritt c) des erfindungsgemäßen Verfahrens erhaltene, insbesondere das wie vorstehend beschrieben enantiomerenabgereicherte oder racemische Isopulegol kann durch weitere Trennverfahren, bevorzugt durch Destillation weiter aufgereinigt werden. Dabei hat sich der Einsatz von Trennwandkolonnen bzw. Zusammenschaltungen von Kolonnen in Form einer thermischen Kopplung als vorteilhaft in verfahrenstechnischer und wirtschaftlicher Hinsicht erwiesen.

### Verfahrenschritt d): katalytische Hydrierung von Isopulegol zu Menthol

Gemäß Verfahrensschritt d) des erfindungsgemäßen Verfahrens führt man eine katalytische Hydrierung von gemäß Verfahrenschritt c) erhaltenem Isopulegol zu Menthol durch. Als Ausgangsstoffe zur Durchführung der katalytischen Hydrierung eigenen sich dabei sowohl das gemäß Verfahrensschritt c) erhaltenene enantio- bzw. diastereomerenangereicherte Isopulegol als auch das als Nebenprodukt im Rahmen der Kristallisation abgetrennte enantiomerenabgereicherte sowie racemisches Isopulegol.

Im Rahmen einer bevorzugten Ausführungsform führt man die katalytische Hydrierung von racemischem oder optisch aktivem Isopulegol gemäß Verfahrensschritt d) des erfindungsgemäßen Verfahrens in Gegenwart eines heterogenen Nickel-haltigen Katalysators durch. Bei Einsatz von enatiomerenangereichertem bzw. enantiomerenreinem Isopulegol, bevorzugt L-Isopulegol führt man die katalytische Hydrierung gemäß Verfahrensschritt d) bevorzugt in Gegenwart eines heterogenen Nickel- und Kupfer-haltigen Katalysators durch.

Die DE 577 036 offenbart ein Verfahren zur Herstellung von synthetischem Menthol durch Hydrierung von Thymol. Als geeignete Katalysatoren werden Nickel-, Nickel/Kupfer- und Kobaltkatalysatoren beschrieben.

Spezielle Nickel-Katalysatoren wurden auch zur katalytischen Hydrierung von Piperitol zu Menthol eingesetzt, wie in der GB 1,503,723 beschrieben.

Die EP 1 532 091 offenbart ein Verfahren zur Herstellung von racemischem Menthol durch katalytische Hydrierung von Isopulegol, das in Form eines Diastereomerengemisches von 70,1% Isopulegol, 18,1% Neo-Isopulegol, 6,8% Iso-Isopulegol und 2,6% Neoiso-Isopulegol eingesetzt wurde. Als Katalysator wurde mit Eisen und Chrom dotiertes Raney-Nickel eingesetzt. Man erhielt Menthol in Form eines Gemisches der möglichen Diastereomeren, das zu 61,4% aus Menthol und zu 35,6% aus den weiteren Diastereomeren des Menthols bestand.

Einen weiteren Zugang zum Menthol stellen Verfahren zur diastereoselektiven Cyclisierung von Citronellal zum Isopulegol dar, wie sie beispielsweise in den vorstehend genannten EP 1 225 163 oder WO 2006/092433 beschrieben ist. Das so erhaltene Isopulegol kann dann in einem weiteren Schritt zum Menthol hydriert werden.

R.H. Pickard et al. beschreiben in J. Chem. Soc. 1920, 1248-1263 die Herstellung von L-Menthol durch katalytische Hydrierung von L-Isopulegol in Gegenwart von kolloidalem Palladium.

B. Dudley Sully et al. beschreiben in P.& E.O.R. 1068, 235-366 die Herstellung von L-Menthol durch Hydrierung von L-Isopulegol in Gegenwart von Raney-Nickel bei einer Temperatur von 120°C.

Die EP 1 053 974 offenbart ein Verfahren zur katalytischen Hydrierung von Isopulegol zu Menthol in Gegenwart eines Katalysators von 5% Palladium auf Kohle bei einem Wasserstoffdruck von 5 bar.

Die EP 0 394 842 betrifft Katalysatoren für die Hydrierung aliphatischer ungesättigter Verbindungen, der Nickel und Kupfer enthält und gekennzeichnet ist durch einen Gehalt von 20 bis 75 Gew.% Nickeloxid, 10 bis 75 Gew.% Zirkoniumdioxis und 5 bis 50 Gew.% Kupferoxid, jeweils bezogen auf den oxidischen, nicht reduzierten Katalysators. Als Substrate werden beispielhaft angegeben: Butin-2-diol-1,4, Buten-2-diol-1,4 und 2-Ethylhexen-2-al.

Gemäß einer im Rahmen von Verfahrensschritt d) des erfindungsgemäßen Verfahrens insbesondere bevorzugten Ausführungsform führt man ein Verfahren zur Herstellung von racemischem oder optisch aktivem Menthol der Formel (XXIV) 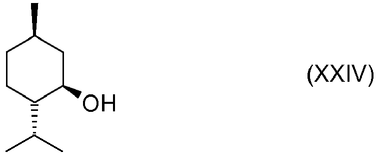 durch katalytische Hydrierung von racemischem oder optisch aktivem Isopulegol der Formel (XX) 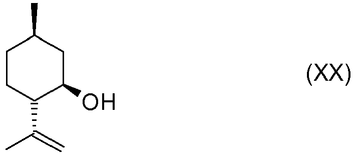 in Gegenwart von Wasserstoff und einem Katalysator, umfassend
- 30 bis 70 Gew.-% sauerstoffhaltige Verbindungen des Nickels, berechnet als NiO,
- 15 bis 45 Gew.-% sauerstoffhaltige Verbindungen des Zirkons, berechnet als ZrO₂,
- 5 bis 30 Gew.-% sauerstoffhaltige Verbindungen des Kupfers, berechnet als CuO und
- 0,1 bis 10 Gew.-% sauerstoffhaltige Verbindungen des Molybdäns, berechnet als MoO₃,
wobei sich die Angaben in Gew.-% auf den trockenen, nicht reduzierten Katalysator beziehen, durch.

Als Ausgangsstoff zur Durchführung des erfindungsgemäß im Rahmen von Verfahrensschritt d) bevorzugten Hydrierfahrens in Gegenwart der genannten Nickel- und Kupfer-haltigen Katalysatoren eignet sich racemisches oder optisch aktives Isopulegol der Formel (XX), wobei im Prinzip Isopulegol jeden Reinheitsgrades eingesetzt werden kann. Das erfindungsgemäße Verfahren eignet sich jedoch bevorzugt zur Umsetzung von Isopulegol hoher Reinheit, d.h. von Isopulegol mit einer Reinheit von 80 Gew.-% oder darüber, bevorzugt von 90 Gew.-% oder darüber. Insbesondere als Ausgangsstoff zur Durchführung des erfindungsgemäßen Verfahrens geeignet ist solches Isopulegol mit einer chemischen Reinheit von 97 Gew.-% oder darüber, bevorzugt von 98 bis 100 Gew.-%, besonders bevorzugt von 98,5 bis 99,9 Gew.-%, ganz besonders bevorzugt von mindestens 99 bis 99,9 Gew.-%. Dabei umfasst der Begriff chemische Reinheit auch die Diastereomerenreinheit des eingesetzten Isopulegols bezüglich der Diastereomere Iso-Isopulegol der Formel (XXI), Neo-Isopulegol der Formel (XXII) und Neoiso-Isopulegol der Formel (XXIII).

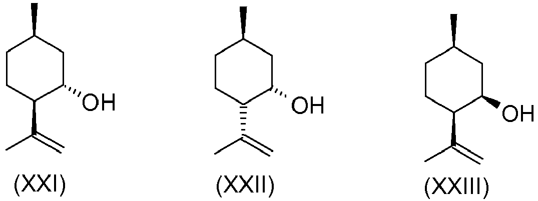

Dementsprechend weist ein als Ausgangsstoff zur Durchführung der katalytischen Hydrierung gemäß Schritt d) des erfindungsgemäßen Verfahrens besonders bevorzugtes Isopulegol eine wie vorstehend beschriebene Diastereomerenreinheit von 97 Gew.-% oder darüber, bevorzugt von 98 bis 100 Gew.-%, besonders bevorzugt von 98,5 bis 99,9 Gew.-% und ganz besonders bevorzugt von mindestens 99 bis 99,9 Gew.-% auf. Dabei können die genannten Formeln wie alle im Rahmen der vorliegenden Erfindung abgebildeten Formeln jeweils für beide Enantiomere (bzw. Gemische derselben) stehen und dienen zur Verdeutlichung der relativen Konfiguration der stereogenen Zentren.

Isopulegol kann im Rahmen von Verfahrensschritt d) erfindungsgemäß in racemischer oder nicht-racemischer, d.h. optisch aktiver Form eingesetzt werden. Bei Einsatz von racemischem Isopulegol der Formel (XX) erhält man erfindungsgemäß racemisches Menthol der Formel (XXIV). Bei Einsatz von optisch aktivem, bevorzugt von enantiomerenangereichertem und besonders bevorzugt von enantiomerenreinem Isopulegol der Formel (XX) erhält man entsprechend optisch aktives Menthol der Formel (XXIV). Setzt man Isopulegol in optisch aktiver Form ein, sind erfindungsgemäß bevorzugt solche Gemische, die zum überwiegenden Teil das Enantiomer L-Isopulegol, wie es in seiner absoluten Konfiguration beispielhaft in Formel (XX) wiedergegeben ist, enthalten.

Im Rahmen von Verfahrensschritt d) des erfindungsgemäßen Verfahrens setzt man im Falle der Umsetzung von enantiomerenangereichertem Isopulegol d.h. D- oder bevorzugt L-Isopulegol mit einem Enatiomerenüberschuss (ee) von 80% oder darüber bevorzugt von 85 oder besser 90% ee oder darüber, besonders bevorzugt 95 bis 100% ee, ganz besonders bevorzugt 96 bis 99,9% ee, weiterhin bevorzugt 97 bis 99,8% ee, noch mehr bevorzugt 98 bis 99,7% ee und insbesondere bevorzugt 98,5 bis 99,6% ee ein. Ausgehend von L-Isopulegol in optisch aktiver Form erhält man in erfindungsgemäßer Weise L-Menthol, wie es in seiner absoluten Konfiguration in Formel (XXIV) wiedergegeben ist, in optisch aktiver Form.

Das erfindungsgemäß bevorzugte katalytische Hydrierverfahren wird in Gegenwart von Wasserstoff und in Gegenwart eines heterogenen Katalysators durchgeführt, wobei der einzusetzende heterogene Katalysator 30 bis 70 Gew.-%, bevorzugt 40 bis 60 Gew.-% sauerstoffhaltige Verbindungen des Nickels, berechnet als NiO, 15 bis 45 Gew.-%, bevorzugt 20 bis 40 Gew.-% sauerstoffhaltige Verbindungen des Zirkons, berechnet als ZrO₂, 5 bis 30 Gew.-%, bevorzugt 10 bis 25 Gew.-% sauerstoffhaltige Verbindungen des Kupfers, berechnet als CuO und 0,1 bis 10 Gew.-%, bevorzugt 0,5 bis 5 Gew.-% sauerstoffhaltige Verbindungen des Molybdäns, berechnet als MoO₃ gegebenenfalls neben weiteren Komponenten in einer Menge von 0 bis 10 Gew.-%, bevorzugt 0 bis 5 Gew.-% wie beispielsweise Graphit enthält. Dabei beziehen sich die Angaben in Gew.-% auf den trockenen, nicht reduzierten Katalysator.

Da sich die Konzentrationsangaben jeweils - falls nicht anders angegeben - auf die katalytisch aktive Masse des Katalysators beziehen, wird die katalytisch aktive Masse des Katalysators im Folgenden als die Summe der Massen der katalytisch aktiven Bestandteile Zirkonium, Nickel, Kupfer und Molybdän im Katalysator, jeweils berechnet als ZrO₂, NiO, CuO bzw. MoO₃, nach dessen letzter Wärmebehandlung und vor dessen Reduktion mit Wasserstoff, definiert.

Im Rahmen einer bevorzugten Ausführungsform setzt man zur Durchführung des erfindungsgemäß bevorzugten Hydrierverfahrens gemäß Verfahrensschritt d) solche Katalysatoren ein, umfassend
- 45 bis 55 Gew.-% sauerstoffhaltige Verbindungen des Nickels, berechnet als NiO,
- 25 bis 35 Gew.-% sauerstoffhaltige Verbindungen des Zirkons, berechnet als ZrO₂,
- 5 bis 20 Gew.-% sauerstoffhaltige Verbindungen des Kupfers, berechnet als CuO,
- 1 bis 3 Gew.-% sauerstoffhaltige Verbindungen des Molybdäns, berechnet als MoO₃ und
- 0 bis 5 Gew.-% weitere Komponenten,
wobei sich die Angaben in Gew.-% zu 100 Gew.-% ergänzen und sich auf den trockenen, nicht reduzierten Katalysator beziehen. Erfindungsgemäß insbesondere bevorzugt sind solche Katalysatoren, die aus den vorstehend genannten Komponenten in den ebenfalls vorstehend genannten Gewichtsanteilen bestehen.

Ein zum Einsatz im Rahmen von Verfahrensschritt d) des erfindungsgemäßen Verfahrens insbesondere bevorzugter Katalysator besteht zu 49 bis 53 Gew.-% aus NiO, zu 15 bis 19 Gew.-% aus CuO, zu 28 bis 32 Gew.-% aus ZrO₂ und zu 1 bis 2 Gew.-% aus MoO₃ sowie gegebenenfalls zu 0 bis 3 Gew.-% aus weiteren Komponenten wie beispielsweise Graphit, wobei sich die jeweils gewählten Gewichtsanteile der einzelnen Komponenten zu 100 Gew.-% ergänzen. Derartige Katalysatoren sind bekannt und können beispielsweise wie in der EP 0 696 572, auf die diesbezüglich umfassend Bezug genommen wird, beschrieben, hergestellt werden.

Die erfindungsgemäß im Rahmen von Verfahrensschritt d) bevorzugt einzusetzenden Katalysatoren können z.B. durch Einsatz von Fällungsmethoden hergestellt werden. So können sie beispielsweise durch eine gemeinsame Fällung der Nickel- und Kupferkomponenten aus einer diese Elemente enthaltenden, wässrigen Salzlösung mittels Mineralbasen in Gegenwart einer Aufschlämmung einer schwerlöslichen, sauerstoffhaltigen Zirkoniumverbindung und anschließendes Waschen, Trocknen und Calcinieren des erhaltenen Präzipitats erhalten werden. Als schwerlösliche, sauerstoffhaltige Zirkoniumverbindungen können beispielsweise Zirkoniumdioxid, Zirkoniumoxidhydrat, Zirkoniumphosphate, -borate und -silikate Verwendung finden. Die Aufschlämmungen der schwerlöslichen Zirkoniumverbindungen können durch Suspendieren feinkörniger Pulver dieser Verbindungen in Wasser unter kräftigem Rühren hergestellt werden. Vorteilhaft werden diese Aufschlämmungen durch Ausfällen der schwerlöslichen Zirkoniumverbindungen aus wässrigen Zirkoniumsalzlösungen mittels Mineralbasen erhalten.

Bevorzugt werden die im Rahmen von Verfahrensschritt d) des erfindungsgemäßen Verfahrens einsetzbaren Katalysatoren über eine gemeinsame Fällung (Mischfällung) aller ihrer Komponenten hergestellt. Dazu wird zweckmäßigerweise eine die Katalysatorkomponenten enthaltende, wässrige Salzlösung in der Wärme und unter Rühren so lange mit einer wässrigen Mineralbase, insbesondere einer Alkalimetallbase - beispielsweise Natriumcarbonat, Natriumhydroxid, Kaliumcarbonat oder Kaliumhydroxid - versetzt, bis die Fällung vollständig ist. Die Art der verwendeten Salze ist im allgemeinen nicht kritisch - da es bei dieser Vorgehensweise vornehmlich auf die Wasserlöslichkeit der Salze ankommt, ist ein Kriterium ihre zur Herstellung dieser verhältnismäßig stark konzentrierten Salzlösungen erforderliche, gute Wasserlöslichkeit. Es wird als selbstverständlich erachtet, dass bei der Auswahl der Salze der einzelnen Komponenten natürlich nur Salze mit solchen Anionen gewählt werden, die nicht zu Störungen führen, sei es, indem sie unerwünschte Fällungen verursachen oder indem sie durch Komplexbildung die Fällung erschweren oder verhindern.

Erfindungsgemäß im Rahmen des Verfahrensschritts d) einsetzbare Katalysatoren mit besonders vorteilhaften Eigenschaften sind dadurch erhältlich, dass man einen Teil der Zirkoniumkomponente des Katalysators, zweckmäßigerweise aus einer wässrigen Zirkoniumsalzlösung, separat in einer Fällungsapparatur durch Zugabe wässriger Mineralbasen fällt. Auf das so erhaltene, vorzugsweise frisch gefällte Zirkoniumoxid-Hydrat, kann dann der restliche Teil der Zirkoniumkomponente des Katalysators zusammen mit den anderen katalytisch aktiven Komponenten in einer Mischfällung gefällt werden, wie oben beschrieben wurde. Dabei erweist es sich in der Regel als besonders zweckmäβig, 10 bis 80 Gew.-%, vorzugsweise 30 bis 70 Gew.-% und insbesondere 40 bis 60 Gew.-% der Gesamtzirkoniummenge der katalytisch aktiven Masse vorzufällen.

Die bei diesen Fällungsreaktionen erhaltenen Niederschläge sind im Allgemeinen chemisch uneinheitlich und bestehen u.a. aus Mischungen der Oxide, Oxidhydrate, Hydroxide, Carbonate und unlöslichen und basischen Salze der genannten Metalle. Es kann sich für die Filtrierbarkeit der Niederschläge als günstig erweisen, wenn sie gealtert werden, d.h. wenn man sie noch einige Zeit nach der Fällung, gegebenenfalls in Wärme oder unter Durchleiten von Luft, sich selbst überlässt.

Die nach diesen Fällungsverfahren erhaltenen Niederschläge können wie üblich zu den fertigen, erfindungsgemäß im Rahmen des Verfahrensschritts d) bevorzgt einsetzbaren Katalysatoren weiterverarbeitet werden. Nach dem Waschen werden sie im Allgemeinen bei 80 bis 200°C, vorzugsweise bei 100 bis 150°C, getrocknet und danach calciniert. Die Calcinierung wird im Allgemeinen bei Temperaturen zwischen 300 und 800°C, vorzugsweise bei 400 bis 600°C, insbesondere bei 450 bis 550°C ausgeführt.

Nach der Calcinierung wird der Katalysator zweckmäßigerweise konditioniert, sei es, dass man ihn durch Vermahlen auf eine bestimmte Korngröße einstellt oder dass man ihn nach seiner Vermahlung mit Formhilfsmitteln wie Graphit oder Stearinsäure vermischt, mittels einer Tablettenpresse zu Formlingen verpresst und tempert. Die Temperaturen entsprechen dabei im Allgemeinen den Temperaturen bei der Calcinierung.

Die auf diese Weise hergestellten Katalysatoren enthalten die katalytisch aktiven Metalle in Form eines Gemisches ihrer sauerstoffhaltigen Verbindungen, d.h. insbesondere als Oxide und Mischoxide.

Die auf diese Weise hergestellten Katalysatoren können als solche gelagert und eingesetzt werden. Vor ihrem Einsatz als Katalysatoren im Rahmen von Verfahrensschritt d) des erfindungsgemäßen Verfahrens werden sie üblicherweise vorreduziert. Sie können jedoch auch ohne Vorreduktion eingesetzt werden, wobei sie dann unter den Bedingungen der erfindungsgemäß durchzuführenden Hydrierung durch den im Reaktor vorhandenen Wasserstoff reduziert werden. Zur Vorreduktion werden die Katalysatoren im allgemeinen zunächst bei 150 bis 200°C über einen Zeitraum von 12 bis 20 Stunden einer Stickstoff-Wasserstoff-Atmosphäre ausgesetzt und anschließend noch bis zu ca. 24 Stunden bei 200 bis 300°C in einer Wasserstoffatmosphäre behandelt. Bei dieser Vorreduktion wird ein Teil der in den Katalysatoren vorliegenden sauerstoffhaltigen Metallverbindungen üblicherweise zu den entsprechenden Metallen reduziert, so dass diese gemeinsam mit den verschiedenartigen Sauerstoffverbindungen in der aktiven Form des Katalysators vorliegen.

Im Allgemeinen werden die im Rahmen von Verfahrensschritt d) des erfindungsgemäβen Hydrierverfahrens einzusetzenden Katalysatoren bevorzugt in Form von Vollkatalysatoren eingesetzt. Mit dem Begriff "Vollkatalysator" wird ein Katalysator bezeichnet, der im Gegensatz zu einem Trägerkatalysator nur aus katalytisch aktiver Masse besteht. Vollkatalysatoren können dergestalt eingesetzt werden, dass man die katalytisch aktive, zu Pulver vermahlene Masse in das Reaktionsgefäß einbringt oder, dass man die katalytisch aktive Masse nach Mahlung, Vermischung mit Formhilfsmitteln, Formung und Temperung als Katalysatorformkörper- beispielsweise als Kugeln, Zylinder, Tabletten, Ringe, Spiralen, Stränge und dergleichen mehr - im Reaktor anordnet.

Im Rahmen einer bevorzugten Ausführungsform des gemäß Schritt d) durchzuführenden katalytischen Hydrierverfahrens setzt man den gewählten heterogenen Katalysator in Form eines Festbettkatalysators ein.

Zur Durchführung der katalytischen Hydrierung gemäß Schritt d) des erfindungsgemäβen Verfahrens bringt man den wie vorstehend beschriebenen Ausgangsstoff Isopulegol mit Wasserstoff und dem gewählten Katalysator in Kontakt. Der Wasserstoff kann dabei in unverdünnter Form, üblicherweise in einer Reinheit von etwa 99,9 Vol.-% oder in verdünnter Form, d.h. in Form von Gemischen mit inerten Gasen wie beispielsweise Stickstoff oder Argon, eingesetzt werden. Bevorzugt setzt man Wasserstoff in unverdünnter Form ein.

Die Hydrierung von Isopulegol kann mit gutem Erfolg ohne Zusatz von Lösungsmittel oder in Gegenwart von unter den Reaktionsbedingungen inerten organischen Lösungsmitteln wie beispielsweise Methanol, Ethanol, Isopropanol, Hexan, Heptan, Cyclohexan und dergleichen mehr durchgeführt werden. Bevorzugt führt man die Hydrierung gemäß Schritt d) des erfindungsgemäßen Verfahrens ohne Zusatz von Lösungsmittel durch.

Die katalytische Hydrierung von Isopulegol gemäß Verfahrensschritt d) kann bei einem Wasserstoffdruck (absolut) im Bereich von 1 bis 200 bar, bevorzugt von 2 oder besser 3 bis 200 bar, besonders bevorzugt von 4 oder 5 bis 150 bar, besonders bevorzugt 5 bis 100 bar und ganz besonders bevorzugt im Bereich von 5 bis 50 bar durchgeführt werden. Als Reaktionstemperatur zur Durchführung der erfindungsgemäßen Hydrierung wählt man vorteilhaft eine Temperatur im Bereich von 20 bis 150°C, bevorzugt 40 bis 130°C, besonders bevorzugt 60 bis 110°C und ganz besonders bevorzugt von 70 bis 100°C.

Praktisch geht man bei der Durchführung der Hydrierung von Isopulegol gemäß Verfahrensschritt d) im Allgemeinen so vor, dass man dem Katalysator, der sich üblicherweise in einem bevorzugt von außen beheizten Festbettreaktor wie beispielsweise einem Rohrreaktor, Autoklaven oder Rohrbündelreaktor befindet, bei der gewünschten Reaktionstemperatur und dem gewünschten Druck das umzusetzende Isopulegol zuführt. Dabei belastet man den Katalysator im Allgemeinen mit 0,1 bis 1,0, bevorzugt 0,1 bis 0,6 und besonders bevorzugt mit 0,2 bis 0,4 kg Isopulegol pro kg Katalysator und Stunde. Hierbei kann es zweckmäßig sein, das einzusetzende Isopulegol bereits vor der Zuführung in das Reaktionsgefäß bzw. den Reaktor zu erwärmen und zwar bevorzugt auf die Reaktionstemperatur.

Der Reaktor kann sowohl in der Sumpf- als auch in der Rieselfahrweise betrieben werden, d.h. man kann die Ausgangsstoffe sowohl von unten nach oben als auch von oben nach unten durch den Reaktor leiten. Das erfindungsgemäße Hydrierverfahren kann sowohl diskontinuierlich als auch kontinuierlich durchgeführt werden. In beiden Fällen kann nicht umgesetztes Edukt zusammen mit dem Wasserstoff im Kreis geführt werden.

Die erfindungsgemäß im Rahmen von Verfahrensschritt d) bevorzugt durchzuführende Hydrierung kann auch stufenweise in einer Kaskade von mehreren, d.h. 2 bis in der Regel 4, bevorzugt 2 oder 3 und insbesondere bevorzugt in zwei hintereinander geschalteten Reaktoren, bevorzugt Festbettreaktoren durchgeführt werden. Dabei wird in dem ersten, üblicherweise als Hauptreaktor bezeichneten Reaktor unter den vorstehend beschriebenen Reaktionsbedingungen der Hauptumsatz der Reaktion erzielt und das erhaltene Rohprodukt einem zweiten, üblicherweise als Nachreaktor bezeichneten Reaktor zugeführt, in dem das noch nicht umgesetzte Ausgangsmaterial in erfindungsgemäßer Weise zumindest weitgehend in Menthol, bzw. bei erfindungsgemäß bevorzugtem Einsatz von enantiomerenangereichertem bzw. enantiomerenreinem L-Isopulegol in L-Menthol überführt wird. Dabei können die Reaktionsbedingungen unabhängig voneinander vorzugsweise in den vorstehend genannten Bereichen gewählt werden.

Das vorstehend beschriebene Hydrierverfahren kann diskontinuierlich, halb- oder vollkontinuierlich durchgeführt werden. Bevorzugt führt man das Verfahren kontinuierlich, insbesondere vollkontinuierlich durch, wobei die Ausgangsstoffe kontinuierlich in den Reaktor eingetragen werden und das erhaltene Reaktionsgemisch bzw. Reaktionsprodukt kontinuierlich aus dem Reaktor ausgetragen wird. Es hat sich weiterhin als vorteilhaft erwiesen, aufgrund der Lage des Schmelzpunktes des erfindungsgemäßen Reaktionsproduktes Menthol, speziell L-Menthol für eine Beheizung der eingesetzten Transportleitungen zu sorgen.

Das dargestellte im Rahmen von Verfahrensschritt d) bevorzugt durchzuführende-Hydrierverfahren erlaubt die Herstellung von wahlweise racemischem oder optisch aktivem Menthol durch katalytische Hydrierung von entsprechendem racemischem oder optisch aktivem Isopulegol, wobei es üblicherweise nur in geringem Ausmaß zur Bildung von unerwünschten Diastereomeren des Menthols kommt. Das erfindungsgemäße Verfahren liefert dementsprechend, bei Einsatz von Isopulegol mit einer entsprechenden Reinheit, Menthol der Formel (XXIV) in einer chemischen Reinheit von 97 Gew.-% oder darüber, bevorzugt von 98 bis 100 Gew.-%, besonders bevorzugt von 98,5 bis 99,9 Gew.-%, ganz besonders bevorzugt von mindestens 99 bis 99,9 Gew.-%. Dabei umfasst der Begriff "chemische Reinheit" auch die Diastereomerenreinheit des erhaltenen Menthols bezüglich der Diastereomere Neoiso-Menthol der Formel (XXV), Neo-Menthol der Formel (XXVI) und Iso-Menthol der Formel (XXVII). Dementsprechend liefert das erfindungsgemäße Verfahren im Rahmen bevorzugt Menthol der Formel (XXIV) mit einer Diastereomerenreinheit von 97 Gew.-% oder darüber, bevorzugt von 98 bis 100 Gew.-%, besonders bevorzugt von 98,5 bis 99,9 Gew.-% und ganz besonders bevorzugt von mindestens 99 bis 99,9 Gew.-%.

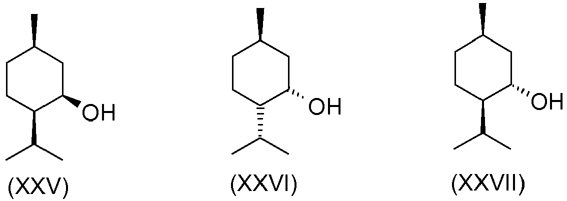

Setzt man Isopulegol in optisch aktiver Form, erfindungsgemäß bevorzugt solche Gemische, die zum überwiegenden Teil das Enantiomer L-Isopulegol enthalten, ein, erhält man als erfindungsgemäßes Verfahrensprodukt im Rahmen von Verfahrensschritt d) in der Regel Menthol in optisch aktiver Form, bevorzugt in Form des (-)- bzw-L-Menthols.

Die dargestellte, erfindungsgemäß bevorzugte katalytische Hydrierung in Gegenwart der vorstehend beschriebenen Nickel- und Kupfer enthaltenden Katalysatoren verläuft im Regelfall weitgehend ohne nennenswerte Racemisierung des eingesetzten Materials. Demnach erhält man, in Abhängigkeit vom Enantiomerenüberschuss des eingesetzten optisch aktiven Isopulegols optisch aktives, bevorzugt bei Einsatz von L-Ispulegol, L-Menthol mit einem Enatiomerenüberschuss (ee) von 80% oder darüber, bevorzugt von 85 oder 90% ee oder darüber, besonders bevorzugt 95 bis 100% ee, besonders bevorzugt 96 bis 99,9% ee, ganz besonders bevorzugt 97 bis 99,8% ee, noch mehr bevorzugt 98 bis 99,7% ee und insbesondere bevorzugt 98,5 bis 99,6% ee ein.

Das erfindungsgemäß erhaltene Menthol, insbesondere das erhaltene optisch aktive Menthol, zeichnet sich darüber hinaus durch einen besonders geringen Gehalt der unerwünschten Nebenprodukte Menthon der Formel (XXVIII) und Isomenthon der Formel (XXIX) und Neoiso-Menthol der Formel (XXV) aus.

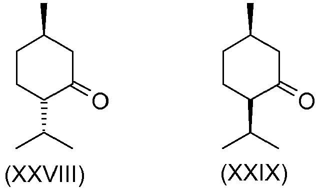

Diese Nebenprodukte werden im Regelfall im Rahmen des erfindungsgemäß bevorzugten Hydrierverfahrens nur in einem auf die Menge an erhaltenem Menthol bezogenen Anteil von bis zu 0,5 Gew.-%, bevorzugt 0,4 Gew.-%, besonders bevorzugt 0,3 Gew.-%, insbesondere 0,2 Gew.-% und ganz besonders bevorzugt 0,1 bis 0 Gew.-% erhalten.

Wie vorstehend beschrieben fällt bei der gemäß Verfahrensschritt c) erfindungsgemäß durchzuführenden Kristallisation, bevorzugt der beschriebenen Schmelzekristallisation bei Einsatz von optisch aktivem Isopulegol enthaltenden Stoffgemischen neben enantiomerenangereichertem bzw. enantiomerenreinem Isopulegol auch, wie unter Verfahrensschritt c) beschrieben racemisches oder enantiomerenabgereichertes Isopulegol an. Dieses racemische oder enantiomerenabgereicherte Isopulegol kann im Rahmen der katalytischen Hydrierung gemäß Verfahrensschritt d) in vorteilhafter Weise mittels der bevorzugten Katalysatoren zum racemischen oder enantiomerenabgereicherten Menthol hydriert werden. Daneben ist es auch möglich, derartiges racemisches oder enantiomerenabgereichertes Isopulegol nach Verfahren des Standes der Technik, beispielsweise durch katalytische Hydrierung in Gegenwart von Wasserstoff und Raney-Nickel als Katalysator zu racemischem oder enantiomerenabgereichertem Menthol zu hydrieren.

### e) Feindestillation von Menthol

Das vorstehend beschriebene Verfahren zur katalytischen Hydrierung von zuvor durch Kristallisation, vorzugsweise durch Schmelzekristallsation aufgereinigtem Isopulegol liefert in der Regel racemisches und optisch aktives Menthol, bevorzugt racemisches oder enantiomerenabgereichertes Menthol und L-Menthol von hoher chemischer Reinheit, auch bezüglich unerwünschter Diastereomere des Menthols und, im Fall des enantiomerenangereicherten optisch aktiven Menthols, von hoher Enantiomerenreinheit. Zur Sicherstellung höchster Qualitätsanforderungen, insbesondere im Hinblick auf die sensorischen, speziell olfaktorischen Eigenschaften des erhaltenen Menthols sowie im Hinblick der Reinheitsanforderungen der Arzneibücher hat es sich als vorteilhaft erwiesen, das gemäß Verfahrensschritt d) erhaltenene enantiomerenangereicherte optisch aktive Menthol bzw. racemische oder enantiomerenabgereicherte Menthol einer abschließenden Destillation zu unterwerfen. Das erfindungsgemäße Verfahren umfasst daher im Rahmen einer bevorzugten Ausführungsform als weiteren optionalen Verfahrensschritt e) die destillative Aufreinigung von gemäß den Verfahrensschritten a) bis d) bzw. von gemäß den Verfahrensschritten 0) bis d) erhaltenem racemischem und/oder optisch aktivem Menthol, bevorzugt mittels einer Trennwandkolonne.

In der Literatur sind verschiedene Reinigungsverfahren für Menthol beschrieben. So sind dem Fachmann neben der fraktionierten Destillation mit und ohne Wasserdampf wie beispielsweise in der DE 568 085 oder DE 1 189 073 sowie der US 1,930,411, JP 27003884 oder JP 32009869 beschrieben, Extraktionsverfahren und Kristallisationsverfahren bekannt.

Diese Verfahren werden zum Teil auch in Kombination, z.B. als Kombinationen aus Kristallisation und fraktionierter Destillation oder auch in Kombination mit chemischen Umsetzungen bzw. Derivatisierungen eingesetzt.

Die GB 285,394 betrifft ein Verfahren zur Herstellung von racemischem Menthol durch Hydrierung von Thymol, fraktionierte Destillation der daraus erhaltenen Gemische und anschließendes Ausfrieren von Neomenthol aus den Mentholfraktionen.

Die GB 285,833 beschreibt ein Verfahren zur Herstellung von Thymol durch fraktionierte Destillation von Gemischen, die aus der Kondensation von Kresol mit Aceton erhalten wurden und neben Thymol isomere Methyl-isopropyl-phenole enthalten.

Die US 2,827,497 offenbart ein Verfahren, bei dem durch fraktionierte Destillation und fraktionierte Kristallisation gewonnene Diastereomerngemische des Menthols einer Oxidation unterzogen werden und anschließend durch abermalige fraktionierte Destillation weiter aufgereinigt werden.

Die EP 0 242 778 beschreibt ein Verfahren zur Trennung von Diastereomerengemischen, wie u.a. Gemischen von Menthol, Isomenthol, Neomenthol und Neoisomenthol durch Extraktivdestillation, d.h. durch Destillation unter Zusatz spezieller Hilfsstoffe, wie beispielsweise Bernsteinsäurediamid.

Die beschriebenen Verfahren weisen zumeist den Nachteil auf, dass Hilfsstoffe verwendet werden (Wasserdampf oder Extraktivdestillation) oder Feststoffe anfallen. Die fraktionierenden Batch-Destillationen sind zumeist nachteilig hinsichtlich ihrer Ausbeute an Wertprodukt, da das Produkt längere Zeit thermisch belastet wird.

Die EP 1 514 955 betrifft ein Verfahren zur destillativen Aufarbeitung des Elektrolyseaustrages der elektrochemischen Oxidation von 1,1,2,2-Tetramethoxyethan mit Methanol zu Trimethylorthoformiat in einem flüssigen Elektrolyten, wobei eine Trennwandkolonne mit 30 bis 150 theoretischen Trennstufen eingesetzt wird.

Die DE 103 30 934 offenbart ein Verfahren zur kontinuierlichen Isolierung von Citronellal oder Citronellol aus einem mindestens eine dieser Verbindungen enthaltenden Rohgemisch durch Rektifikation. Dabei werden vorzugsweise solche Ausgangsgemische eingesetzt, die durch partielle Hydrierung von Citral bzw. Citronellal erhalten werden.

Die DE 102 23974 betrifft ein Verfahren zur kontinuierlichen Isolierung zweier stereoisomerer Isoprenoidalkohole, speziell Nerol und Geraniol, aus einem Rohgemisch durch Rektifikation, wobei das Rohgemisch seitlich in eine Zulaufkolonne eingeführt wird, man wenigstens eine mit der Zulaufkolonne gekoppelte Abzugskolonne vorsieht und aus der Abzugskolonne einen ersten und einen zweiten Isoprenoidalkohol abzieht. Dabei werden die Zulauf- und die Abzugskolonne so gekoppelt, dass zumindest im Bereich des Abzugs der Isoprenoidalkohole keine Quervermischung von Brüden und Kondensat erfolgt.

Die destillative Reinigung des Menthols, speziell des L-Menthols von seinen Diastereomeren Neoiso- und Isomenthol, ist insbesondere aufgrund des sehr geringen Siedepunktsunterschiedes von ca. 2°C bei Umgebungsdruck üblicherweise sehr aufwendig.

Im Rahmen einer bevorzugten Ausführungsform führt man den optionalen Verfahrensschritt e) des erfindungsgemäßen Verfahrens in Form eines kontinuierlichen Verfahrens zur Herstellung von racemischem oder optisch aktivem Menthol der Formel (XXIV) 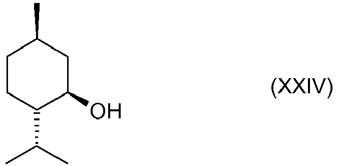 in reiner oder angereicherter Form durch destillative Abtrennung von racemischem oder optisch aktivem Menthol aus Stoffgemischen enthaltend racemisches oder optisch aktives Menthol und Diastereomere des Menthols aus, wobei man die destillative Abtrennung in einer Trennwandkolonne mit 50 bis 300 theoretischen Trennstufen und einer oder mehrerer Seitenabzugsstellen bei einem absoluten Betriebsdruck von 5 bis 500 mbar durchführt.

Als Ausgangsstoffe zur Durchführung des im Rahmen des optionalen Verfahrensschritts e) bevorzugt durchzuführenden Trennverfahrens dienen Stoffgemische, die racemisches oder optisch aktives Menthol, bevorzugt optisch aktives Menthol, besonders bevorzugt L-Menthol und Diastereomere des Menthols enthalten.

Als Diastereomere des Menthols seien die vorstehend abgebildeten Verbindungen Neoisomenthol der Formel (XXV), Neomenthol der Formel (XXVI) und Isomenthol der Formel (XXVII) genannt, die, je nach Art des als Ausgangsstoff dienenden Gemisches in racemischer oder nicht-racemischer, d.h. optisch aktiver Form vorliegen können. Die genannten Diastereomere können in den erfindungsgemäß im Rahmen von Verfahrensschritt e) einzusetzenden Stoffgemischen einzeln oder in Form von Gemischen untereinander vorliegen. Die als Ausgangsstoff im Rahmen des erfindungsgemäß bevorzugten Trennverfahrens einzusetzenden Stoffgemische enthalten neben Menthol der Formel (XXIV) in racemischer oder optisch aktiver Form zumindest eines der Diastereomere der Formeln (XXV), (XXVI) oder (XXVII), üblicherweise jedoch ein Gemisch aus zwei oder allen drei der genannten Diastereomere.

Im Rahmen des gewünschtenfalls gemäß Verfahrensschritt e) durchzuführenden Verfahrens können bevorzugt auch solche Stoffgemische eingesetzt werden, die neben den vorstehend genannten Diastereomeren des Menthols auch Isopulegol der Formel (XX) 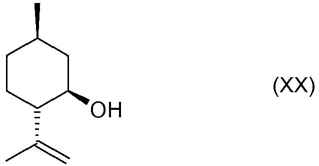 und/oder dessen Diastereomere sowie gegebenenfalls wie vorstehend abgebildetes Menthon der Formel (XXVIII) und/oder Isomenthon der Formel (XXIX) enthalten.

Die genannten Verbindungen können dabei, je nach Art, Ursprung oder Herstellverfahren des jeweils eingesetzten Stoffgemisches in racemischer oder optisch aktiver Form vorliegen.

Als Diastereomere des Isopulegols der Formel (XX), insbesondere L-Isopulegol seien die ebenfalls vorstehend abgebildeten Verbindungen iso-Isopulegol der Formel (XXI), neo-Isopulegol der Formel (XXII), neoiso-Isopulegol der Formel (XXIII) und genannt, die ebenfalls, je nach Art des als Ausgangsstoff dienenden Gemisches in racemischer oder nicht-racemischer Form vorliegen können.

Eine bevorzugte Ausführungsform des gemäß dem optionalen Verfahrensschritt e) bevorzugt durchzuführenden Verfahrens betrifft die Herstellung von L-Menthol in reiner oder angereicherter Form durch destillative Abtrennung von L-Menthol aus Stoffgemischen enthaltend L-Menthol und Diastereomere des Menthols der Formeln (XXV), (XXVI) und/oder (XXVII) und gegebenenfalls Isopulegol der Formel (XX) und/oder dessen Diastereomere der Formeln (XXI), (XXII) und/oder (XXIII) und gegebenenfalls Menthon der Formel (XXVIII) bzw. Isomenthon der Formel (XXIX).

Als Einsatzstoffe zur Durchführung des im Rahmen des optionalen Verfahrensschritts e) durchzuführenden Verfahrens eignen sich Stoffgemische die racemisches oder optisch aktives Menthol, bevorzugt L-Menthol in optisch aktiver Form enthalten, bevorzugt solche, die zum überwiegenden Teil aus racemischem oder optisch aktivem Menthol, bevorzugt L-Menthol bestehen. Bevorzugt sind darunter solche Stoffgemische, die mindestens 80 Gew.-% oder besser 85 oder noch besser 90 Gew.-% bis 99,9 Gew.-%, besonders bevorzugt 95 bis 99,8 Gew.-% und ganz besonders bevorzugt mindestens 96 Gew.-%, 97 Gew.-% oder am meisten bevorzugt mindestens 98 Gew.-% bis 99,7 Gew.-%, 99,6 Gew.-% oder am meisten bevorzugt bis 99,5 Gew.-% racemisches oder optisch aktives Menthol, bevorzugt L-Menthol enthalten und daneben in geringem Ausmaß, d.h. zu einem Anteil von bis zu 20 Gew.-%, bevorzugt von 0,1 bis zu 10 Gew.-% und besonders bevorzugt von 0,2 bis zu 5 Gew.-%, insbesondere bevorzugt von 0,3 oder besser 0,4 Gew.-% bis zu 2,5 Gew.-%, noch mehr bevorzugt bis zu 1,5 Gew.-%, besser bis zu 1 Gew.-% und am meisten bevorzugt bis zu 0,5 Gew.-% noch weitere Komponenten wie beispielsweise Diastereomere des Menthols, Nebenprodukte wie Isopulgol bzw. dessen Diastereomere oder Menthon oder Isomenthon oder sonstige Verunreinigungen wie beispielsweise Lösemittelreste oder Wasser enthalten.

Bei Einsatz von Stoffgemischen, die Menthol in optisch aktiver Form, bevorzugt L-Menthol enthalten, liegt dieses üblicherweise in einem Enantiomerenüberschuss von 90% ee oder darüber, bevorzugt 95% ee, besonders bevorzugt 97% ee oder noch mehr bevorzugt 98% ee oder darüber, d.h. bis 100% ee oder bevorzugt bis 99,9% ee vor. Aus diesen Stoffgemischen werden im Rahmen des erfindungsgemäß bevorzugt durchzuführenden Trennverfahrens entsprechend optisch aktives Menthol, bevorzugt L-Menthol in reiner oder angereicherter Form erhalten, wobei der Enantiomerenüberschuss des erhaltenen Produktes in der Regel dem Enantiomerenüberschuss des Menthols im eingesetzten Stoffgemisch zumindest weitestgehend entspricht. Bei Einsatz von racemischem Menthol enthaltenden Stoffgemischen erhält man erfindungsgemäß racemisches Menthol der Formel (I) in reiner oder angereicherter Form.

Im Rahmen einer bevorzugten Ausführungsform des gemäß dem optionalen Verfahrensschritt e) durchzuführenden Verfahrens dient als Ausgangsstoff ein Stoffgemisch, das einen Enantiomerenüberschuß von mehr als 99,4% aufweist. Im Rahmen einer weiteren bevorzugten Ausführungsform des erfindungsgemäßen Verfahrens dient als Ausgangsstoff ein Stoffgemisch, dass zu mindestens 98 Gew.-% aus Menthol (L- bzw. auch D-Menthol, bevorzugt L-Menthol) und in Summe zu bis zu 2 Gew.-% (jeweils bezogen auf das Gemisch) aus Diastereomeren des Menthols und/oder Isopulegol und dessen Diastereomere (jeweils in der D- oder L-Form) und/oder Isomenthon bzw. Menthon und/oder anderen Komponenten, wie Alkohole, Ketone, Aldehyde, Kohlenwasserstoffe oder Wasser besteht, wobei der Gehalt an Menthon und/oder Isomenthon und der Gehalt an anderen Komponenten jeweils weniger als 1 Gew.% (bezogen auf das Gemisch) beträgt.

Die gewünschtenfalls im Rahmen des Verfahrensschritts e) durchzuführende destillative Abtrennung wird üblicherweise so durchgeführt, dass man das eingesetzte Menthol, bevorzugt L-Menthol enthaltende Stoffgemisch in jeweils eine oder mehrere Leichtsieder-, Mittelsieder- und Schwersiederfraktion bzw. -fraktionen auftrennt und Menthol, bevorzugt L-Menthol in reiner oder angereicherter Form als Mittelsiederfraktion an der Seitenabzugsstelle der eingesetzten Trennwandkolonne in flüssiger oder gasförmiger Form entnimmt.

Bei dem im Rahmen von Verfahrensschritt e) gewünschtenfalls durchzuführenden Verfahren handelt es sich demnach auch um ein kontinuierliches Verfahren zur Isolierung von Menthol, bevorzugt von L-Menthol, vorzugsweise um ein kontinuierliches Verfahren zur Isolierung von Menthol in reiner oder angereicherter Form durch destillative Abtrennung von Menthol aus Stoffgemischen enthaltend Menthol und seine wie vorstehend beschriebenen Diastereomere, wobei man die destillative Abtrennung in einer Trennwandkolonne mit 50 bis 300 theoretischen Trennstufen und einer oder mehrerer Seitenabzugsstellen bei einem absoluten Betriebsdruck von 5 bis 500 mbar durchführt.

Die dazu einzusetzende Trennwandkolonne weist eine Gesamtstufenzahl von 50 bis 300, bevorzugt 100 bis 200 und ganz besonders bevorzugt 120 bis 180 theoretischen Trennstufen und eine oder mehrere, bevorzugt 1 bis 3, insbesondere 1 oder 2 und ganz besonders bevorzugt 1 Seitenabzugsstelle bzw. Seitenabzugsstellen auf.

Das gemäß optionalem Verfahrensschritt e) bevorzugt durchzuführende Verfahren wird bei einem absoluten Betriebsdruck in der Trennwandkolonne von 5 bis 500 mbar, bevorzugt von 10 bis 200 mbar, besonders bevorzugt von 20 bis 120 mbar und ganz besonders bevorzugt von 20 bis 100 mbar und insbesondere bevorzugt bei einem absoluten Betriebsdruck von 40 bis 100 mbar durchgeführt. Bevorzugt betreibt man dabei die Trennwandkolonne so, dass der absolute Kopfdruck 10 bis 100 mbar, besonders bevorzugt 10 bis 80 mbar, ganz besonders bevorzugt 10 bis 60 mbar, noch mehr bevorzugt 20 bis 60 mbar und insbesondere bevorzugt 40 bis 60 mbar beträgt. Ebenfalls bevorzugt betreibt man dabei die Trennwandkolonne so, dass der absolute Sumpfdruck 20 bis 500 mbar, besonders bevorzugt 30 bis 200 mbar oder besser bis 100 mbar, noch mehr bevorzugt 40 bis 200 mbar oder besser bis 100 mbar und ganz besonders bevorzugt 50 bis 100 mbar beträgt.

Das Rücklaufverhältnis kann bei der Durchführung des bevorzugten Trennverfahrens gemäß Schritt e) in breiten Grenzen variiert werden und liegt üblicherweise bei etwa 5 zu 1 bis etwa 2000 zu 1, bevorzugt etwa 20 zu 1 bis 1000 zu 1 und besonders bevorzugt bei etwa 50 zu 1 bis etwa 500 zu 1. Vorteilhaft ist auch eine Dephlegmator-Fahrweise, d.h. es wird im Kopfkondensator der Kolonne nur der Rücklauf kondensiert und wieder der Kolonne zugeführt. In einem solchen energetisch günstigen Fall der Teilkondensation fällt das auszuschleusende Kopfprodukt ausschließlich im Nachkühler an, der bei niedrigerer Temperatur betrieben werden kann. Dabei ist es vorteilhaft, einen Wärmeträgerkreislauf so vorzusehen, dass die Temperatur des Kühlmediums im Nachkühler in einem Bereich von 5°C bis von etwa 50°C temperiert werden kann, um gegebenenfalls durch Desublimation gebildete Feststoffe von Zeit zu Zeit wieder aufschmelzen zu können.

Aus diesem Grund ist es auch von Vorteil, eine Möglichkeit vorzusehen, den Hauptkondensator und/oder den Nachkondensator der Kolonne mit einem von 0°C bis 60°C, bevorzugt von 20 bis 60°C temperierbaren Wärmeträgermedium (Kühlmedium) zu speisen. Zu diesem Zweck kann beispielsweise Wasser mit Hilfe einer Kreiselpumpe über den Wärmetauscher umgepumpt werden und über eine Temperaturregelung je nach Bedarf kaltes oder warmes Wasser in diesen Umpumpkreis eingespeist werden. Selbstverständlich ist auch eine elektrische Beheizung dieses Kreises mit einem in den Kreislauf eingebauten Durchlauferhitzer oder eine konventionelle Beheizung mit Dampf möglich.

Durch das erfindungsgemäß bevorzugte Trennverfahren gemäß dem optionalen Verfahrensschritt e) ist Menthol, vorzugsweise L-Menthol in reiner oder angereicherter Form zugänglich. Unter dem Begriff "Menthol in angereicherter Form" sind Menthol, bevorzugt L-Menthol-haltige Stoffgemische zu verstehen, die einen höheren Gehalt an Menthol bzw. L-Menthol aufweisen als das jeweils erfindungsgemäß eingesetzte Menthol bzw. bevorzugt L-Menthol enthaltende Stoffgemisch. Bevorzugt ist unter dem Begriff "Menthol in angereicherter Form" solches Menthol, bevorzugt L-Menthol zu verstehen, dass eine Reinheit, d.h. einen Gehalt von mehr als 80 bis 99,5 Gew.-%, bevorzugt von 85 bis 99,5 Gew.-%, besonders bevorzugt von 90 Gew.-% oder noch mehr, bevorzugt von 95 Gew.-% bis 99,5 Gew.-% aufweist. Das im Rahmen von Verfahrensschritt e) bevorzugte Verfahren ermöglicht auch die Herstellung von Menthol, bevorzugt L-Menthol in reiner Form. Unter dem Begriff "Menthol in reiner Form" ist Menthol, bevorzugt L-Menthol mit einem Gehalt von größer oder gleich 99 Gew.-%, bevorzugt größer oder gleich 99,1 Gew.-%, bevorzugt von mindestens 99,2 Gew.-%, weiter bevorzugt von mindestens 99,3 Gew.-%, noch mehr bevorzugt von mindestens 99,4 Gew.-% und insbesondere bevorzugt von mindestens 99,5 Gew.-% bis, wiederum bevorzugt von mindestens 99,6 Gew.-%, weiterhin bevorzugt von mindestens 99,7 Gew.-% und am meisten bevorzugt von 99,8 Gew.-% bis 99,99 Gew.-%, bevorzugt bis 99,98 Gew.-%, besonders bevorzugt bis 99,97 Gew.-%, noch mehr bevorzugt bis 99,96 Gew.-% und am meisten bevorzugt bis 99,95 Gew.% zu verstehen. Dabei beziehen sich die Angaben in Gew.-% wie alle Angaben in Gew.-% im Rahmen der vorliegenden Erfindung auf die Gesamtmenge des jeweiligen Gemisches.

Der Zulauf, d.h. das einzusetzende Stoffgemisch kann flüssig oder gasförmig in die Trennwandkolonne geführt und dort in eine Kopf- und Sumpffraktion sowie einen oder mehrere Seitenabläufe, bevorzugt in einen Seitenablauf aufgetrennt werden. In einem Seitenablauf fällt das Wertprodukt Menthol, bevorzugt L-Menthol in der gewünschten Reinheit, d.h. in angereicherter oder reiner Form an. In einer besonderen Ausführungsform des bevorzugten Verfahrens zur Herstellung von reinem oder angereichertem Menthol ist dem Kopfkondensator der Kolonne ein Nachkondensator nachgeschaltet, der, wie oben ausgeführt, mit einer im Temperaturbereich von 0 bis 60°C, bevorzugt von 20 bis 60°C temperierbaren Kühlflüssigkeit (beispielsweise mit glykolhaltigem Wasser) gekühlt ist und in dem noch eine Menthol-arme Leichtsiederfraktion anfällt.

Verfahrensvarianten für die kontinuierliche destillative Zerlegung von Mehrstoffgemischen sind vorstehend im Rahmen des Verfahrens zur Herstellung von Neral in reiner oder angereicherter Form durch destillative Abtrennung von Neral aus Neral- und Geranial enthaltenden Stoffgemischen aufgeführt.

Figur 1 zeigt eine bevorzugte Ausführungsform der erfindungsgemäß bevorzugt im Rahmen von Verfahrensschritt e) durchzuführenden Auftrennung des einzusetzenden Menthol enthaltenden Stoffgemisches in eine Menthol-arme Kopffraktion (j), eine Menthol-reiche Seitenfraktion (f) und eine Sumpffraktion (g). Der Menthol-haltige Zulauf zur Trennwandkolonne kann flüssig (b), gasförmig (c), bzw. gasförmig und flüssig erfolgen.

Das erfindungsgemäß bevorzugte Verfahren gemäß optionalem Verfahrensschritt e) wird vorzugsweise kontinuierlich durchgeführt. Demzufolge werden die als Ausgangsstoff einzusetzenden Menthol, bevorzugt L-Menthol enthaltenden Stoffgemische der Trennwandkolonne vorzugsweise kontinuierlich zugeführt und die erfindungsgemäß erhaltenen Produkte (Fraktionen) bzw. Nebenprodukte vorzugsweise kontinuierlich ausgetragen.

Der dazu einzusetzenden Kolonne wird üblicherweise ein weiterer Kondensator nachgeschaltet, dessen Arbeitstemperatur 10 bis 40 K, bevorzugt 20 bis 30 K unter der Arbeitstemperatur des Kopfkondensators der Trennwandkolonne liegt. Mit dessen Hilfe kann ein Großteil der noch im Kopfstrom (k) enthaltenen Leichtsieder niedergeschlagen werden.

Darüber hinaus kann es bei Trennwandkolonnen von Vorteil sein, den Zulaufstrom einer Vorverdampfung zu unterziehen und anschließend zweiphasig oder in Form von zwei Strömen der Kolonne zuzuführen. Diese Vorverdampfung bietet sich besonders dann an, wenn der Zulaufstrom größere Mengen an Leichtsiedern enthält. Durch die Vorverdampfung kann der Abtriebsteil der Kolonne wesentlich entlastet werden.

Die bevorzugt im Rahmen von Verfahrensschritt e) einzusetzenden Trennwandkolonnen können sowohl als Packungskolonnen mit Füllkörpern oder geordneten Packungen oder als Bodenkolonnen ausgeführt werden. Bei dem erfindungsgemäß bevorzugten Verfahren zur Herstellung von Menthol in reiner oder angereicherter Form empfiehlt es sich, Packungskolonnen einzusetzen. Dabei sind geordnete Blech- oder Gewebepackungen mit einer spezifischen Oberfläche von etwa 100 bis 750 m²/m³, bevorzugt etwa 350 bis 500 m²/m³ besonders geeignet.

Falls, wie im Fall der vorliegenden Trennung, besonders hohe Anforderungen an die Reinheiten der Produkte gestellt werden, ist es günstig, die Trennwand mit einer thermischen Isolierung auszustatten. Eine Beschreibung der verschiedenen Möglichkeiten der thermischen Isolierung der Trennwand findet sich in EP-A 0 640 367. Eine doppelwandige Ausführung mit einem zwischen liegenden engen Gasraum ist besonders günstig.

Bei der Trennung von Mehrstoffgemischen in eine Leichtsieder-, Mittelsieder- und Hochsiederfraktion existieren üblicherweise Spezifikationen über den maximal zulässigen Anteil an Leichtsiedern und Hochsiedern in der Mittelsiederfraktion. Hierbei werden entweder einzelne für das Trennproblem kritische Komponenten, sogenannte Schlüsselkomponenten, oder die Summe von mehreren Schlüsselkomponenten spezifiziert. Diese Schlüsselkomponenten sind im Rahmen der vorliegenden Erfindung Isomenthol als hochsiedende Nebenkomponente und Neomenthol bzw. ein Gemisch von Neo- und Neosiomenthol als tiefsiedende Nebenkomponente.

Die Einhaltung der Spezifikation für die Hochsieder in der Mittelsiederfraktion kann beispielsweise über das Aufteilungsverhältnis der Flüssigkeit am oberen Ende der Trennwand geregelt werden. Dabei wird das Aufteilungsverhältnis der Flüssigkeit am oberen Ende der Trennwand vorzugsweise so eingestellt, dass die Konzentration der Schlüsselkomponenten für die Hochsiederfraktion in der Flüssigkeit am oberen Ende der Trennwand 10 bis 80%, bevorzugt 30 bis 50%, des Wertes ausmacht, der im Seitenabzugsprodukt erzielt werden soll. Die Flüssigkeitsaufteilung wird vorzugsweise dahingehend eingestellt, dass bei höheren Gehalten an Schlüsselkomponenten der Hochsiederfraktion mehr und bei niedrigeren Gehalten an Schlüsselkomponenten der Hochsiederfraktion weniger Flüssigkeit auf den Zulaufteil geleitet wird.

Entsprechend kann die Spezifikation für die Leichtsieder in der Mittelsiederfraktion durch die Heizleistung geregelt werden. Hierbei wird beispielsweise die Heizleistung im Verdampfer so eingestellt, dass die Konzentration an Schlüsselkomponenten der Leichtsiederfraktion in der Flüssigkeit am unteren Ende der Trennwand 10 bis 80, bevorzugt 30 bis 50% des Wertes ausmacht, der im Seitenabzugsprodukt erzielt werden soll, und die Heizleistung wird vorzugsweise dahingehend eingestellt, dass bei höherem Gehalt an Schlüsselkomponenten der Leichtsiederfraktion die Heizleistung erhöht und bei niedrigerem Gehalt an Schlüsselkomponenten der Leichtsiederfraktion die Heizleistung verringert wird.

Zur Kompensation von Störungen der Zulaufmenge oder der Zulaufkonzentration erwies es sich zudem als vorteilhaft, durch einen entsprechenden Regelmechanismus beispielsweise durch geeignete Regelvorschriften im Prozessleitsystem sicherzustellen, dass die Mengenströme der Flüssigkeiten, die auf die Kolonnenteile (2), d.h. den Verstärkungsteil des Zulaufteils, und (5), d.h. den Abtriebsteil des Entnahmeteils, nicht unter 30% ihres Normalwertes sinken können.

Zur Entnahme und Aufteilung der Flüssigkeiten am oberen Ende der Trennwand und an der Seitenentnahmestelle eignen sich sowohl innenliegende als auch außerhalb der Kolonne angeordnete Auffangräume für die Flüssigkeit, welche die Funktion einer Pumpenvorlage übernehmen oder für eine ausreichend hohe statische Flüssigkeitshöhe sorgen, die eine durch Stellorgane, beispielsweise Ventile, geregelte Flüssigkeitsweiterleitung ermöglichen. Bei der Verwendung von gepackten Kolonnen wird die Flüssigkeit zunächst in Sammlern gefasst und von dort aus in einen Innen liegenden oder Außen liegenden Auffangraum geleitet.

Im Rahmen einer besonders bevorzugten Ausführungsform führt man das erfindungsgemäß bevorzugte Trennverfahren gemäß optionalem Verfahrensschritt e) in einer Anlage durch, wie sie schematisch in Figur 1 gezeigt ist. Die bevorzugte Ausführungsform zeichnet sich dadurch aus, dass man eine Trennwandkolonne (TK) einsetzt, die eine Trennwand (T) in Kolonnenlängsrichtung unter Ausbildung eines oberen gemeinsamen Kolonnenbereichs (1), eines unteren gemeinsamen Kolonnenbereichs (6), eines Zulaufteils (2, 4) mit Verstärkungsteil (2) und Abtriebsteil (4) sowie eines Entnahmeteils (3, 5) mit Abtriebsteil (3) und Verstärkungsteil (5) aufweist.

Das als Einsatzstoff dienende Menthol enthaltende Stoffgemisch (a) wird vorzugsweise in den mittleren Bereich des Zulaufteils (2, 4) zugeführt, das Menthol, bevorzugt L-Menthol in reiner oder angereicherter Form als Seitenabzug aus dem mittleren Bereich des Entnahmeteils (3, 5) gewonnen und eine oder mehrere Leichtsiederfraktionen aus dem oberen gemeinsamen Kolonnenbereich (1) und eine oder mehrere Hochsiederfraktionen aus dem unteren gemeinsamen Kolonnenbereich (6) abgeführt.

Der Zulaufstrom (a) kann über einen Vorheizer (VH) als flüssiger (b), gasförmiger (c) oder teilweise flüssig und gasförmiger Strom in die Kolonne (TK) eingeleitet werden. Der Kopfstrom der Kolonne wird im Kondensator (K) ganz oder teilweise kondensiert. Bei einer teilweisen Kondensation (Dephlegmator-Betrieb) enthält der Abgasstrom (k) des Kopfkondensators (K) üblicherweise noch merkliche Mengen an kondensierbaren Leichtsiedern, die dann in einem bei niedriger Temperatur betriebenen Nachkondensator niedergeschlagen werden können.

Der Kopfkondensator (K) und/oder der Nachkondensator können beispielsweise als Plattenapparat ausgeführt und in den Kolonnenmantel, bevorzugt in den Kolonnenkopf integriert sein. Zur Vermeidung von Feststoffbildung kann es von Vorteil sein, den Kondensator der Kolonne zu temperieren, beispielsweise auf Temperaturen von etwa 30 bis etwa 50°C.

Das im Kondensator (K) niedergeschlagene Kopfprodukt wird in dem Destillatbehälter (DB) gepuffert und über die Rücklaufpumpe (RP) als Kolonnenrücklauf (i) der Kolonne wieder zugeführt. Bei Bedarf lässt sich hieraus auch eine Destillatfraktion (j) gewinnen. Bei Integration des Kondensators in den Kolonnenkopf kann auf den Destillatbehälter (DB) und die Rücklaufpumpe verzichtet (RP) werden.

Der Sumpfstrom wird vorteilhaft über die Umlaufpumpe (UP) dem Sumpfverdampfer (SV) zugeführt, der bevorzugt als Fallfilmverdampfer ausgeführt ist. Diesem Umpumpstrom kann auch der Sumpfaustrag (g) der Kolonne (TK) entnommen werden. Vorteilhaft wird der Sumpfstrom (Hochsiederfraktion) der Kolonne als flüssiger Strom (h) hinter dem Sumpfverdampfer, ggf. mit Hilfe einer kleineren Pumpe (SP), entnommen.

Als Sumpfverdampfer für die Trennwandkolonne kann vorteilhaft ein Dünnschichtapparat, beispielsweise ein Fallfilmverdampfer, verwendet werden.

Das Wertprodukt, d.h. Menthol bzw. L-Menthol in reiner oder angereicherter Form wird kann als flüssiger Seitenabzug, Strom (f), aus dem Entnahmeteil der Trennwandkolonne (TK) abgezogen werden. Es ist auch möglich, bei Bedarf den Wertprodukt-Strom (f) als gasförmigen Abzug zu entnehmen, dann wird aber üblicherweise ein weiterer Kondensator benötigt. Aufgrund des Festpunktes von L-Menthol in reiner oder angereicherter Form zwischen 41 und 44°C ist es vorteilhaft, alle Produkt führenden Apparate (neben der Kolonne auch alle Behälter und Pumpen) und Leitungen sowie bevorzugt alle Apparate und Leitungen des Vakuumsystems zu isolieren, d.h. thermisch mit geeigneten Materialien zu isolieren und mit Begleitheizungen zu versehen. Dabei sind z.B. den Rohren beigelegte elektrische Heizleitungen, die mit geeigneten Geräten auf Temperaturen von bis zu 70°C, bevorzugt von 45 bis 70°C, noch mehr bevorzugt auf Temperaturen bis zu 60°C, insbesondere bevorzugt von 45 bis 60°C geregelt werden, vorteilhaft. Alternativ können auch konventionelle Begleitheizungssysteme wie z.B. durch mit Warmwasser durchströmte Doppelmantelrohre zum Einsatz kommen.

Der obere gemeinsame Teilbereich (1) der Kolonne weist üblicherweise 5 bis 50%, der Verstärkungsteil (2) des Zulaufteils der Kolonne 5 bis 50%, der Abtriebsteil (4) des Zulaufteils der Kolonne 2 bis 50%, der Abtriebsteil (2) des Entnahmeteils der Kolonne 5 bis 50%, der Verstärkungsteil (5) des Entnahmeteils 2 bis 50%, und der gemeinsame untere Teil (6) der Kolonne 5 bis 50% der Gesamtzahl der theoretischen Trennstufen der Kolonne auf, wobei sich die gewählten Prozentangaben zu 100% ergänzen.

Bevorzugt weist der obere gemeinsame Teilbereich (1) der Kolonne 10 bis 25%, der Verstärkungsteil (2) des Zulaufteils der Kolonne 15 bis 30%, der Abtriebsteil (4) des Zulaufteils der Kolonne 15 bis 30%, der Abtriebsteil (2) des Entnahmeteils der Kolonne 15 bis 30%, der Verstärkungsteil (5) des Entnahmeteils 15 bis 30%, und der gemeinsame untere Teil (6) der Kolonne 10 bis 25% der Gesamtzahl der theoretischen Trennstufen der Kolonne auf, wobei sich die gewählten Prozentangaben zu 100% ergänzen.

Die Summe der Zahl der theoretischen Trennstufen der Teilbereiche (2) und (4) im Zulaufteil beträgt bevorzugt 80 bis 110%, besonders bevorzugt 95 bis 105% der Summe der Zahl der Trennstufen der Teilbereiche (3) und (5) im Entnahmeteil.

Im Rahmen einer bevorzugten Ausführungsform des bevozugten Trennverfahrens gemäß Verfahrensschritt e) sind die Zulaufstelle und die Seitenabzugsstelle hinsichtlich der Lage der theoretischen Trennstufen auf unterschiedlicher Höhe in der Kolonne angeordnet, indem die Zulaufstelle um 1 bis 40, bevorzugt um 5 bis 20 theoretische Trennstufen höher oder niedriger angeordnet ist als die Seitenabzugsstelle.

Es hat sich darüber hinaus als vorteilhaft erwiesen wenn der durch die Trennwand unterteilte Teilbereich der Kolonne bestehend aus den Teilbereichen (2), (3), (4) und (5) oder Teilen davon mit geordneten Packungen oder Füllkörpern (beispielsweise Gewebepackungen wie Montz A3-500, Sulzer BX oder CY oder Blechpackungen wie Montz B1-500 (Fa. Montz) oder Mellapak (Fa. Sulzer) bestückt ist.

Der Brüdenstrom am unteren Ende der Trennwand kann durch die Wahl und/oder Dimensionierung der Trenneinbauten und/oder den Einbau druckverlusterzeugender Vorrichtungen, beispielsweise von Blenden, so eingestellt wird, dass das Verhältnis des Brüdenstroms im Zulaufteil zu dem des Entnahmeteils 0,8 bis 1,2, bevorzugt 0,9 bis 1,1 beträgt.

Die aus dem oberen gemeinsamen Teil (1) der Kolonne ablaufende Flüssigkeit wird vorteilhaft in einem in der Kolonne oder außerhalb der Kolonne angeordneten Auffangraum gesammelt und gezielt durch eine Festeinstellung oder Regelung am oberen Ende der Trennwand so aufgeteilt, dass das Verhältnis des Flüssigkeitsstroms zum Zulaufteil zu dem zum Entnahmeteil 0,1 bis 2,0 bei größtenteils flüssigen Zulauf und 1,0 bis 2 bei gasförmigem Zulauf beträgt. Dabei ist der flüssige Zulauf erfindungsgemäß bevorzugt.

Die aus dem oberen gemeinsamen Teilbereich (1) auf den Zulaufteil ablaufende Flüssigkeit kann über eine Pumpe gefördert oder über eine statische Zulaufhöhe von mindestens 1 m mengengeregelt aufgegeben werden, bevorzugt über eine Kaskadenregelung in Verbindung mit der Flüssigkeitsstandregelung des Auffangraums. Die Regelung wird bevorzugt so eingestellt, dass die auf den Zulaufteil aufgegebene Flüssigkeitsmenge nicht unter 30 % des gewünschten Normalwertes sinken kann. Darüber hinaus wird die Aufteilung der aus dem Teilbereich (3) im Entnahmeteil der Kolonne ablaufenden Flüssigkeit auf den Seitenabzug und auf den Teilbereich (5) im Entnahmeteil der Kolonne durch eine Regelung vorteilhaft so eingestellt, dass die auf den Teilbereich (5) aufgegebene Flüssigkeitsmenge nicht unter eine Höhe von 30% des gewünschten Normalwertes sinken kann. Als Normalwert sind dabei vorteilhaft die zwei- bis vierfache Menge, bezogen auf die Zulaufmenge anzunehmen.

Die Trennwandkolonne weist vorzugsweise am oberen und unteren Ende der Trennwand Probenahmemöglichkeiten auf, aus der Kolonne kontinuierlich oder in zeitlichen Abständen flüssig oder gasförmig Proben entnommen und hinsichtlich ihrer Zusammensetzung, bevorzugt gaschromatographisch, untersucht werden können.

Das Aufteilungsverhältnis der Flüssigkeit am oberen Ende der Trennwand wird vorzugsweise so eingestellt, dass die Konzentration an denjenigen Komponenten der Hochsiederfraktion, für die im Seitenabzug ein bestimmter Grenzwert für die Konzentration erzielt werden soll (speziell Isomenthol), in der Flüssigkeit am oberen Ende der Trennwand 10 bis 80% des Wertes ausmacht, der im Seitenabzugsprodukt erzielt werden soll. Die Flüssigkeitsaufteilung sollte bevorzugt dahingehend eingestellt werden, dass bei höheren Gehalten an Komponenten der Hochsiederfraktion mehr und bei niedrigeren Gehalten an Komponenten der Hochsiederfraktion weniger Flüssigkeit auf den Zulaufteil geleitet wird.

Die Heizleistung im Verdampfer (SV) stellt man bevorzugt so ein, dass die Konzentration an denjenigen Komponenten der Leichtsiederfraktion, für die im Seitenabzug ein bestimmter Grenzwert für die Konzentration erzielt werden soll (speziell Neoisomenthol), in der Flüssigkeit am unteren Ende der Trennwand 10 bis 80% des Wertes ausmacht, der im Seitenabzugsprodukt erzielt werden soll. Die Heizleistung stellt man vorteilhaft dahingehend ein, dass bei höherem Gehalt an Komponenten der Leichtsiederfraktion die Heizleistung erhöht und bei niedrigerem Gehalt an Komponenten der Leichtsiederfraktion die Heizleistung verringert wird.

Die Destillatentnahme, d.h. die Entnahme der tiefsiedenden Nebenprodukte erfolgt bevorzugt temperaturgeregelt oder aber mengengeregelt, in Abhängigkeit der Menge der im Zulaufgemisch vorhandenen abzutrennenden leichtersiedenden Nebenkomponenten. Als Regeltemperatur verwendet man mit Vorteil eine Messstelle im Teilbereich (1) der Kolonne, die um 3 bis 10, bevorzugt 4 bis 6 theoretische Trennstufen unterhalb des oberen Endes der Kolonne angeordnet ist.

Die Entnahme des Sumpfprodukts erfolgt bevorzugt temperaturgeregelt oder aber mengengeregelt in Abhängigkeit von der Zulaufmenge.

Die Entnahme des als Seitenprodukt gewonnenen Verfahrensproduktes Menthol, bevorzugt L-Menthol in reiner oder angereicherter Form erfolgt vorzugsweise standgeregelt, wobei als Regelgröße vorzugsweise der Flüssigkeitsstand im Kolonnensumpf verwendet wird.

Der Zulaufstrom des erfindungsgemäß einzusetzenden Menthol-haltigen Stoffgemisches wird vorzugsweise teilweise oder vollständig vorverdampft und der Kolonne zweiphasig oder in Form eines gasförmigen und eines flüssigen Stromes zugeführt.

Im Rahmen einer bevorzugten Ausführungsform setzt man auch im Rahmen des optionalen Verfahrensschritts e) des erfindungsgemäßen Verfahrens eine Trennwandkolonne ein, deren Trennwand nicht in die Kolonne eingeschweißt ist, sondern in Form von lose gesteckten und adäquat abgedichteten Teilsegmenten gestaltet ist.

Die Flüssigkeitsverteilung in den einzelnen Teilbereichen der Kolonne kann bevorzugt gezielt ungleichmäßig eingestellt werden, wobei insbesondere in den Teilbereichen (2) und (5) die Flüssigkeit verstärkt im Wandbereich aufgegeben wird und in den Teilbereichen (3) und (4) die Flüssigkeit reduziert im Wandbereich aufgegeben wird.

Das Verteilungsverhältnis der rücklaufenden Flüssigkeit zwischen Abnahme- und Zulaufseite der Trennwand beträgt vorzugsweise etwa 1 zu 1 bis etwa 3 zu 1, bevorzugt etwa 1 zu 1 bis etwa 2 zu 1.

Die Lage der Trennwand in den einzelnen Teilbereichen der Kolonne kann vorteilhaft so angepasst werden, dass die Querschnitte von Zulauf- und Entnahmeteil verschiedene Flächen aufweisen.

Das erfindungsgemäß zugängliche L-Menthol in reiner oder angereicherter Form kann über den Seitenabzug, bzw. im Fall, dass weitere Seitenabzüge vorgesehen sind, über den mittleren Seitenabzug (f) bevorzugt kontinuierlich gewonnen werden und weist im Rahmen einer bevorzugten Ausführungsform einen Menthol-Gehalt von über 99,5 Gew.-%, bevorzugt von 99,5 bis 99,95 Gew.-%, und einen Gehalt der anderen, wie vorstehend beschriebenen Diastereomere des Menthols von bis zu 0,3 Gew.-% (jeweils bezogen auf das erhaltene Produkt), gegebenenfalls neben geringsten Mengen weiterer Verunreinigungen, auf.

Im Rahmen einer weiteren bevorzugten Ausführungsform weist das erfindungsgemäß erhaltene Menthol, bevorzugt L-Menthol in reiner oder angereicherter, bevorzugt in reiner Form einen Gehalt an Isopulegol und dessen wie vorstehend beschriebenen Diasteromeren von zusammen bis zu 0,5 Gew.-%, bevorzugt bis zu 0,3 und besonders bevorzugt bis zu 0,1 Gew.-% (bezogen auf die Gesamtmenge des erhaltenen Produkts) auf. Im Rahmen einer weiteren bevorzugten Ausführungsform weist das erfindungsgemäß erhaltene Menthol, bevorzugt L-Menthol in reiner oder angereicherter, bevorzugt in reiner Form einen Gehalt an Menthon und Isomenthon von bis zu 0,5 Gew.-%, bevorzugt bis zu 0,3 und besonders bevorzugt bis zu 0,1 Gew.-% (bezogen auf die Gesamtmenge des erhaltenen Produkts) auf.

Ein weiterer Aspekt dieses optionalen Verfahrensschrittes e) der vorliegenden Erfindung betrifft eine Vorrichtung zur Durchführung des wie vorstehend beschriebenen kontinuierlichen Destillationsverfahrens zur Herstellung von racemischem oder optisch aktivem Menthol in reiner oder angereicherter Form.

Die erfindungsgemäße Vorrichtung ist in Fig. 1 dargestellt und umfasst eine Trennwandkolonne (TK) mit 50 bis 300 theoretischen Trennstufen und einer oder mehrerer Seitenabzugsstellen, die eine Trennwand (T) in Kolonnenlängsrichtung unter Ausbildung eines oberen gemeinsamen Kolonnenbereichs (1), eines unteren gemeinsamen Kolonnenbereichs (6), eines Zulaufteils (2, 4) mit Verstärkungsteil (2) und Abtriebsteil (4) sowie eines Entnahmeteils (3, 5) mit Abtriebsteil (3) und Verstärkungsteil (5) aufweist, wobei alle Produkt führenden Bestandteile der Vorrichtung (neben der Kolonne auch alle Behälter, Pumpen und Leitungen) sowie bevorzugt alle Apparate und Leitungen des Vakuumsystems mit geeigneten Materialien thermisch isoliert und mit Begleitheizungen versehen sind.

Dabei sind wie bereits vorstehend beschrieben, z.B. den Rohren beigelegte elektrische Heizleitungen vorteilhaft, die mit geeigneten Geräten auf Temperaturen von bis zu 70°C, bevorzugt von 45 bis 70°C, noch mehr bevorzugt auf Temperaturen bis zu 60°C, insbesondere bevorzugt von 45 bis 60°C geregelt werden. Alternativ können auch konventionelle Begleitheizungssysteme wie z.B. durch mit Warmwasser durchströmte Doppelmantelrohre zum Einsatz kommen.

Die vorstehend beschriebenen Verfahrensschritte a) bis d) sowie die optionalen, d.h. gewünschtenfalls zusätzlich durchzuführenden Verfahrensschritte 0) und e) eröffnen einen aus wirtschaftlicher und verfahrenstechnischer Sicht sehr vorteilhaften Zugang zu hochreinem Menthol. Dabei kann wahlweise optisch aktives oder racemisches Menthol hergestellt werden. Die vorligegende Erfindung betrifft daher in einer bevorzugten Ausführungsform auch ein Verfahren zur Herstellung von optisch aktivem Menthol, umfassend die Verfahrensschritte
a.2) asymmetrische katalytische Hydrierung von Neral und/oder Geranial zu optisch aktivem Citronellal,
b.2) Cyclisierung von gemäß Schritt a.2) erhaltenem optisch aktivem Citronellal zu optisch aktivem Isopulegol in Gegenwart eines sauren Katalysators,
c.2) Aufreinigung von gemäß Schritt b.2) erhaltenem optisch aktivem Isopulegol durch Kristallisation und
d.2) katalytische Hydrierung von gemäß Schritt c.2) erhaltenem optisch aktivem Isopulegol zu optisch aktivem Menthol.

Gemäß Verfahrensschritt a.2) führt man eine asymmetrische Hydrierung von Neral und/oder Geranial, d.h. von Neral der Formel (II) oder Geranial der Formel (III) oder wie vorstehend beschriebenen Neral- und Geranial enthaltenden Stoffgemischen durch. Bevorzugt führt man eine wie vorstehend unter Verfahrensschritt a) beschriebene asymmetrische katalytische Hydrierung von reinem oder angereichertem Neral durch. In einer wiederum bevorzugten Ausführungsform führt man diese asymmetrische katalytische Hydrierung gemäß Verfahrensschritt a.2) im Anschluss an den optionalen Verfahrensschritt 0) zur Herstellung von reinem oder angereichertem Neral durch destillative Abtrennung von Neral aus Stoffgemischen enthaltend Geranial und Neral durch. Auf diese Weise ist optisch aktives Citronellal, wahlweise, je nach Ausgestaltung der asymmetrischen katalytischen Hydrierung, in Form eines der beiden Enantiomere, bevorzugt in Form von D-Citronellal zugänglich.

Das gemäß Verfahrensschritt a.2) zugängliche optisch aktive Citronellal kann dann gemäß Verfahrensschritt b.2) zu optisch aktivem Isopulegol in Gegenwart eines sauren Katalysators cyclisiert werden. Als geeignete saure Katalysatoren seien die vorstehend unter Verfahrensschritt b) beschriebenen sauren oder Lewis-sauren Katalysatoren genennt, insbesondere die genannten Aluminium-haltigen Lewis-sauren Katalysatoren, wie die erfindungsgemäß bevorzugten Diarylphenoxy-Aluminium-Verbindungen, die erhältlich sind durch Umsetzung der vorstehend genannten Liganden der Formel (I) mit speziellen Aluminiumverbindungen, wie zum Beispiel Trimethyl- oder Triethylaluminium.

Das so zugängliche optisch aktive Isopulegol wird gemäß Verfahrensschritt c.2) im Rahmen dieser bevorzugten Ausführungsform durch Kristallisation aufgereinigt. Dabei können im Prinzip alle dem Fachmann an sich bekannten Kristallisationsverfahren aus der Lösung wie auch aus der Schmelze angewendet werden. Bevorzugt führt man eine wie unter Verfahrensschritt c) beschriebene Kristallisation aus der Schmelze, besonders bevorzugt eine statische Schmelzekristallisation und ganz besonders bevorzugt eine wie vorstehend beschriebene statische Schichtkristallisation durch. Dabei erhält man im Rahmen dieser bevorzugten Ausführungsform des erfindungsgemäßen Verfahrens aufgereinigtes, d.h. wie vorstehend beschriebenes, enantiomeren- und diastereomerenangereichertes Isopulegol, bevorzugt L-Isopulegol.

Das so zugängliche optisch aktive Isopulegol wird dann im Rahmen dieser Ausführungsform des erfindungsgemäßen Verfahrens gemäß Verfahrensschritt d.2) katalytisch zu optisch aktivem Menthol hydriert. Die katalytische Hydrierung von Isopulegol zu Menthol ist dem Fachmann bekannt und kann mit einer Vielfalt üblicher hetereogener Hydrierkatalysatoren durchgeführt werden. Es hat sich als vorteilhaft erwiesen, die katalytische Hydrierung in Gegenwart der vorstehend unter Verfahrensschritt d) beschriebenen Nickel-, Kupfer, Zirkon- und molybdän-haltigen Katalysatoren durchzuführen, da dabei lediglich im geringstmöglichen Ausmaß unerwünschte Nebenprodukte wie die vorstehend beschriebenen Diastereomere des Menthols bzw. die Menthone gebildet werden, was insbesondere für die geruchlichen und geschmacklichen Eigenschaften des so erhaltenen Produkt von Bedeutung ist.

Gemäß einer weiteren bevorzugten Ausführungsform betrifft die vorliegende Erfindung ein Verfahren zur Herstellung von enantiomerenangereichertem optisch aktivem Menthol und racemischem oder enantiomerenabgreichertem Menthol, umfassend die Verfahrensschritte
a.3) asymmetrische katalytische Hydrierung von Neral und/oder Geranial zu optisch aktivem Citronellal mit einem Enantiomerenüberschuss im Bereich von 70 bis 99%,
b.3) Cyclisierung von gemäß Schritt a.3) erhaltenem optisch aktivem Citronellal zu optisch aktivem Isopulegol in Gegenwart eines sauren Katalysators,
c.3) Aufreinigung von gemäß Schritt b.3) erhaltenem optisch aktivem Isopulegol durch Kristallisation unter Erhalt von enantiomerenangereichertem Isopulegol und (entweder) racemischem oder enantiomerenabgereichertem Isopulegol und
d.3) katalytische Hydrierung von gemäß Schritt c.3) erhaltenem enantiomerenangereichertem optisch aktivem Isopulegol zu optisch aktivem Menthol und katalytische Hydrierung von racemischem oder enantiomerenabgereichertem Isopulegol zu racemischem oder enantiomerenabgereichertem Menthol.

Gemäß Schritt a.3) dieser bevorzugten Ausführungsform des erfindungsgemäßen Verfahrens führt man eine, wie beispielsweise vorstehend unter Verfahrenschritt a) beschriebene, asymmetrische katalytische Hydrierung von Neral oder Geranial zu optisch aktivem Citronellal mit einem Enantiomerenüberschuss im Bereich von 70 bis 99%, bevorzugt 80 bis 99% ee und besonders bevorzugt 85 bis 95 % ee durch. Bevorzugt setzt man dazu Neral, besonders bevorzugt wie vorstehend unter dem optionalen Verfahrensschritt 0) beschrieben zugängliches Neral in angereicherter oder reiner Form ein. Als geeignetes Katalysatorsystem zur Durchführung der asymmetrischen, d.h. e-nantoselektiven Hydrierung hat sich insbesondere der vorstehend beschriebene, aus einer im Reaktionsgemisch löslichen Rhodium-Verbindung und insbesondere den chiralen Liganden (R,R)-Chiraphos oder (S,S)-Chiraphos, bevorzugt (R,R)-Chiraphos gebildete Katalysator erwiesen. Bevorzugt behandelt man den einzusetzenden Katalysator mit einem Kohlenmonoxid und Wasserstoff enthaltenden Gasgemisch vor und/oder führt die Hydrierung in Gegenwart von dem Reaktionsgemisch zusätzlich zugeführtem Kohlenmonoxid durch, wie in der WO 2006/040096 beschrieben. Es hat sich darüber hinaus, wie unter Verfahrensschritt a) beschrieben, als vorteilhaft erwiesen, überschüssiges Kohlenmonoxid nach der Vorbehandlung des Katalysators abzutrennen und die asymmetrische Hydrierung in Gegenwart von Wasserstoff mit einem Kohlenmonoxidgehalt von 100 bis 1200 ppm durchzuführen.

Der Enantiomerenüberschuss des so zugänglichen Citronellals kann dabei über die Reinheit des eingesetzten Nerals, insbesondere über den Gehalt an Geranial im einzusetzenden Neral gesteuert werden, da unter den gewählten Reaktionsbedingungen, beispielsweise bei der wie vorstehend unter Verfahrenssschritt a) beschriebenen asymmetischen Hydrierung von Neral in Gegenwart eines aus einer Rhodiumverbindung und (R,R)-Chiraphos gebildeten Katalysators Neral zum gewünschten D-Citronellal mit hoher asymmetrischer Induktion umgesetzt wird, wohingegen aus dem gegebenenfalls vorliegenden Geranial das andere Enantiomere L-Citronellal gebildet wird.

Das so zugängliche optisch aktive Citronellal mit einem Enatiomerenüberschuss von 70 bis 99%, bevorzugt 80 bis 99% ee und besonders bevorzugt 85 bis 95 % ee wird dann gemäß Schritt b.3) zu optisch aktivem Isopulegol in Gegenwart eines sauren Katalysators cyclisiert, vorzugsweise nach dem vorstehend unter Verfahrensschritt b) sowie in der WO 2006/092433 beschriebenen Verfahren in Gegenwart von Diarylphenoxy-Aluminium-Verbindungen. Dabei erhält man in der Regel weitgehend ohne Verlust der absoluten stereochemischen Information des eingesetzten optisch aktiven Citronellals optisch aktives Isopulegol, das sich üblicherweise bereits durch eine hohe Diastereomerenreinheit auszeichnet.

Gemäß Schritt c.3) dieser bevorzugten Ausführungsform des erfindungsgemäßen Verfahrens führt man eine Aufreinigung des so erhaltenen, optisch aktiven Isopulegols durch Kristallisation unter Erhalt von enantiomerenangereichertem Isopulegol und entweder racemischem oder enantiomerenabgereichertem Isopulegol durch. Die Kristallisation kann dabei nach dem Fachmann bekannten Methoden, beispielsweise als Lösungskristallisation, durchgeführt werden. Bevorzugt führt man jedoch eine wie vorstehend unter Verfahrensschritt b) beschriebene Schmelzekristallisation, besonders bevorzugt in Form einer statischen Schichtkristallisation durch. Dabei gelingt es, das gemäß Verfahrensschritt b.3) erhaltene optisch aktive Isopulegol weiter aufzureinigen, insbesondere im Hinblick auf die Enantiomeren- und Diastereomerenreinheit. Man erhält neben enantiomerenangereichertem optisch aktivem Isopulegol von hoher Enantiomerenreinheit auch wie vorstehend unter Verfahrensschritt b) beschriebenes enantiomerenabgereichertes oder racemisches Isopulegol. Beide so erhaltenen Produkte können einer gesonderten Weiterbehandlung zugeführt werden.

Gemäß Schritt d.3) dieser Ausführungsform des erfindungsgemäßen Verfahrens führt man eine katalytische Hydrierung von enantiomerenangereichertem optisch aktivem Isopulegol zu enantiomerenangreichertem optisch aktivem Menthol und eine katalytische Hydrierung von racemischem oder enantiomerenabgereichertem Isopulegol zu racemischem oder enantiomerenabgereichertem Menthol durch. Die beiden Hydrierungen werden separat durchgeführt, so dass es zu keiner Rückvermischung der durch die vorangegangene Kristallisation getrennten enantiomerenangereicherten und racemischen bzw. enatiomerenabgereicherten Ströme kommen kann. Zur katalytischen Hydrierung der ethylenischen Doppelbindung des Isopulegols stehen dem Fachmann auch dabei verschiedene Katalysatorsysteme zur Verfügung, wie unter Verfahrenschritt d) beschrieben. Es hat sich alllerdings, wie bereits vorstehend unter Verfahrensschritt d) erwähnt, als vorteilhaft erwiesen, die katalytische Hydrierung in Gegenwart der vorstehend unter Verfahrensschritt d) beschriebenen Nickel-, Kupfer, Zirkon- und Molybdän-haltigen Katalysatoren durchzuführen, insbesondere wenn eine hohe Enantio- und Diastereomerenreinheit des Produktes gewünscht ist.

Eine im Rahmen der vorliegenden Erfindung insbesondere bevorzugte Ausführungsform betrifft ein Verfahren zur Herstellung von L-(-)-Menthol, umfassend die Verfahrensschritte
a.4)asymmetrische katalytische Hydrierung von Neral zu D-(+)-Citronellal,
b.4) Cyclisierung von gemäß Schritt a.4) erhaltenem D-(+)-Citronellal zu L-(-)-Isopulegol in Gegenwart eines sauren Katalysators,
c.4) Aufreinigung von gemäß Schritt b.4) erhaltenem L-(-)-Isopulegol durch Kristallisation und
d.4) katalytische Hydrierung von gemäß Schritt c.4) erhaltenem L-(-)-Isopulegol zu L-(-)-Menthol.

Als Ausgangsstoff zur Durchführung dieser bevorzugten Ausführungsform des erfindungsgemäßen Verfahrens dient Neral, vorzugsweise solches in reiner oder angereicherter Form, wie es beipielsweise durch den vorstehend beschriebenen optionalen Verfahrensschritt 0) aus Geranial- und Neral-haltigen Gemischen hergestellt werden kann. Eine wiederum bevorzugte Variante dieser Ausführungsform umfasst demnach zusätzlich den Verfahrensschritt 0) zur Herstellung von Neral in reiner oder angereicherter Form durch destillative Trennung von Neral- und Geranial-haltigen Stoffgemischen.

Gemäß Schritt a.4) setzt man Neral durch katalytische asymmetrische Hydrierung zu D-(+)-Citronellal, d.h. (R)-Citronellal um, vorzugsweise nach dem vorstehend unter Verfahrensschritt b) beschriebenen Verfahren unter Einsatz von (R,R)-Chiraphos als chiralem Ligand und besonders bevorzugt in Gegenwart von Kohlenmonoxid. Durch das wie unter Verfahrensschritt b) beschriebene Cyclisierungsverfahren in Gegenwart eines sauren, vorzugsweise Lewis-sauren, Aluminium-haltigen Katalysators, wie beispielsweise den in der EP-A-1 225 163 beschriebenen, oder den bevorzugten Diarylphenoxy-Aluminium-Verbindungen, erhält man gemäß Schritt b.4) aus D-(+)-Citronellal L-(-)-Isopulegol. Dieses kann, wie vorstehend beschrieben, durch Kristallisation, bevorzugt durch Schmelzekristallisation gemäß Schritt c.4) aufgereinigt und anschließend gemäß Schritt d.4) durch wie vorstehend beschriebene katalytische Hydrierung in L-(-)-Menthol überführt werden.

Eine im Rahmen der vorliegenden Erfindung ganz besonders bevorzugte Ausführungsform betrifft ein Verfahren zur Herstellung von L-(-)- Menthol und racemischem oder enantiomerenabgereichertem Menthol, umfassend die Verfahrensschritte
a.5) asymmetrische katalytische Hydrierung von Neral zu D-(+)-Citronellal mit einem Enantiomerenüberschuss von 70 bis 99%,
b.5) Cyclisierung von gemäß Schritt a.5) erhaltenem D-(+)-Citronellal zu L-(-)-Isopulegol in Gegenwart eines sauren Katalysators,
c.5) Aufreinigung von gemäß Schritt b.5) erhaltenem L-(-)-Isopulegol durch Kristallisation unter Erhalt von enantiomerenangereichertem L-(-)-Isopulegol und (entweder) racemischem oder enantiomerenabgereichertem Isopulegol und
d.5) katalytische Hydrierung von gemäß Schritt c.5) erhaltenem enantiomerenangreichertem L-(-)- Isopulegol zu L-(-)- Menthol und katalytische Hydrier-ung von gemäß Schritt c.5) erhaltenem racemischem oder enantiomeren-abgereichertem Isopulegol zu racemischem oder enantiomerenabgereichertem Menthol.

Das Verfahren gemäß dieser Ausführungsform erlaubt die Herstellung von enantiomerenangereichertem optisch aktivem L-Menthol und von racemischem oder enantiomerenabgereichertem Menthol, die nebeneinander als Verfahrensprodukte in hoher chemischer Reinheit erhalten werden. Als Ausgangsstoff dient wiederum Neral, bevorzugt solches, wie es nach dem optionalen zusätzlichen Verfahrensschritt 0) erhalten werden kann. Im Rahmen einer wiederum bevorzugten Ausführungsform umfasst das Verfahren zur Herstellung von L-(-)- Menthol und racemischem oder enantiomerenabgereichertem Menthol demnach auch den wie vorstehend beschriebenen Verfahrensschritt 0) betreffend die Herstellung von Neral in reiner oder angereicherter Form durch destillative Abtrennug von Neral aus Neral- und Geranial-haltigen Gemischen, bevorzugt unter Einsatz einer wie vorstehend beschriebenen Trennwandkolonne mit 80 bis 200 theoretischen Stufen bei einem absoluten Betriebsdruck von 5 bis 200 mbar.

Gemäß Schritt a.5) dieser Ausführugsform führt man eine wie vorstehend beschriebene asymmetrische katalytische Hydrierung von Neral zu D-(+)-Citronellal mit einem Enantiomerenüberschuss von 70 bis 99%, bevorzugt 80 bis 99% ee und besonders bevorzugt 85 bis 95 % ee durch. Das so gewonnene D-(+)-Citronellal wird anschließend gemäß Schritt b.5) zu L-(-)-Isopulegol in Gegenwart eines wie vorstehend beschriebenen sauren Katalysators, bevorzugt in Gegenwart eines Lewis-sauren, Aluminium-haltigen Katalysators cyclisiert.

Anschließend führt man zur Aufreinigung von L-(-)-Isopulegol gemäß Schritt c.5) die bereits beschriebene Kristallisation, vorzugsweise Schmelzekristallisation durch und erhält nebeneinander enantiomerenangereichertes L-(-)-Isopulegol und racemisches oder enantiomerenabgereichertes Isopulegol, d.h. Isopulegol mit einem Enantiomerenüberschuss von bis zu 15%, bevorzugt von bis zu 10% ee, besonders bevorzugt von bis zu 7% ee und ganz besonders bevorzugt von bis zu 5% ee.

Abschließend führt man gemäß Schritt d.5) dieser besonders bevorzugten Ausführungsform des erfindungsgemäßen Verfahrens eine katalytische Hydrierung von gemäß Verfahrensschritt c.5) gewonnenem enantiomerenangreichertem L-(-)-Isopulegol zu L-(-)-Menthol und eine katalytische Hydrierung von ebenfalls gemäß Verfahrensschritt c.5) gewonnenem racemischem oder enantiomerenabgereichertem Isopulegol zu racemischem oder enantiomerenabgereichertem Menthol wie vorstehend beschrieben durch.

Das so zugängliche racemische oder enantiomerenabgereicherte Menthol kann entsprechend dem Enantiomerenüberschuss des eingesetzten, wie vorstehend definierten racemischen oder enantiomerenabgereicherten Isopulegols ebenfalls einen Enantiomerenüberschuss von von bis zu 15%, bevorzugt von bis zu 10% ee, besonders bevorzugt von bis zu 7% ee und ganz besonders bevorzugt von bis zu 5% ee aufweisen.

Das im Rahmen dieser bevorzuten Ausführungsform des erfindungsgemäßen Verfahrens gewonnene racemische oder optisch aktive Menthol, speziell L-Menthol, kann anschließend gewünschtenfalls weiter aufgereingt werden, um die Reinheit der gewonnenen Verfahrensprodukte noch weiter zu steigern. Dementsprechend können die vorstehend beschriebenen Ausführungsformen jeweils auch als zusätzlichen Schritt den optionalen Verfahrenschritt e) umfassen, betreffend ein kontinuierliches Verfahren zur Herstellung von optisch aktivem oder racemischem Menthol in reiner oder angereicherter Form durch destillative Abtrennung von optisch aktivem oder racemischem Menthol aus Stoffgemischen enthaltend racemisches oder optisch aktives Menthol und Diastereomere des Menthols, wobei man die Abtrennung in einer Trennwandkolonne mit 50 bis 300 theoretischen Trennstufen und einer oder mehrerer Seitenabzugsstellen bei einem absoluten Betriebsdruck von 5 bis 500 mbar durchführt.

Das erfindungsgemäße Verfahren liefert in einer bevorzugten Ausführungsform L-(-)-Menthol mit einer chemischen Reinheit von mindestens 99 Gew.-%, bevorzugt von 99,5 bis 99,99 Gew.-%, insbesondere bevorzugt von 99,5 bis 99,9 Gew.-% und einem Enantiomerenüberschuss von mindestens 99%, bevorzugt von 99,5 bis 99,9 % ee.

Das durch das erfindungsgemäße Verfahren zugängliche racemische oder optisch aktive Menthol, bevorzugt L-Menthol kann in allen dem Fachmann geläufigen Formen, in der Regel in Form von teilweise oder ganz erstarrten Schmelzen oder in verfestigter Form, z.B. als Schuppen, Preßlinge, Pellets, Droplets und dergleichen mehr weiterverwertet bzw. vertreiben werden. Verfahren zur Verfestigung von Menthol sind dem Fachmann bekannt. So ist die einfache Schuppung auf einer Schuppenwalze beispielsweise in der US 3,023,253 beschrieben.

Die WO 2003/101924 offenbart Menthol Presslinge mit einem Gehalt an alpha-Menthol von mindestens 70 Gew.-% sowie ein Verfahren zur Herstellung derselben.

Ein Granulierungsverfahren zur Herstellung von spärischen Menthol-Partikeln ist beispielsweise aus der WO 2007/071512 bekannt. Dabei wird geschmolzenes Menthol direkt in Wasser mit einer Temperatur von 0 bis 12°C eingetragen.

Im Falle des wie vorstehend beschriebenen racemischen oder enenatiomerenabgreicherten Menthols hat sich die Schmelze aufgrund des geringeren Schmelzpunktes im Bereich von 28 bis 30°C insbesondere als gebräuchlichste Handhabungsform erwiesen.

Optisch aktives Menthol, bevorzugt wie vorstehend beschriebenes L-Menthol in reiner Form wird hingegen aufgrund der beseren Handhabungseigenschaften vorzugsweise in verfestigter Form weiterverwertet bzw. vertrieben.

Das erfindungsgemäße Verfahren eröffnet einen besonders wirtschaftlichen Zugang zu optisch aktivem und racemischem oder enantiomerenabgereichertem Menthol, wobei die dabei erhaltenen Produkte in niedriger Gesamtstufenzahl, hoher Ausbeute und hoher, den Qualitätsanforderungen vieler Pharmakopoen entsprechender Qualität zugänglich sind. Als Ausgangsstoff dienen dabei Geranial oder Neral oder bevorzugt Gemische von Geranial und Neral. Ein erfindungsgemäß besonders bevorzugter Ausgangsstoff ist das in technischem Maßstab gut verfügbare Citral, das seinerseits aus den Grundchemikalien Isobuten bzw. Isoprenol und Prenol in praktisch jedem Maßstab und unabhängig von natürlichen Quellen herstellbar ist.

Als besonderer Vorteil des erfindungsgemäßen Verfahrens ist hervorzuheben, dass es den Zugang zu optisch aktivem, bevorzugt praktisch enantiomeren- und diastereomerenreinem L-Menthol und zu racemischen oder enantiomerenabgereichertem Menthol eröffnet. Das Mengenverhältnis der erfindungsgemäß erhaltenen optisch aktiven bzw. racemischen bzw. oder enantiomerenabgereicherten Produkte kann dabei, gewünschtenfalls über die Zusammensetzung des eingesetzten Neral- und Geranial-haltigen Stoffgemisches gesteuert werden. So führt ein höherer Gehalt an Neral bei der asymmetrischen Hydrierung zu einem höheren Enantiomerenüberschuss des gebildeten optisch aktiven Citronellals, wodurch sich die Ausbeute an optisch aktivem Isopulegol bei der sich anschließenden Schmelzekristallisation, bei der optisch aktives von racemischem Isopulegol getrennt wird, erhöht.

Die folgenden Beispiele dienen der Erläuterung der Erfindung, ohne sie in irgendeiner Weise zu beschränken:

### Destillative Trennung von Neral- und Geranial-haltigen Gemischen

### Beispiel 1:

Die für die folgenden Beispiele verwendete Trennwandkolonne wurde aus fünf je 1,2 m langen Glasschüssen mit 64 mm Innendurchmesser aufgebaut. In die drei mittleren Schüsse wurde eine Trennwand aus Metallblech gesteckt. Unterhalb und oberhalb des Trennwandbereiches wurden Laborpackungen (Sulzer CY) und im Trennwandbereich Metall-Geweberinge aus Edelstahl mit 5 mm Durchmesser eingebaut. Bei Trennleistungsmessungen, die mit dem Xylol-Isomerengemischen bei einem Kopfdruck von 60 mbar durchgeführt wurden, konnte eine Gesamttrennleistung von 100 theoretischen Böden über die gesamte Kolonne und etwa 55 theoretischen Böden im Trennwandbereich gemessen werden. Die Gesamtzahl der vorhandenen theoretischen Böden betrug also in etwa 155. Die Kolonne wurde mit einem ölbeheizten Dünnschichtverdampfer (0,1 m²) und einem mit Kühlwasser gekühlten Kondensator ausgerüstet.

Temperaturen an verschiedenen Höhen in der Kolonne sowie der Kopfdruck und der Druckverlust über die Kolonne wurden mittels eines Messwerterfassungssystems gemessen. Die Kolonne verfügte über Durchflussmessungen in den Zu- und Abläufen sowie eine Mengenmessung des Rücklaufs, die als Regelgröße für die Vorlauftemperatur des Ölthermostaten diente. Durch diese Regelung wurde eine konstante Rücklaufmenge gewährleistet, wodurch sich auch ein konstanter Differenzdruck einstellte. Die Aufteilung der Flüssigkeitsmenge oberhalb der Trennwand auf Zulauf- und Entnahmeteil der Trennwand wurde über einen zeitlich getakteten Schwenktrichter realisiert.

Der Kolonne wurde in einer Höhe von 136 cm zum Zulaufteil der Trennwand 461 g/h eines auf 110°C vorgewärmten flüssigen Gemisches aus 48,7 GC-Flächen-% Neral, 47,8 GC-Flächen-% Geranial und 1,4 GC-Flächen-% andere Citral-Isomere zugeführt. Die Kolonne wurde bei 10 mbar Kopfdruck und einem Rücklauf von 2,5 kg/h betrieben. Dabei stellte sich ein Druckverlust von etwa 34 mbar (± 1 mbar) ein. Am Kopf der Kolonne wurde eine Temperatur von 82,3°C und im Sumpf eine Temperatur von 128,4 °C (± 0,5 K) gemessen. Der Sumpfabzug wurde über eine Waagensteuerung fest auf 240 g/h und die Destillatabnahme auf 20 g/h (± 1 g/h) eingestellt. Das Rücklaufverhältnis betrug somit etwa 125 : 1. Die Flüssigkeit wurde oberhalb der Trennwand in einem Verhältnis von 1 : 1,1 (Zulauf- : Entnahmeteil) aufgeteilt. In einer Höhe von 490 cm im Abnahmeteil der Trennwand wurde ein gasförmiger Seitenabzug (f) entnommen und in einem Glaskühler kondensiert, aus dem in Abhängigkeit vom Sumpffüllstand etwa 200 g/h Reinprodukt über eine Pumpe abgezogen wurde.

Die gewonnenen Fraktionen wurden gaschromatografisch mit Hilfe eines Standard-GC analysiert. Gaschromatographische Analysen wurden nach der folgenden Methode durchgeführt:
25m OV-1, ID.: 0,32 mm, FD.: 0,31 µm; 50 °C/2min - 10 °C/min auf 150 °C, 5 min - 20 °C/min auf 280 °C/15 min; t_{R} (Citral-Isomer III): 10,4 min; t_{R} (Citral-Isomer IV): 10,7 min; t_{R} (Citral-Isomer V): 11,0 min; t_{R} (Neral I): 12,3 min; t_{R} (Geranial II): 12,6 min

Das am Seitenabzug gewonnene Reinprodukt enthielt neben 98,5 GC-Flächen-% Neral auch 0,3 GC-Flächen-% Geranial und 0,65 GC-Flächen-% andere Citral-Isomere. Im Sumpfabzug wurden durch GC-Analyse 92,5 GC-Flächen-% Geranial und 6,8 GC-Flächen-% Neral bestimmt, das Destillat enthielt 32,1 GC-Flächen-% Neral und 39,6 GC-Flächen-% andere Citral-Isomere.

### Beispiel 2:

Die im Beispiel 1 beschriebene Kolonne wurde durch einen weiteren, gasförmigen Seitenabzug (n) im oberen gemeinsamen Kolonnenteil (1), bei etwa 590 cm Höhe, ergänzt, der wiederum mit einem Seitenkondensator versehen wurde. Über eine Waagensteuerung wurde dort eine Abnahmemenge von 15 g/h (± 1 g/h) eingestellt.

Der Kolonne wurde in einer Höhe von 136 cm zum Zulaufteil der Trennwand 460 g/h eines auf 110°C vorgewärmten flüssigen Gemisches aus 50,2 GC-Flächen-% Neral, 47,2 GC-Flächen-% Geranial und 0,9 GC-Flächen-% anderer Citral-Isomere zugeführt. Die Kolonne wurde bei 10 mbar Kopfdruck und einem Rücklauf von 2,5 kg/h betrieben. Dabei stellte sich ein Druckverlust von etwa 37 mbar (± 1 mbar) ein. Am Kopf der Kolonne wurde eine Temperatur von 68,8°C und im Sumpf eine Temperatur von 130,1 °C (± 0,5 K) gemessen. Der Sumpfabzug wurde über eine Waagensteuerung fest auf 240 g/h und die Destillatabnahme auf etwa 3 g/h (± 1 g/h) eingestellt. Das Rücklaufverhältnis betrug somit etwa 600 bis 1200 : 1. Die Flüssigkeit wurde oberhalb der Trennwand in einem Verhältnis von 1:1,1 (Zulauf- : Entnahmeteil) aufgeteilt. In einer Höhe von 490 cm im Abnahmeteil der Trennwand wurde wiederum ein gasförmiger Seitenabzug (f) entnommen und in einem Glaskühler kondensiert, aus dem in Abhängigkeit vom Sumpffüllstand etwa 200 g/h Reinprodukt über eine Pumpe abgezogen wurde.

Das am Seitenabzug (f) gewonnene Reinprodukt enthielt neben 98,5 GC.-Flächen-% Neral auch 0,3 GC-Flächen-% Geranial und 0,5 GC-Flächen-% andere Citralisomere. Der obere Seitenabzug enthielt neben 55,5 GC-Flächen % Neral 29,5 GC-Flächen-% andere Citral-Isomere. Im Sumpfabzug wurden durch GC-Analyse 90,3 GC-Flächen-% Geranial und 8,9 GC-Flächen-% Neral bestimmt, das Destillat enthielt nur Spuren von Neral und 48,5 GC-Flächen-% andere Citral-Isomere.

### Beispiel 3:

In einer mit 6 m Sulzer CY-Packung ausgerüsteten einfachen Glas-Laborkolonne ohne Trennwand und ohne Seitenabzug (theoretische Stufenzahl von etwa 90) mit einem Innendurchmesser von 50 mm wurde ein Gemisch aus 50,2 GC.-Flächen % Neral, 47 GC.-Flächen % Geranial und 1,3 GC.-Flächen % andere Citral-Isomere bei 5 mbar Kopfdruck kontinuierlich destilliert. Die Zulaufmenge betrug 500 g/h, am Sumpf wurden 250 g/h ausgetragen. Der Druckverlust über die Kolonne betrug bei einem Rücklaufverhältnis von 11 : 1 etwa 28 mbar, die Sumpftemperatur 121 °C und die Kopftemperatur 81 °C.

Am Kopfkondensator wurden bei etwa 20°C ca. 250 g/h eines flüssigen Destillats mit einem Neral-Gehalt von 88,1 GC.-Flächen % und einem Geranial-Gehalt von 2,7 GC.-Flächen % gewonnen, der Gehalt an anderen Citral-Isomeren im Destillat betrug in Summe 7,0 GC.-Flächen %, was auf eine merkliche Bildung dieser Isomere unter Destillationsbedingungen schließen lässt.

### Beispiele Verfahrensschritt a): Katalytische Hydrierung von Neral und/oder Geranial zu Citronellal

### Beispiel 4: Asymmetrische Hydrierung von cis-Citral in Gegenwart von Kohlenmonoxid

17,9 mg Rh(CO)₂acac und 38,5 mg (R,R)-Chiraphos wurden unter Schutzgasatmosphäre in 20 g Toluol gelöst und in einen 100 ml Autoklaven überführt, der zuvor 3 mal mit einem Gemisch aus Kohlenmonoxid und Wasserstoff (1:1, Vol./Vol.) gespült wurde. Es wurde bei einem Druck von 8 bar CO/H₂ 1:1 und 60°C für 3 h gerührt und anschließend auf Raumtemperatur abgekühlt. Mittels einer Druckschleuse wurden dann 10,94 g Neral (Verhältnis der Doppelbindungsisomere Neral/Geranial = 99,1:0,9; Verhältnis Substrat/Katalysator = 1000) mit 15 bar H₂ zugepresst. Der Reaktionsdruck wurde durch Zupressen von Wasserstoff auf 80 bar eingestellt. Zur Reduktion des CO-Partialdrucks wurde der Druck drei mal und nach 3 h ein weiteres mal auf 8 bar erniedrigt und mittels Wasserstoff auf 80 bar nachgepresst. Nach 18 h wurde gaschromatographisch ein Umsatz von 99,9% und eine Ausbeute an D-Citronellal von 99,8% mit einer optischen Reinheit von 90% ee bestimmt.

### Beispiel 5: Asymmetrische Hydrierung von Neral in Gegenwart von Kohlenmonoxid

17,0 mg Rh(CO)₂acac und 43,8 mg (R,R)-Chiraphos wurden in 0,8 ml THF gelöst und in einem Autoklaven bei 80 bar Synthesegas (H₂/CO = 1:1, Vol./Vol.) und 60°C für 8 h gerührt. Anschließend wurden 39,00 g Neral (Verhältnis der Doppelbindungsisomere Neral/Geranial = 95,2:4,8; Verhältnis Substrat/Katalysator = 4000) gelöst und zusammen mit der Katalysatorlösung in einen 100 ml Autoklaven gegeben, der zuvor drei mal mit CO/H₂ 1:1 (Vol./Vol.) gespült wurde. Der Reaktionsdruck wurde durch Zupressen von Wasserstoffgas, das 1000 ppm Kohlenmonoxid enthielt, auf 80 bar eingestellt. Nach 144 h wurde gaschromatographisch ein Umsatz von 84,3% und eine Ausbeute von 80,9% an D-Citronellal mit einer optischen Reinheit von 64% ee bestimmt.

### Beispiel 6: Asymmetrische Hydrierung von Neral unter Wiederverwendung des Katalysators

23,7 mg Rh(CO)₂acac und 55,7 mg (R,R)-Chiraphos wurden unter Schutzgasatmosphäre in 24 g THF gelöst und in einen 100 ml Autoklaven gegeben, der zuvor 3 mal mit CO/H₂ 1:1 (Vol./Vol.) gespült wurde. Es wurde bei einem Druck von 80 bar CO/H₂ 1:1 und 60°C 3 h gerührt. Anschließend wurde auf Raumtemperatur abgekühlt und auf einen Druck von 8 bar CO/H₂ 1:1 entspannt. Mittels einer Druckschleuse wurden 13,2 g) Neral (Verhältnis der Doppelbindungsisomere Neral/Geranial = 99,4:0,6) mit 15 bar H₂ zugepresst. Der Reaktionsdruck wurde durch Zupressen von Wasserstoff auf 80 bar eingestellt. Zur Reduktion des CO-Partialdrucks wurde der Druck 5 mal auf 8 bar erniedrigt und mit Wasserstoff auf 80 bar nachgepresst. Der gaschromatographisch bestimmte Gehalt an CO im Gasraum betrug 510 ppm. Nach jeweils 20 h und 40 h werden weitere 13,20 g bzw. 19,80 g Neral zugegeben. Nach 66 h wurde gaschromatographisch ein Umsatz von 75,8% und eine Ausbeute von 72,8% D-Citronellal mit einer optischen Reinheit von 87% ee bestimmt.

Die Gesamt-turn-over-number bezogen auf die Ausbeute an D-Citronellal betrug 1030.

### Beispiel 7: Asymmetrische Hydrierung von cis-Citral unter Abdestillieren des Produkts und Wiederverwendung des Katalysators

8,4 mg Rh(CO)₂acac und 21,6 mg (R,R)-Chiraphos wurden in 0,8 ml THF gelöst und in einem Autoklaven bei 80 bar Synthesegas (H₂/CO = 1:1, Vol./Vol.) und 60°C für 8 h gerührt. Danach wurden 9 g Neral (Verhältnis der Doppelbindungsisomere Neral/Geranial = 95,2:4,8) in den Autoklaven gefüllt. Der Reaktionsdruck wurde durch Zupressen von Wasserstoffgas, das 1000 ppm Kohlenmonoxid enthielt, auf 80 bar eingestellt. Nach 24 h war ein Umsatz von 99% erreicht, der ee des erhaltenen D-Citronellals betrug 83%.

Nach Abdestillieren des Produktes werden weitere 8,5g Neral (Verhältnis der Doppelbindungsisomere Neral/Geranial = 95,2:4,8) zugegeben und 48 h bei 80 bar Wasserstoffgas, das 1000 ppm Kohlenmonoxid enthielt, hydriert. Der Umsatz betrugt 36%, der ee des erhaltenen D-Citronellals betrug 54%.

Nach abermaligem Abdestillieren des Produktes werden weitere 6,8 g Neral (Verhältnis der Doppelbindungsisomere Neral/Geranial = 95,2:4,8) zugegeben und 72 h bei 80 bar Wasserstoffgas, das 1000 ppm Kohlenmonoxid enthielt, hydriert. Der Umsatz betrug 13%, der ee des erhaltenen D-Citronellals betrug 30%.

Die Gesamt-turn-over-number bezogen auf die Ausbeute an D-Citronellal betrug 2312.

### Beispiel 8: Asymmetrische Hydrierung von Neral unter Präformierung, Entfernung des Produkts und Wiederverwendung des Katalysators

30 mg Rh(CO)₂acac und 75 mg (R,R)-Chiraphos wurden in 3 ml THF gelöst und in einem Autoklaven bei 60°C in Gegenwart von 80 bar Synthesegas (H₂/CO = 1:1, Vol./Vol) für 20 h gerührt. Anschließend wurden 37 g Neral (Verhältnis der Doppelbindungsisomere Neral/Geranial = 96,6:3,4) zugegeben und die Lösung in einen 100 ml Autoklaven gegeben, der zuvor drei mal mit CO/H₂ 1:1 (Vol./Vol.) gespült wurde. Der Reaktionsdruck wurde durch Zupressen von Wasserstoffgas, das 1000 ppm Kohlenmonoxid enthält, auf 80 bar eingestellt. Nach 24 h war ein Umsatz von >99% erreicht; der ee des erhaltenen D-Citronellals betrug 87%.

Nach Abdestillieren des Produktes wurde der Destillationsrückstand mit THF verdünnt und in einem Autoklaven bei 60°C in Gegenwart von Synthesegas (H₂/CO = 1:1) bei einem Druck von 80 bar für 20 h gerührt. Danach wurden weitere 32 g Neral (Verhältnis der Doppelbindungsisomere Neral/Geranial = 96,6:3,4) zugegeben und 24 h bei einem Druck von 80 bar Wasserstoffgas, das 1000 ppm Kohlenmonoxid enthielt, hydriert. Der Umsatz betrug >99%, der ee des erhaltenen D-Citronellals betrug 87%.

Nach abermaligem Abdestillieren des Produktes wurde der Destillationsrückstand mit THF verdünnt und in einem Autoklaven bei 60°C in Gegenwart von 80 bar Synthesegas (H₂/CO = 1:1) für 20 h gerührt. Danach wurden weitere 32,96 g Neral (Verhältnis der Doppelbindungsisomere Neral/Geranial = 96,6:3,4) zugegeben und 24 h bei einem Druck von 80 bar Wasserstoffgas, das 1000 ppm Kohlenmonoxid enthielt, hydriert. Der Umsatz betrug 90%, die optische Reinheit des erhaltenen D-Citronellals betrug 88% ee.

Das Experiment wurde nochmals wiederholt unter Zugabe von 33 g Neral (Verhältnis der Doppelbindungsisomere Neral/Geranial = 96,6:3,4). Man erhielt bei einem Umsatz von 17% D-Citronellal mit einer optischen Reinheit von 89% ee.

Die Gesamt-turn-over-number bezogen auf die Gesamtausbeute an D-Citronellal betrug 4975.

### Beispiel 9: Kontinuierlich betriebene asymmetrische Hydrierung von Neral

In einer kontinuierlich betriebenen Laboranlage wurden eine Lösung von 2,13 g Rh(CO)₂acac und 6,00 g (R,R)-Chiraphos in 70 g THF und 60 g Oxoöl 9N (BASF Aktitengesellschaft), die zuvor 20 h bei 60°C und einem Druck von 80 bar CO/H₂ 1:1 (Vol./Vol.) gerührt worden war und 170 g Neral (Verhältnis der Doppelbindungsisomere Neral/Geranial ca. 95:5) eingefüllt, woraufhin die Gasmischung im Präformierreaktor der Anlage auf 10000 ppm Kohlenmonoxid in Wasserstoff (80 bar), die Temperatur auf 60°C eingestellt wurde. Im Hydrierreaktor wurde eine Gasmischung von 1000 ppm Kohlenmonoxid in Wasserstoff (80 bar) und eine Temperatur von 25°C eingestellt.

Der Zulauf von frischem Edukt wurde auf 6 g/h eingestellt. Eine produkthaltige Fraktion wurde im Vakuum kontinuierlich so abdestilliert, dass der Anlageninhalt nahezu konstant blieb. Es wurden innerhalb von 19 Tagen 6,01 mol (927,7 g) D-Citronellal erhalten. Die Gesamt-turn-over-number bezogen auf die Ausbeute an D-Citronellal betrug 10914.

### Beispiel 10: Asymmetrische Hydrierung von Neral

12,3 mg Rh₄(CO)₁₂ und 31,5 mg (S,S)-Chiraphos wurden unter Schutzgasatmosphäre in 15 g Toluol gelöst und in einen 100 ml Autoklaven überführt, der zuvor 3 mal mit H₂ gespült worden war. Es wurde bei 1,5 bar H₂ für 1,5 h gerührt, auf Normaldruck entspannt und 1 g Neral (Verhältnis der Doppelbindungsisomere Neral/Geranial = 98,7:1,3; Verhältnis Substrat/Katalysator = 100) gelöst in 15 g Toluol mittels einer Spritze zugegeben. Der Reaktionsdruck wurde durch Aufpressen von Wasserstoff auf 90 bar eingestellt. Gaschromatographische Reaktionskontrolle zeigte nach 15 h vollständigen Umsatz und eine gaschromatographisch bestimmte Ausbeute von 98% L-Citronellal mit einer optischen Reinheit von 86% ee.

### Beispiele Verfahrenschritt b): Cyclisierung von Citronellal zu Isopulegol in Gegenwart eines sauren Katalysators

### Beispiel 11: Cyclisierung von Citronellal zu Isopulegol unter Rückgewinnung von 1,1-Bis(2,6-diphenylphenol)-1-trifluormethylethan (Ia₂-3)

Gaschromatographische Analysen (GC) wurden nach folgender Methode durchgeführt: 50 m CP-WAX, ID.: 0,32 mm, FD.: 1,2 µm; 80 °C, 3 °C/min -200 °C, 15 °C/min auf 250 °C; t_{R} (Phenylcyclohexan): 30,7; t_{R} (Isopulegol): 26,3; t_{R} (Citronellal): 21,8.

Folgende HPLC-Methode wurde verwendet: CC250/4 Nucleodur C18 Gravity, 5 µm; C: Wasser - 0,05 % H₃PO₄; D: Acetonitril 20 : 80; Ausgang: 93 bar, 25°C; t_{R} (Isopulegol): 3,3; t_{R} (Phenylcyclohexan): 10,5; t_{R} (Ligand (Ia₂-3)): 14,0.
Konzentrationen der erhaltenen Reaktionsprodukte im Destillationssumpf und in der Mutterlauge (jeweils in Gew.-%) wurden GC- analytisch mittels internem Standard ermittelt.

### 11.a) Cyclisierung von Citronellal

In einem Doppelmantelglasreaktor mit Rührer wurde 1,1-Bis(2,6-diphenylphenol)-1-trifluormethylethan (Ia₂-3) (461 g, 0,785 mol) in wasserfreiem Toluol (7,2 kg) vorgelegt. Zu der klaren Lösung des Liganden wurde bei Raumtemperatur eine Lösung von Triethylaluminium in Toluol (445 ml, 400 mmol, 12% AlEt₃ in Toluol) gegeben. Die Lösung wurde für 1 h bei 25°C gerührt. Die resultierende Katalysator-Suspension wurde auf 0°C gekühlt und über einen Zeitraum von 3 h mit einem Gemisch aus Citronellal (6697 g, 43 mol) und Brenztraubensäuremethylester (33,6 g, 329 mmol) versetzt. Nach beendeter Zugabe wurde die Reaktionsmischung 3 h bei 0°C und weitere 2 h bei 10°C nachgerührt. Toluol wurde unter vermindertem Druck abgetrennt. Anschließend wurde ein Isopulegol-Rohprodukt destillativ als Kopfprodukt unter Zugabe von Phenylcyclohexan (2770 g) abgetrennt. Dabei wurden 3584 g eines Sumpfprodukts erhalten.

### 11.b) Isolierung des Liganden (Ia₂-3)

In einem Doppelmantelreaktor mit Rührer und Rückflusskühler wurden 3564 g des Sumpfproduktes aus der Cyclisierung von Citronellal in Gegenwart eines Bis(diarylphenoxy)-Aluminium-Katalysators, enthaltend Phenylcyclohexan (69,9 Gew.-%), Isopulegol (3,05 Gew.-%) Citronellal (0,16 Gew.-%) und Citronellol (3,05 Gew.-%), bei einer Temperatur von 90°C vorgelegt. Zu der erwärmten Lösung wurden 1792 g einer erwärmten 2%igen NaOH-Lösung gegeben. Nach einstündigem Rühren bei 90°C wurden 1777 g der wässrigen Phase von der organischen Phase abgetrennt. Das restliche Wasser aus der organischen Phase wurde bei 120°C und 10 mbar abdestilliert. Das hydrolysierte Sumpfprodukt wurde innerhalb von 12 Stunden auf 25°C abgekühlt. Die erhaltene Suspension des Liganden der Formel (Ia₂-3) wurde filtriert und der so gewonnene Ligand der Formel (Ia₂-3) wurde bei 3 mbar und 95°C von volatilen Bestandteilen befreit. Der Ligand der Formel (Ia₂-3) wurde als weißer Feststoff mit einer Ausbeute von 282 g und einer Reinheit von 95% isoliert. Die Mutterlauge (3130 g) enthielt laut HPLC-Analytik Phenylcyclohexan (72,3 Gew.-%), Isopulegol (6,8 Gew.-%) und Ligand der Formel (Ia₂-3) (4,9 Gew.-%). Dies belegt, dass sich die erfindungsgemäß verwendeten Liganden in vorteilhafter Weise für eine kontinuierliche Aufarbeitung eignen. Mit den in der EP-A 1 225 163 beschriebenen Liganden hingegen ist eine Trennung der Phasen nicht in jedem Fall gewährleistet, da diese in stärkerem Maße zur Bildung stabiler Emulsionen neigen.

### Beispiel 12: Cyclisierung von Citronellal zu Isopulegol unter kontinuierlicher Rückgewinnung von 1,1-Bis(2,6-diphenylphenol)-1-trifluormethylethan (Ia₂-3)

### Analytik

Gaschromatographische Analysen wurden nach der folgenden Methode durchgeführt: 50 m CP-WAX, ID.: 0,32 mm, FD.: 1,2 µm; 80 °C, 3 °C/min 200 °C, 15 °C/min auf 250 °C; t_{R} (Citronellal): 20,7; t_{R} (Isopulegol): 24,7; t_{R} (Phenylcyclohexan): 29,3; t_{R} (Citronellol): 31,7; t_{R} (Citronellsäurecitronellylester): 48,2; t_{R} (Citronellsäureisopulegylester): 49,5.

### 12.a) Cyclisierung von Citronellal bei kontinuierlicher Aufarbeitung

In einem Doppelmantelglasreaktor mit Rührer wurde zu einer klaren Lösung von 1,1-Bis(2,6-diphenylphenol)-1-trifluormethylethan (Ia₂-3) (114 g, 0,195 mol) in Toluol (wasserfrei, 1800 g) bei 20 °C eine Lösung von Triethylaluminium in Toluol (15%ig, 85 ml, 0,096 mol) innerhalb von etwa 10 min zugegeben. Anschließend wurde die Lösung 1 h bei 20°C gerührt. Die resultierende Katalysator-Suspension wurde in einen weiteren Doppelmantelglasreaktor mit Rührer überführt, auf 0°C abgekühlt und über einen Zeitraum von 3 h mit einem Gemisch aus D-Citronellal (1620 g, 10,3 mol) und Brenztraubensäuremethylester (8,1 g) versetzt. Nach beendeter Zugabe wurde die Reaktionslösung bei 0°C gerührt bis ein Gehalt von < 10 GC-FI.% an D-Citronellal erreicht war, auf 10°C erwärmt und weitere 2 h bei dieser Temperatur gerührt. Anschließend wurde zunächst die Reaktionslösung in einen Pufferbehälter überführt.

Die Reaktionslösung wurde einer Bodenkolonne (15 Böden, DN 50) kontinuierlich mit einer Zulaufgeschwindigkeit von 300 g/h zugeführt. Toluol wurde der Kolonne bei einem Kopfdruck von etwa 100 mbar an einem Seitenabzug auf dem 10. Boden im Verstärkungsteil entnommen, wobei die Sumpftemperatur etwa 120°C und die Temperatur des Seitenabzugs und des Kolonnenkopfes 45°C betragen. Am Kopf dieser Kolonne wurden die Leichtsieder der Reaktionslösung ausgeschleust.

Ein Austrag des Sumpfproduktes der Bodenkolonne wurde kontinuierlich (120 bis 140 g/h) einer Packungskolonne (DN 50 x 120 cm, Labor-Gewebe-Packung, Sulzer DX) mittig zugeführt. Unter kontinuierlicher Zugabe von Phenylcyclohexan (70 bis 90 g/h) in den Sumpf dieser Packungskolonne wurde bei einer Sumpftemperatur von 110°C und einem Kopfdruck von 10 mbar L-Isopulegol als Kopfprodukt abdestilliert. L-Isopulegol wurde in einer Ausbeute von 1625 g und in einer Reinheit von 93% isoliert.

### 12.b) Isolierung des Liganden (Ia₂-3) bei kontinuierlicher Fahrweise

Ein Austrag des Destillationssumpfes der Packungskolonne wurde kontinuierlich (100 bis 120 g/h) einer auf 95°C temperierten Mixer-Settler-Anlage bestehend aus zwei kaskadierten 250 ml-Rührbehältern und einem 150 ml-Phasenscheider zugeführt. Im ersten 250 ml-Rührbehälter wurde der Austrag des Destillationssumpfes der Packungskolonne kontinuierlich mit einem Zulauf 2%iger Natriumhydroxid-Lösung (50 bis 60 g/h) versetzt. Ein Austrag (150 bis 180 g/h) der Mischphase aus dem ersten Rührbehälter wurde über den weiteren 250 ml-Rührbehälter in den 150 ml-Phasenscheider überführt. Im Phasenscheider erfolgte die kontinuierliche Trennung der Phasen bei einer Temperatur von 90 bis 95°C. Die Höhe der Phasentrennschicht wurde dabei mit Hilfe von Leitfähigkeitsmessungen geregelt.

Der Austrag der organischen Phase aus dem Phasenscheider wurde kontinuierlich (100 bis 120 g/h) in einem weiteren auf 40 bis 50°C temperierten Rührbehälter aufgefangen und vor der Isolierung des Liganden (Ia₂-3) der Kristallisation überlassen. Ein Austrag der wässrigen Phase aus dem Phasenscheider wurde kontinuierlich abgepumpt.

Der auskristallisierte Ligand (Ia₂-3) wurde batchweise über einen Druckfilter bei einem Stickstoffdruck von 4 bar filtriert. Anschließend wurde der Filterkuchen mit Phenylcyclohexan gewaschen. Der gewaschene Ligand (106 g; HPLC-Gew.-%: Ligand 77%; Phenylcyclohexan 22%) wurde in Toluol gelöst und zur Katalysatorherstellung in Schritt 2.a) wiederverwendet. Das Filtrat (919 g; Gew.-% laut GC: Phenylcyclohexan 66%; L-Isopulegol 5%; Citronellol 6,1%; Citronellsäureisopulegylester 4,3%; Citronellsäurecitronellylester 3,6%; Gew.-% laut HPLC: Ligand 3,1 %) wurde in die unter 11.a) beschriebene Packungskolonne zurückgeführt.

### Beispiel 13: Kontinuierliche Aufreinigung von Isopulegol in einer Trennwandkolonne

Eine Labor-Trennwandkolonne wurde aus drei Glasschüssen mit 43 mm Innendurchmesser aufgebaut. Der mittlere Kolonnenschuss mit einer Gesamtlänge von 105 cm wurde mit einer fest eingeschmolzenen Trennwand aus etwa 1 mm starkem Glas versehen. Im Bereich der Trennwand ist die Kolonne mit 1 m Sulzer DX-Packung auf der Zulaufseite und 0,9 m DX-Packung auf der Abnahmeseite ausgerüstet. Oberhalb und unterhalb der Trennwand wurden 50 mm lange Glasschüsse eingesetzt, die je mit 33 cm Sulzer DX-Packungen ausgerüstet wurden.

Bei Trennleistungsmessungen, die mit dem Xylol-Isomerengemischen bei einem Kopfdruck von 60 mbar durchgeführt wurden, konnte eine Gesamttrennleistung von etwa 32 theoretischen Stufen über die gesamte Kolonne und etwa 18 theoretischen Stufen im Trennwandbereich gemessen werden. Die Gesamtzahl der vorhandenen theoretischen Stufen betrug also etwa 50. Die Kolonne wurde mit einem ölbeheizten Dünnschichtverdampfer (0,1 m²) und mit einem auf 10°C temperierbaren Kühlwasser gekühlten Kondensator ausgerüstet. Zu- und Ablauf befanden sich jeweils mittig im Trennwandteil.

Die Temperaturen an verschiedenen Höhen in der Kolonne sowie der Kopfdruck und der Druckverlust über die Kolonne wurden mittels eines Messwerterfassungssystems gemessen. Die Kolonne verfügte über Durchflussmessungen in den Zu- und Abläufen sowie eine Mengenmessung mit Regelung des Rücklaufs. Durch diese Regelung wurde eine konstante Rücklaufmenge gewährleistet, wodurch sich auch ein konstanter Differenzdruck einstellte. Die Aufteilung der Flüssigkeitsmenge oberhalb der Trennwand auf Zulauf- und Entnahmeteil der Trennwand wurde über einen zeitlich getakteten Schwenktrichter realisiert.

Der Trennwandkolonne wurde in der Kolonnenmitte zum Zulaufteil der Trennwand kontinuierlich 250 g/h eines auf 80°C vorgewärmten Isopulegolgemisches , das durch Zyklisierung von Citronellal, wie im Beispiel 12 beschriebenen erhalten wurde, zugeführt. Das Isopulegolgemisch enthielt neben 93 Gew.-% Isopulegol, 3,6 Gew.-% Phenylcyclohexan, 0,3 Gew.-% Citronellal, 0,1 Gew.-% Citronellol und 1,3 GC-Flächen % Toluol.

Die gewonnenen Fraktionen wurden gaschromatographisch mit Hilfe eines Standard-GC analysiert. Als interner Standard für die Gew.-% Bestimmung wurde 1-Nonanol verwendet (Einwage ca. 10% der Gesamtprobenmenge).

Gaschromatographische Analysen wurden nach der folgenden Methode durchgeführt: 50 m CP-Wax 52 CB , ID.: 0,32 mm, FD.: 1,2 µm; Injektor: 200°C; Detektor: 250 °C; 80 °C, 3 °C/min - 200 °C, 15 °C/min auf 250 °C; t_{R} (Citronellal): 20,7; t_{R} (Isopulegol): 24,7; t_{R} (Phenylcyclohexan): 29,3; t_{R} (Citronellol): 31,7.

Die Kolonne wurde bei 100 mbar Kopfdruck und einem Rücklauf von 1000 g/h betrieben. Dabei stellte sich ein Druckverlust von etwa 1 mbar ein. Am Kopf der Kolonne wurde eine Temperatur von 109°C und im Sumpf eine Temperatur von 139 °C (± 0,5 K) gemessen. Die Kolonne wurde mit 16 g/h (± 2 g/h) Sumpfabzug betrieben und die Destillatabnahme über eine Waagensteuerung auf 4 g/h (± 1 g/h) eingestellt. Das Rücklaufverhältnis betrug somit etwa 200:1. Der Kondensator der Kolonne wurde auf 10°C temperiert.

Die Flüssigkeit wurde oberhalb der Trennwand in einem Verhältnis von 1 : 2,4 (Zulauf-: Entnahmeteil) aufgeteilt. In der Mitte des Abnahmeteil der Trennwand wurde mit Hilfe einer Membranpumpe ein flüssiger Seitenabzug (f) von etwa 230 g/h (± 5 g/h) entnommen

Das am Seitenabzug gewonnene Reinprodukt enthielt neben 98,6 Gew.-% Isopulegol auch 0,3 Gew.-% Phenylcyclohexan. Die Destillationsausbeute an Isopulegol lag damit bei 97,5%. Das Destillat enthielt neben 50 GC-FI.-% Toluol, 47 GC-Gew.-% Isopulegol und 1,0 GC-Gew.-% Citronellal. Im Sumpf wurden neben 55 Gew-% Phenycyclohexan noch 39,6 Gew.-% Isopulegol und 1% Citronellol analysiert.

### Beispiele Schritt c): Aufreinigung von Isopulegol durch Kristallisation

### Beispiel 14: Statische Schichtkristallisation einer Isopulegol-Schmelze

In einem doppelwandigen Glasrohr als Kristallisator wurden 205 g Isopulegol der Zusammensetzung 95% (-)-n-Isopulegol und 5% (+)-n-Isopulegol (90% ee) mit einem Schmelzpunkt von 13°C bei einer Temperatur 15°C vorgelegt. Der Kristallisator wurde innerhalb von 30 h bis auf 9°C abgekühlt. Das anfangs flüssige Produkt lag am Versuchsende zum großen Teil erstarrt vor. Anschließend wurde die Manteltemperatur innerhalb von 10 h von 13°C auf °C 25°C angehoben. Dabei wurden neben 70 g Mutterlauge und 50 g Schwitzfraktionen 85 g an abgeschmolzener Kristallschicht erhalten. Dieses Endprodukt wies eine optische Reinheit von 99,9% ee bezogen auf (-)-n-Isopulegol auf.

### Beispiel 15: Dynamische Schichtkristallisation einer Isopulegol-Schmelze

In einem Rührapparat mit planem, über einen Mantel gekühltem Boden (wie in G.F. Arkenbout, Melt Crystallization Technology, Lancater/PA, Technomic Publ. Co., 1995 (Kap. 10.4.1) beschrieben) wurden 1003 g Isopulegol der Zusammensetzung 94,7% (-)-n-Isopulegol und 5,3% (+)-n-Isopulegol (89,4 % ee) mit einem Schmelzpunkt von 10°C bei einer Temperatur von 12°C vorgelegt. Der Kühlmantel des Kristallisatorbodens wurde im Laufe von 2 h auf -14°C abgekühlt. Dabei bildete sich eine 12 mm dicke Kristallschicht mit einem Gewicht von 124 g. Anschließend wurde der Apparat um 180° gedreht und die Manteltemperatur innerhalb von 10 h von 8°C auf 13°C angehoben. Dabei wurden 52 g Schwitzfraktionen und 124 g abgeschmolzene Kristallschicht erhalten. Dieses Endprodukt wies eine optische Reinheit von 99% bezogen auf (-)-n-Isopulegol auf.

### Beispiel 16: Suspensionskristallisation einer Isopulegol-Schmelze

In einem 11-Rührwerkskristallisator (wie bei Arkenbout, Kap. 10.4.2 beschrieben) wurden 860 g eines Isopulegol-Isomerengemischs mit einer optischen Reinheit bezüglich (-)-n-Isopulegol: 95,2% (90,4 % ee) als Schmelze vorgelegt. Die Schmelztemperatur der Mischung betrug ca. 10°C. Als Rührer diente ein wandgängiger Wendelrührer. Eine in-situ Impfung der Schmelze wurde durch kurzzeitiges Unterkühlen bis auf 3°C und anschließendes Aufheizen auf 9°C erreicht. Anschließend wurde unter Rühren innerhalb von 1,5 h auf 7°C abgekühlt. Hierdurch stellte sich ein Feststoffgehalt der Suspension von ca. 35 Gew.-% ein. Aus dieser Suspension wurde eine Probe gezogen und durch Abzentrifugieren von anhaftender Mutterlösung befreit. Nach einminütigem Zentrifugieren wiesen die Kristalle eine Reinheit von 99% ee bezogen auf (-)-n-Isopulegol, nach fünf Minuten Zentrifugieren von 99,4% auf.

### Vergleichsbeispiel 1: Lösungskristallisation von Menthol

In einem 1I-Rührwerkskristallisator wurden 560 g eines Menthol-Isomerengemischs (80% ee, Reinheit bezüglich (-)-Menthol: 90%) in 240 g Aceton gelöst. Die Sättigungstemperatur der Mischung betrug 5,8°C. Nach Abkühlen auf 5,7°C wurde die übersättigte Lösung mit 14 g Impfkristallen reinen (-)-Menthols angeimpft und mit einer Rate von 0,5 bis 1 K/h weiter abgekühlt. Bei Erreichen einer Temperatur von -6,9°C und einem Feststoffgehalt von 22,4 Gew.-% in der Suspension wurde eine Probe gezogen und durch Abzentrifugieren von anhaftender Mutterlösung befreit. Die Kristalle wiesen eine Reinheit von 98,2% (96,4% ee) auf.

### Vergleichsbeispiel 2: Schmelzekristallisation von Menthol

In einem doppelwandigen Glasrohr als Kristallisator wurden 324 g Menthol der Zusammensetzung 95% (-)-Menthol und 5% (+)-Menthol (90% ee) vorgelegt. Der Schmelzpunkt der Mischung lag bei 38°C. Der Kristallisator wurde im Laufe von 15 h von 38,4°C auf 37,4°C abgekühlt. Das anfangs flüssige Produkt lag am Versuchsende fast vollständig erstarrt vor. Anschließend wurde die Manteltemperatur innerhalb von 5 h von 38°C auf 39°C angehoben. Dabei wurden zwei Schwitzfraktionen (51 g und 198 g) sowie 75 g abgeschmolzene Kristallschicht erhalten. Eine Analyse zeigte, dass Ausgangslösung, beide Schwitzfraktionen und die Kristallschicht praktisch identische ee-Werte um 90% aufwiesen.

### Beispiele Schritt d): Katalytische Hydrierung von Isopulegol zu Menthol

Gaschromatographische Analysen wurden nach der folgenden Methode durchgeführt: 50 m CP-WAX, ID.: 0,32 mm, FD.: 1,2 µm; 80°C, 3°C/min - 200°C, 10°C/min auf 230°C; t_{R} (Menthon): 26,9; t_{R} (Menthon): 28,1; t_{R} (Isopulegol): 30,7; t_{R} (Neomenthol): 31,2; t_{R} (Neoisomenthol): 32,6; t_{R} (Menthol): 32,7; t_{R} (Isomenthol): 34,1.

Das eingesetzte Isopulegol wurde gaschromatographisch folgendermaßen analysiert: 50 m CP-WAX, ID.: 0,32 mm, FD.: 1,2 µm; 80°C, 3°C/min - 200°C, 15°C/min auf 250°C; t_{R} (Citronellal): 21,6; t_{R} (Isopulegol-Isomer): 25,4; t_{R} (Isopulegol): 25,9;
t_{R} (Citronellol): 32,7.

### Beispiel 17:

Es wurde eine Hydrierapparatur bestehend aus einem Hauptreaktor (HR) und einem Nachreaktor (NR) eingesetzt. Der Hauptreaktor wies 5 in Reihe geschaltete Röhren mit einem Innendurchmesser von 5 mm und einer Länge von 1,3 m auf, die mit 61 g (127 ml) eines Festbettkatalysators enthaltend 50 Gew.-% NiO, 17 Gew.-% CuO, 30,5 Gew.-% ZrO₂, 1,5 Gew.-% MoO₃ und 1 Gew.-% Graphit in Form von Tabletten mit einem Durchmesser und einer Höhe von jeweils von 3 mm gefüllt waren. Der Nachreaktor (Doppelmantel) bestand aus einem Rohr mit einem Innendurchmesser von 5 mm und einer Länge von 2,05 m, der mit 19 g des gleichen Katalysators gefüllt war.

Der im Hauptreaktor und Nachreaktor eingebaute Festbettkatalysator enthaltend 50 Gew.-% NiO, 17 Gew.-% CuO, 30,5 Gew.-% ZrO₂, 1,5 Gew.-% MoO₃ und 1 Gew.-% Graphit wurde nach folgender Vorschrift aktiviert. Die Reaktoren wurden mit 42 NI/h Stickstoff und 1,2 NI/h Wasserstoff drucklos auf 180 °C geheizt und 19 h bei diesen Bedingungen gehalten. Der Wasserstoff wurde von 1,2 auf 6,5 NI/h erhöht und weitere 7,5 h bei einer Temperatur von 180 °C gehalten. Der Stickstoff-Zulauf wurde abgedreht und mit 6,5 NI/h Wasserstoff 12 h bei 180 °C die Aktivierung fortgesetzt. Anschließend wurde der Wasserstoff-Zulauf abgedreht und der Stickstoff-Zulauf auf 6 NI/h eingestellt. Die Reaktoren wurden auf eine Temperatur von 60 °C abgekühlt. Der Wasserstoff-Zulauf wurde auf 1,6 NI/h reduziert und mit dem Isopulegol-Zulauf gestartet.

Mittels einer Kreiselpumpe wurde ein Umwälzkreislauf über den Hauptreaktor mit einer Rate von etwa 500 g/h bei einem Zulauf an L-Isopulegol von 24,5 g/h (Gesamtmenge 588 g) mit einer Reinheit von 99,9 Gew-% und 99,8% ee gepumpt. Der Wasserstoffdruck wurde konstant bei 40 bar gehalten. Der Hauptreaktor wurde mit einer Temperatur von 85°C und der Nachreaktor mit 75°C betrieben. Sämtliche Rohrleitungen wurden zur Vermeidung der Auskristallisation des enantiomerenreinen L-Menthols (Smp. 44°C) mit einer elektrischen Begleitheizung versehen. Man erhielt L-Menthol in einer Menge von 597 g entsprechend einer Rate von 24,9 g/h. Das so erhaltene L-Menthol (99,8% ee) wurde gaschromatographisch analysiert. Die chemische Reinheit des Austrags L-Menthol ist in Tabelle 1 zusammengestellt.

**Tabelle 1: GC-Analytik des Austrags L-Menthol (GC-Fl.-%)**

| Menthon / Isomenthon | L-Menthol | Neomenthol | Neoisomenthol | Isomenthol | L-Isopulegol |
|---|---|---|---|---|---|
| 0 | 99,6 | 0,19 | 0 | 0 | 0,19 |

### Beispiel 18:

Beispiel 17 wurde unter Einsatz von L-Isopulegol mit einer Reinheit von 99,9 Gew.-% und 99,8% ee, das mit einer Rate von 12,6 g/h (Gesamtmenge 303 g) in den Reaktor eingetragen wurde, bei einem Wasserstoffdruck von 40 bar wiederholt. Der Hauptreaktor wurde auf 80°C, der Nachreaktor auf 75°C temperiert. Man erhielt L-Menthol (99,8% ee) in einer Menge von 306 g entsprechend einer Rate von 12,8 g/h. Die chemische Reinheit des Austrags L-Menthol ist in Tabelle 2 zusammengestellt.

**Tabelle 2: GC-Analytik des Austrags L-Menthol (GC-FI.-%)**

| Menthon / Isomenthon | L-Menthol | Neomenthol | Neoisomenthol | Isomenthol | L-Isopulegol |
|---|---|---|---|---|---|
| 0 | 99,7 | 0,25 | 0 | 0 | 0 |

### Beispiel 19:

Beispiel 17 wurde unter Einsatz von L-Isopulegol mit einer Reinheit von 97,1 % und 84% ee, das mit einer Rate von 24,5 g/h (Gesamtmenge 466 g) in den Reaktor eingetragen wurde bei einem Wasserstoffdruck von 40 bar wiederholt. Der Hauptreaktor wurde auf 80°C, der Nachreaktor auf 70°C temperiert. Das eingesetzte L-Isopulegol wies die folgende Zusammensetzung auf: L-Isopulegol: 97,1 GC-Gew.-%, Citronellol: 0,05 GC-Gew.-%, Citronellal: 0,40 GC-Gew.-%, Isopulegol-Isomer 0,45 GC-Gew-%, Nebenkomponente: 0,34 GC-Gew.-%. Man erhielt L-Menthol (84% ee) in einer Menge von 468 g entsprechend einer Rate von 24,6 g/h. Die chemische Reinheit des Austrags L-Menthol ist in Tabelle 3 dargestellt.

**Tabelle 3: GC-Analytik des Austrags L-Menthol (GC-FI.-%)**

| Menthon / Isomenthon | L-Menthol | Neomenthol | Neoisomenthol | Isomenthol | L-Isopulegol | Neben-Komp. |
|---|---|---|---|---|---|---|
| 0,08/0 | 97,3 | 1,0 | 0,29 | 0,20 | 0,29 | 0,33 |

### Beispiel 20:

Es wurde eine Hydrierapparatur bestehend aus einem Hauptreaktor (HR) und einem Nachreaktor (NR) eingesetzt. Der Hauptreaktor wies 5 in Reihe geschaltete Röhren mit einem Innendurchmesser von 5 mm und einer Länge von 1,3 m auf, die mit 104 g (127 ml) eines Festbettkatalysator bestehend aus 0,47 Gew.-% Palladium auf einem γ-Al₂O₃-Träger in Form von Strängen mit einer Länge von 4 mm gefüllt waren. Der Nachreaktor (Doppelmantel) bestand aus einem Rohr mit einem Innendurchmesser von 5 mm und einer Länge von 1,9 m, das mit 27 g (35 ml) des gleichen Katalysators gefüllt war.

Mittels einer Kreiselpumpe wurde ein Umwälzkreislauf über den Hauptreaktor mit einer Rate von etwa 500 g/h bei einem Zulauf an L-Isopulegol von 24,5 g/h (Gesamtmenge 588 g) mit einer Reinheit von 99,8% und 99,8% ee bei einem konstanten Wasserstoffdruck von 30 bar betrieben. Der Hauptreaktor wurde mit einer Temperatur von 50°C und der Nachreaktor mit 60°C betrieben. Sämtliche Rohrleitungen wurden zur Vermeidung der Auskristallisation des enantiomerenreinen L-Menthols (Smp. 44°C) mit einer elektrischen Begleitheizung versehen. Man erhielt L-Menthol (99,8% ee) in einer Menge von 597 g entsprechend einer Rate von 24,9 g/h. Das so erhaltene Produkt wurde gaschromatographisch analysiert. Die Ergebnisse sind in Tabelle 4 zusammengestellt.

**Tabelle 4: GC-Analytik des erhaltenen L-Menthols (GC-FI.-%)**

| Menthon / Isomenthon | L-Menthol | Neomenthol | Neoisomenthol | Isomenthol | L-Isopulegol | Neben-Komp. |
|---|---|---|---|---|---|---|
| 0,64 / 0,56 | 97,5 | 0 | 0,66 | 0 | 0,29 | 0,10 |

### Beispiele Schritt e): Feindestillation von Menthol

### Beispiel 21:

Eine Labor-Trennwandkolonne wurde aus fünf je 1,2 m langen Glasschüssen mit 64 mm Innendurchmesser aufgebaut. In die drei mittleren Schüsse wurde eine Trennwand aus Metallblech gesteckt. Unterhalb und oberhalb des Trennwandbereiches wurden Laborpackungen (Sulzer CY) und im Trennwandbereich Metall-Geweberinge aus Edelstahl mit 5 mm Durchmesser eingebaut. Bei Trennleistungsmessungen, die mit dem Xylol-Isomerengemischen bei einem Kopfdruck von 60 mbar durchgeführt wurden, konnte eine Gesamttrennleistung von 100 theoretischen Stufen über die gesamte Kolonne und etwa 55 theoretischen Stufen im Trennwandbereich gemessen werden. Die Gesamtzahl der vorhandenen theoretischen Stufen betrug also etwa 155. Die Kolonne wurde mit einem ölbeheizten Dünnschichtverdampfer (0,1 m²) und einem mit Kühlwasser gekühlten Kondensator ausgerüstet.

Temperaturen an verschiedenen Höhen in der Kolonne sowie der Kopfdruck und der Druckverlust über die Kolonne wurden mittels eines Messwerterfassungssystem gemessen. Die Kolonne verfügte über Durchflussmessungen in den Zu- und Abläufen sowie eine Mengenmessung des Rücklaufs, die als Regelgröße für die Vorlauftemperatur des Ölthermostaten diente. Durch diese Regelung wurde eine konstante Rücklaufmenge gewährleistet, wodurch sich auch ein konstanter Differenzdruck einstellte. Die Aufteilung der Flüssigkeitsmenge oberhalb der Trennwand auf Zulauf- und Entnahmeteil der Trennwand wurde über einen zeitlich getakteten Schwenktrichter realisiert.

Der Kolonne wurde in der Kolonnenmitte einer Höhe von 331 cm zum Zulaufteil der Trennwand 1000 g/h eines auf 90°C vorgewärmten flüssigen Menthols pflanzlicher Herkunft, das 99,58 GC-Flächen-% Menthol, 0,22 GC-Flächen-% Isopulegol, 0,11 GC-Flächen-% andere Neomenthole und 0,03 GC-Flächen-% Isomenthol sowie 0,02 GC-Flächen-% Neoisomenthol enthielt zugeführt. Die Kolonne wurde bei 50 mbar Kopfdruck und einem Rücklauf von 3,0 kg/h betrieben. Dabei stellte sich ein Druckverlust von etwa 34 mbar (± 1 mbar) ein. Am Kopf der Kolonne wurde eine Temperatur von 121°C und im Sumpf eine Temperatur von 135 °C (± 0,5 K) gemessen. Der Sumpfabzug wurde über eine Waagensteuerung fest auf 2 g/h (± 1 g/h) und die Destillatabnahme auf 4 g/h (± 1 g/h) eingestellt. Das Rücklaufverhältnis betrug somit etwa 750 : 1. Der Kondensator der Kolonne wurde auf 25°C temperiert, um Feststoffbildung zu vermeiden.

Die Flüssigkeit wurde oberhalb der Trennwand in einem Verhältnis von 1 : 1 (Zulauf- : Entnahmeteil) aufgeteilt. In einer Höhe von 300 cm im Abnahmeteil der Trennwand wurde ein gasförmiger Seitenabzug (f) entnommen und in einem Glaskühler kondensiert, aus dem in Abhängigkeit vom Sumpffüllstand etwa 992 bis 995 g/h Reinprodukt über eine Pumpe abgezogen wurde.

Die gewonnenen Fraktionen wurden gaschromatographisch mit Hilfe eines Standard-GC analysiert. Probenvorbereitung: Die (fest gewordene) Probe wurde unter Schmelzen auf etwa 50°C erwärmt und in Toluol gelöst. Die toluolische Lösung wurde in den Gaschromatografen eingespritzt, bei der Integration wurde entsprechend der Toluol-Peak ausgeblendet.

Gaschromatographische Analysen wurden nach der folgenden Methode durchgeführt: 50 m CP-Wax 52 CB, ID.: 0,32 mm, FD.: 1,2 µm; Injektor: 200°C; Detektor: 250°C; 80°C - 3°C/min auf 200°C, - 10°C/min auf 230°C /15 min;
t_{R} (Isopulegol): 30,07 min; t_{R} (Neomenthol): 31,08 min; t_{R} (Neoisomenthol): 32,5 min; t_{R} (Menthol): 32,8 min; t_{R} (Isomenthol): 33,8 min

Das am Seitenabzug gewonnene Reinprodukt enthielt neben 99,94 GC-Flächen-% L-Menthol auch 0,02 GC-Flächen-% Isomenthol und Spuren anderer Menthol-Diastereomere. Im Sumpfabzug wurden durch GC-Analyse 96,12 GC-Flächen-% L-Menthol bestimmt, das Destillat enthielt 44,7 GC-Flächen-% L-Menthol, 33,9 GC-Flächen-% Isopulegol, 12,9 GC-Flächen-% Neo-Menthol und 2,02 GC-Flächen-% Neoisomenthol. Die Destillationsausbeute am Seitenabzug lag somit oberhalb 99 %.

### Beispiel 22:

Der Trennwandkolonne aus Beispiel 21 wurde in der Kolonnenmitte auf einer Höhe von 331 cm zum Zulaufteil der Trennwand 900 g/h eines auf 105°C vorgewärmten flüssigen L-Menthols synthetischer Herkunft, das durch katalytische Hydrierung von L-Isolulegol an einem Nickel-haltigen Katalysator erhalten wurde, zugeführt, das 99,39 GC-Flächen-% L-Menthol, 0,29 GC-Flächen-% Isopulegol, 0,25 GC-Flächen-% Neomenthol und 0,011 GC-Flächen-% Isomenthol sowie 0,044 GC-Flächen-% Neoisomenthol enthielt. Die Kolonne wurde bei 50 mbar Kopfdruck und einem Rücklauf von 3,0 kg/h betrieben. Dabei stellte sich ein Druckverlust von etwa 35 mbar (± 1 mbar) ein. Am Kopf der Kolonne wurde eine Temperatur von 120°C und im Sumpf eine Temperatur von 135 °C (± 0,5 K) gemessen. Die Kolonne wurde ohne Sumpfabzug betrieben und die Destillatabnahme über eine Waagensteuerung auf 15 g/h (± 1 g/h) eingestellt. Das Rücklaufverhältnis betrug somit etwa 200 : 1. Der Kondensator der Kolonne wurde auf 40°C temperiert, um Feststoffbildung zu vermeiden.

Die Flüssigkeit wurde oberhalb der Trennwand in einem Verhältnis von 1 : 1 (Zulauf- : Entnahmeteil) aufgeteilt. In einer Höhe von 300 cm im Abnahmeteil der Trennwand wurde ein gasförmiger Seitenabzug (f) entnommen und in einem Glaskühler kondensiert, aus dem in Abhängigkeit vom Sumpffüllstand etwa 885 bis 890 g/h Reinprodukt über eine Pumpe abgezogen wurde.

Das am Seitenabzug gewonnene Reinprodukt enthielt neben 99,93 GC-Flächen-% L-Menthol auch 0,027 GC-Flächen-% Neomenthol und Spuren anderer Menthol-Diastereomere. Das auch bei Raumtemperatur flüssige Destillat enthielt 73,1 GC-Flächen-% L-Menthol, 13,5 GC-Flächen-% Isopulegol, 10,9 GC-Flächen-% Neomenthol und 1,79 GC-Flächen-% Neoisomenthol. Der kontinuierlich betriebenen Kolonne wurde innerhalb 24,5 h mit 22,05 kg Zulauf zugeführt und 21,6 kg Reinprodukt am Seitenabzug entnommen. Die Destillationsausbeute am Seitenabzug lag somit oberhalb 98,5%.

### Beispiel 23:

In einer mit 1 m Sulzer DX-Packung ausgerüsteten Glas-Laborkolonne (theoretische Stufenzahl von etwa 20) mit einem Innendurchmesser von 50 mm, die mit einer Blase und einem umgepumpten Dünnschichtverdampfer (0,05m²) ausgerüstet ist, wurden 614 g eines synthetisch hergestellten L-Menthols mit 98,0 GC.-Flächen-% L-Menthol, 1,69 GC.-Flächen-% Isopulegol und 0,33 GC.-Flächen-% Neomenthol batchweise bei einem Kopfdruck von 50 mbar destilliert. Der Kondensator der Kolonne wurde mit auf 40°C temperiertem Wasser betrieben.

Die Temperaturen am Kopf der Kolonne lagen zwischen 122 und 123°C, die Sumpftemperatur zwischen 124°C zu Beginn und 125°C gegen Ende der Destillation. Der Destillatbehälter wurde dabei auf etwa 60°C elektrisch beheizt, um ein Festwerden der Fraktion zu verhindern. Bei einem Rücklaufverhältnis von 15 : 1 wurden 3 Fraktionen (31, 45 und 138 g) sowie bei einem Rücklaufverhältnis von 10 : 1 eine weitere Destillat-Fraktion von 116 g gewonnen. Die erste gewonnene Fraktion enthielt 75,5 GC-Flächen-% L-Menthol, 19,6 GC-Flächen-% Isopulegol und 3,01 GC-Flächen-% Neomenthol und blieb auch bei Raumtemperatur flüssig. Die zweite Fraktion enthielt 90,6 GC-Flächen-% Menthol, 7,03 GC-Flächen-% Isopulegol und 1,49 GC-Flächen-% Neomenthol, die dritte entsprechend 98,09 GC-Flächen-% L-Menthol, 0,98 GC-Flächen-% Isopulegol und 0,3 GC-Flächen-% Neomenthol. In der vierten Fraktion wurde schließlich eine Menthol-Reinheit von 99,52 GC-Flächen-% erreicht. Aus der Blase wurden 197 g Rückstand isoliert, mit 98,5 GC-Flächen-% L-Menthol.

### Beispiel 24:

Eine weitere Labor-Trennwandkolonne wurde aus drei Glasschüssen mit 43 mm Innendurchmesser aufgebaut. Der mittlere Kolonnenschuss mit einer Gesamtlänge von 105 cm wurde mit einer fest eingeschmolzenen Trennwand aus etwa 1 mm starkem Glas versehen. Im Bereich der Trennwand ist die Kolonne mit 1 m Sulzer DX-Packung auf der Zulaufseite und 0,9 m DX-Packung auf der Abnahmeseite ausgerüstet. Oberhalb und unterhalb der Trennwand wurden 50 mm lange Glasschüsse eingesetzt, die je mit 33 cm Sulzer DX-Packungen ausgerüstet wurden.

Bei Trennleistungsmessungen, die mit dem Xylol-Isomerengemischen bei einem Kopfdruck von 60 mbar durchgeführt wurden, konnte eine Gesamttrennleistung von etwa 32 theoretischen Stufen über die gesamte Kolonne und etwa 18 theoretischen Stufen im Trennwandbereich gemessen werden. Die Gesamtzahl der vorhandenen theoretischen Stufen betrug also etwa 50. Die Kolonne wurde mit einem ölbeheizten Dünnschichtverdampfer (0,1 m²) und einem mit auf 25°C temperierbaren Kühlwasser gekühlten Kondensator ausgerüstet. Zu- und Ablauf befanden sich jeweils mittig in der Trennwand und waren jeweils beheizt ausgeführt. Ebenso waren Rücklaufleitungen und Sumpfaustragsleitungen mit elektrischen Begleitheizungen versehen.

Die Temperaturen an verschiedenen Höhen in der Kolonne sowie der Kopfdruck und der Druckverlust über die Kolonne wurden mittels eines Messwerterfassungssystems gemessen. Die Kolonne verfügte über Durchflussmessungen in den Zu- und Abläufen sowie eine Mengenmessung mit Regelung des Rücklaufs. Durch diese Regelung wurde eine konstante Rücklaufmenge gewährleistet, wodurch sich auch ein konstanter Differenzdruck einstellte. Die Aufteilung der Flüssigkeitsmenge oberhalb der Trennwand auf Zulauf- und Entnahmeteil der Trennwand wurde über einen zeitlich getakteten Schwenktrichter realisiert.

Der Trennwandkolonne wurde in der Kolonnenmitte zum Zulaufteil der Trennwand kontinuierlich 120 g/h eines auf 80°C vorgewärmten flüssigen, nahezu racemischem Menthols synthetischer Herkunft, das durch katalytische Hydrierung von Isolulegol an einem Nickel-haltigen Katalysator erhalten wurde, zugeführt, das 85,1 Gew.-% Menthol, 0,2 Gew.-% Isopulegol, 3,4 Gew.-% Neomenthol und 0,98 Gew.-% Isomenthol sowie 1,25 GC-Flächen-% Neoisomenthol enthielt. Außerdem waren 1,5 GC-Gew.-% des Kohlenwasserstoffs Phenylcyclohexan enthalten.

Die gewonnenen Fraktionen wurden gaschromatographisch mit Hilfe eines Standard-GC analysiert. Probenvorbereitung: Die (zum Teil fest gewordene) Probe wurde unter Schmelzen auf etwa 50°C erwärmt und in Toluol gelöst. Die toluolische Lösung wurde in den Gaschromatographen eingespritzt, bei der Integration wurde entsprechend der Toluol-Peak ausgeblendet. Als interner Standard für die Gew.-% Bestimmung wurde Diethylenglykoldiethylether verwendet (Einwage ca. 10% der Gesamtprobenmenge).

Gaschromatographische Analysen wurden nach der folgenden Methode durchgeführt: 50 m CP-Wax 52 CB , ID.: 0,32 mm, FD.: 1,2 µm; Injektor: 200°C; Detektor: 250 °C; 80°C - 3 °C/min auf 200 °C, - 10 °C/min auf 230 °C /15 min;
t_{R} (Diethylenglykoldiethylether): 23,0 min, t_{R} (Isopulegol): 30,07 min; t_{R} (Neo-Menthol): 31,08 min; t_{R} (Neoiso-Menthol): 32,5 min; t_{R} (Menthol): 32,8 min; t_{R} (Iso-Menthol): 33,8 min, t_{R} (Phenylcyclohexan): 35,2 min

Die Kolonne wurde bei 18 mbar Kopfdruck und einem Rücklauf von 850 g/h betrieben. Dabei stellte sich ein Druckverlust von etwa 3 mbar ein. Am Kopf der Kolonne wurde eine Temperatur von 101 °C und im Sumpf eine Temperatur von 105 °C (± 0,5 K) gemessen. Die Kolonne wurde mit 15 g/h (± 2 g/h) Sumpfabzug betrieben und die Destillatabnahme über eine Waagensteuerung auf 50 g/h (± 5 g/h) eingestellt. Das Rücklaufverhältnis betrug somit etwa 17: 1. Der Kondensator der Kolonne wurde auf 25°C temperiert, um Feststoffbildung zu vermeiden.

Die Flüssigkeit wurde oberhalb der Trennwand in einem Verhältnis von 3 : 4 (Zulauf- : Entnahmeteil) aufgeteilt. In der Mitte des Abnahmeteil der Trennwand wurde mit Hilfe einer Membranpumpe ein flüssiger Seitenabzug (f) von etwa 55 g/h (± 5 g/h) entnommen

Das am Seitenabzug gewonnene Reinprodukt enthielt neben 98,2 Gew.-% Menthol auch 0,14 Gew.-% Neomenthol und 0,92 GC-Gew.-% Isomenthol sowie 0,25 GC-Flächen-% Neoisomenthol und etwa 0,45 Gew.-% Phenylcyclohexan. Das Reinprodukt wies eine spezifische Drehung von -0,9 grd/(ml*g) auf (Bestimmung nach USP30/NF25 "Menthol").

Das auch bei Raumtemperatur flüssige Destillat enthielt 79,6 Gew.-% Menthol, 0,67 GC-Gew.-% Isopulegol, 6,9 GC-Gew.-% Neo-Menthol und 2,5 GC-Flächen-% Neoisomenthol sowie 3,0 Gew.-% Phenylcyclohexan. Im Sumpf wurden neben 85,7 Gew-% Menthol noch 2,9 Gew.-% Isomenthol gemessen.

## Patentansprüche

1. Verfahren zur Herstellung von Menthol, umfassend die Verfahrensschritte
a.1) katalytische Hydrierung von Neral und/oder Geranial zu Citronellal,
b.1) Cyclisierung von Citronellal zu Isopulegol in Gegenwart eines sauren Katalysators,
c.1) Aufreinigung von Isopulegol durch Kristallisation und
d.1) katalytische Hydrierung von Isopulegol zu Menthol.

2. Verfahren nach Anspruch 1 zur Herstellung von optisch aktivem Menthol, umfassend die Verfahrensschritte
a.2) asymmetrische katalytische Hydrierung von Neral und/oder Geranial zu optisch aktivem Citronellal,
b.2) Cyclisierung von optisch aktivem Citronellal zu optisch aktivem Isopulegol in Gegenwart eines sauren Katalysators,
c.2) Aufreinigung von optisch aktivem Isopulegol durch Kristallisation und
d.2) katalytische Hydrierung von optisch aktivem Isopulegol zu optisch aktivem Menthol.

3. Verfahren nach Anspruch 1 oder 2 zur Herstellung von optisch aktivem Menthol und racemischem oder enantiomerenabgerichertem Menthol, umfassend die Verfahrensschritte
a.3) asymmetrische katalytische Hydrierung von Neral und/oder Geranial zu optisch aktivem Citronellal mit einem Enantiomerenüberschuss im Bereich von 70 bis 99%,
b.3) Cyclisierung von optisch aktivem Citronellal zu optisch aktivem Isopulegol in Gegenwart eines sauren Katalysators,
c.3) Aufreinigung von optisch aktivem Isopulegol durch Kristallisation unter Erhalt von enantiomerenangereichertem Isopulegol und racemischem oder enantiomerenabgereichertem Isopulegol und
d.3) katalytische Hydrierung von enantiomerenangereichertem optisch aktivem Isopulegol zu optisch aktivem Menthol und katalytische Hydrierung von racemischem oder enantiomerenabgereichertem Isopulegol zu racemischem oder enantiomerenabgerichertem Menthol.

4. Verfahren nach einem der Ansprüche 1 bis 3 zur Herstellung von L-(-)-Menthol, umfassend die Verfahrensschritte
a.4) asymmetrische katalytische Hydrierung von Neral zu D-(+)-Citronellal,
b.4) Cyclisierung von D-(+)-Citronellal zu L-(-)-Isopulegol in Gegenwart eines sauren Katalysators,
c.4) Aufreinigung von L-(-)-Isopulegol durch Kristallisation und
d.4) katalytische Hydrierung von L-(-)-Isopulegol zu L-(-)-Menthol.

5. Verfahren nach einem der Ansprüche 1 bis 4 zur Herstellung von enantiomerenangereichertem L-(-)- Menthol und racemischem oder enantiomerenabgereichertem Menthol, umfassend die Verfahrensschritte
a.5) asymmetrische katalytische Hydrierung von Neral zu D-(+)-Citronellal mit einem Enantiomerenüberschuss von 80 bis 99%,
b.5) Cyclisierung von D-(+)-Citronellal zu L-(-)-Isopulegol in Gegenwart eines sauren Katalysators,
c.5) Aufreinigung von L-(-)-Isopulegol durch Kristallisation unter Erhalt von enantiomerenangereichertem L-(-)-Isopulegol und racemischem oder enantiomerenabgereichertem Isopulegol und
d.5) katalytische Hydrierung von enantiomerenangreichertem L-(-)- Isopulegol zu enantiomerenangereichertem L-(-)- Menthol und katalytische Hydrierung von racemischem oder enantiomerenabgereichertem Isopulegol zu racemischem oder enantiomerenabgereichertem Menthol.

6. Verfahren nach einem der Ansprüche 1 bis 5, zusätzlich umfassend als Verfahrensschritt 0) die destillative Trennung von Geranial und Neral enthaltenden Gemischen unter Erhalt von angereichertem oder reinem Geranial oder Neral.

7. Verfahren nach Anspruch 6, **dadurch gekennzeichnet, dass** man die destillative Trennung von Geranial und Neral enthaltenden Gemischen in Form eines kontinuierlichen Verfahrens zur Herstellung von Neral in reiner oder angereicherter Form durch destillative Abtrennung von Neral aus Stoffgemischen enthaltend Neral und Geranial durchführt, wobei man die destillative Abtrennung in einer Trennwandkolonne oder in einer Zusammenschaltung von zwei Destillationskolonnen in Form einer thermischen Kopplung mit 80 bis 200 theoretischen Trennstufen und einer oder mehrerer Seitenabzugsstellen bei einem absoluten Betriebsdruck von 5 bis 200 mbar durchführt.

8. Verfahren nach einem der Ansprüche 6 oder 7, **dadurch gekennzeichnet, dass** man als Geranial und Neral enthaltendes Gemisch Citral einsetzt.

9. Verfahren nach einem der Ansprüche 2 bis 8, **dadurch gekennzeichnet, dass** man gemäß Verfahrensschritt a) eine asymmetrische katalytische Hydrierung von Neral und/oder Geranial in Gegenwart von Wasserstoff und eines chiralen Übergangsmetallkatalysators, der mindestens einen optisch aktiven PhosphinLiganden aufweist, durchführt.

10. Verfahren nach einem der Ansprüche 2 bis 9, **dadurch gekennzeichnet, dass** man die asymmetrische katalytische Hydrierung gemäß Verfahrensschritt a) in Gegenwart von im Reaktionsgemisch löslichen, optisch aktiven Übergangsmetallkatalysatoren, die mindestens einen Kohlenmonoxid-Liganden aufweisen, durchführt, wobei man den Katalysator mit einem Kohlenmonoxid und Wasserstoff enthaltenden Gasgemisch vorbehandelt und/oder die asymmetrische Hydrierung in Gegenwart von dem Reaktionsgemisch zusätzlich zugeführtem Kohlenmonoxid durchführt.

11. Verfahren nach einem der Ansprüche 1 bis 10, **dadurch gekennzeichnet, dass** man die Cyclisierung gemäß Verfahrensschritt b) in Gegenwart eines Lewissauren Aluminium-haltigen Katalysators durchführt.

12. Verfahren gemäß einem der Ansprüche 1 bis 11, **dadurch gekennzeichnet, dass** man die Cyclisierung gemäß Verfahrensschritt b) in Gegenwart von Diarylphenoxy-Aluminium-Verbindungen als Katalysator durchführt, die erhältlich sind durch Umsetzung eines Bis(diarylphenol)-Liganden der Formel (I) 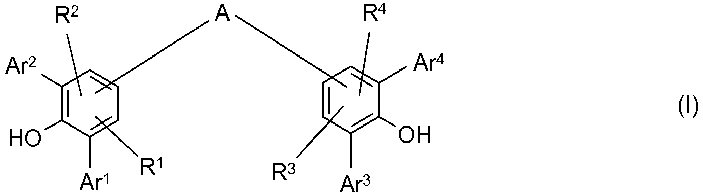 wobei
Ar¹, Ar², Ar³, Ar⁴ gleich oder verschieden sind und jeweils unabhängig voneinander einen Arylrest mit 6 bis15 Kohlenstoffatomen oder einen Heteroarylrest mit 2 bis 15 Kohlenstoffatomen, die gegebenenfalls jeweils 1 bis 7 gleiche oder verschiedene Substituenten, ausgewählt aus der Gruppe der Substituenten C₁- bis C₆-Alkyl, C₁- bis C₆-Perfluoralkyl, C₁- bis C₆-Alkoxy, C₇- bis C₁₂-Aralkyl, Halogen, -SiR^{5a}R^{6a}R^{7a}, substituiertes oder unsubstituiertes C₆- bis C₁₀-Aryl, -NR^{8a}R^{9a}, -SR^{10a} und -NO₂ tragen können, bedeuten,
R¹, R², R³, R⁴ gleich oder verschieden sind und jeweils unabhängig voneinander Wasserstoff, C₁- bis C₆-Alkyl, C₁- bis C₆-Perfluoralkyl, C₁-bis C₆-Alkoxy, C₇-bis C₁₂-Aralkyl, Halogen, -SiR^{5b}R^{6b}R^{7b}, substituiertes oder unsubstituiertes C₆- bis C₁₀-Aryl, -NR^{8b}R^{9b}, -SR10^{b} und/oder -NO₂ bedeuten und R¹ oder R² und/oder R³ oder R⁴ gemeinsam mit A einen aromatischen oder nicht-aromatischen Cyclus bilden kann, bedeuten und
A
(1) für einen geradkettigen oder verzweigten und/oder cyclischen Kohlenwasserstoffrest mit 1 bis 25 Kohlenstoffatomen steht, der gesättigt oder einoder mehrfach ungesättigt und/oder teilweise aromatisch sein kann und gegebenenfalls eines oder mehrere gleiche oder verschiedene Heteroatome, ausgewählt aus der Gruppe der Heteroatome O, S und NR¹¹ und/oder eine oder mehrere gleiche oder verschiedene funktionelle Gruppen, ausgewählt aus der Gruppe der funktionellen Gruppen C(O), S(O) und S(O)₂, aufweisen kann und gegebenenfalls einen oder mehrere gleiche oder verschiedene Substituenten, ausgewählt aus der Gruppe der Substituenten C₁- bis C₆-Alkyl, C₁- bis C₆-Perfluoralkyl, C₁- bis C₆-Alkoxy, C₁- bis C₁₀- Acyloxy, C₇- bis C₁₂-Aralkyl, Halogen, -SiR^{5c}R^{6c}R^{7c}, substituiertes oder unsubstituiertes C₆- bis C₁₀-Aryl, substituiertes oder unsubstituiertes C₂- bis C₁₀-Hetaryl, -NR^{8c}R^{9c}, -SR^{10c}, -NO₂, C₁-bis C₁₂-Acyl und C₁- bis C₁₀- Carboxyl tragen kann, oder
(2) für einen Arylrest mit 6 bis 15 Kohlenstoffatomen oder einen Heteroarylrest mit 2 bis 15 Kohlenstoffatomen steht, die gegebenenfalls jeweils 1 bis 5 Substituenten, ausgewählt aus der Gruppe der Substituenten C₁- bis C₆-Alkyl, C₁- bis C₆-Perfluoralkyl, C₁- bis C₆-Alkoxy, C₇- bis C₁₂-Aralkyl, Halogen,-SiR^{5d}R^{6d}R^{7d}, substituiertes oder unsubstituiertes C₆- bis C₁₀-Aryl, -NR^{8d}R^{9d}, SR^{10d} und NO₂ tragen können, oder
(3) für eine funktionelle Gruppe oder ein Heteroatom ausgewählt aus der Gruppe -O-, -S-, -N(R¹¹)-, -S(O)-, -C(O)-, -S(O)₂-, -P(R¹¹)-, -(R¹¹)P(O)- und-Si(R¹²R¹³) steht,
wobei die Reste R^{5a}, R^{6a}, R^{7a}, R^{8a}, R^{9a}, R^{10a} bis R^{5d}, R^{6d}, R^{7d} R^{8d}, R^{9d}, R^{10d} und R¹¹ bis R¹³ jeweils unabhängig voneinander für C₁- bis C₆-Alkyl, C₇- bis C₁₂-Aralkyl und/oder substituiertes oder unsubstituiertes C₆- bis C₁₀-Aryl stehen, und die Reste R^{8a} und R^{9a}, R^{8b} und R^{9b}, R^{8c} und R^{9c}, R^{8d} und R^{9d} unabhängig voneinander jeweils gemeinsam auch einen cyclischen Kohlenwasserstoffrest mit 2 bis 8 Kohlenstoffatomen bilden können, der eines oder mehrere gleiche oder verschiedene Heteroatome ausgewählt aus der Gruppe O, S und NR^{11a} aufweisen kann und R^{11a} die für R¹¹ angegebenen Bedeutungen haben kann,
mit einer Aluminium-Verbindung der Formel (XIV)
(R¹⁴)₃₋ₚAlHp (XIV),
wobei
Al Aluminium bedeutet und
R¹⁴ einen verzweigten oder unverzweigten Alkylrest mit 1 bis 5 Kohlenstoffatomen und
p 0 oder eine ganze Zahl von 1 bis 3 bedeutet,
und/oder
mit einer Aluminium-Verbindung der Formel (XV)
MAlH₄ (XV),
wobei
Al Aluminium bedeutet und
M Lithium, Natrium oder Kalium bedeutet.

13. Verfahren nach Anspruch 12, **dadurch gekennzeichnet, dass** man die Cyclisierung gemäß Verfahrensschritt b) in Gegenwart einer Diarylphenoxy-Aluminium-Verbindung als Katalysator durchführt, die erhältlich ist durch Umsetzung eines Bis(diarylphenol)-Liganden der Formel (Ia₂-3) 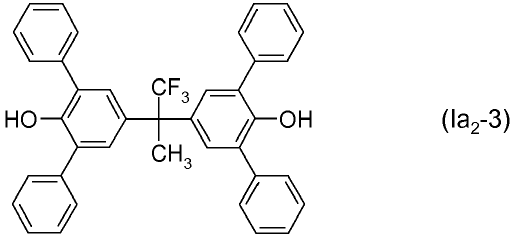 mit einer Aluminium-Verbindung der Formel (XIV) und/oder mit einer Aluminium-Verbindung der Formel (XV).

14. Verfahren nach einem der Ansprüche 1 bis 13, **dadurch gekennzeichnet, dass** man das erhaltene Isopulegol destillativ aufreinigt, wobei man die Aufreinigung in einer Trennwandkolonne mit 30 bis 200 theoretischen Trennstufen und einer oder mehrerer Seitenabzugsstellen bei einem absoluten Betriebsdruck von 10 bis 500 mbar durchführt.

15. Verfahren nach einem der Ansprüche 1 bis 14, **dadurch gekennzeichnet, dass** man die Aufreinigung von Isopulegol gemäß Verfahrensschritt c) in Form einer Schmelzekristallisation durchführt.

16. Verfahren nach Anspruch 15, **dadurch gekennzeichnet, dass** man die Schmelzekristallisation in Form einer statischen Schichtkristallisation durchführt.

17. Verfahren nach einem der Ansprüche 1 bis 16, **dadurch gekennzeichnet, dass** man die katalytische Hydrierung gemäß Verfahrensschritt d) in Form eines Verfahrens zur Herstellung von racemischem oder optisch aktivem Menthol der Formel (XXIV) 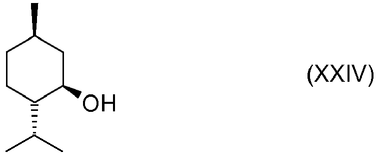 durch katalytische Hydrierung von racemischem oder optisch aktivem Isopulegol der Formel (XX) 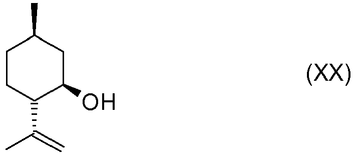 in Gegenwart von Wasserstoff und einem Katalysator, umfassend
- 30 bis 70 Gew.-% sauerstoffhaltige Verbindungen des Nickels, berechnet als NiO,
- 15 bis 45 Gew.-% sauerstoffhaltige Verbindungen des Zirkons, berechnet als ZrO₂,
- 5 bis 30 Gew.-% sauerstoffhaltige Verbindungen des Kupfers, berechnet als CuO und
- 0,1 bis 10 Gew.-% sauerstoffhaltige Verbindungen des Molybdäns, berechnet als MoO₃,
wobei sich die Angaben in Gew.-% auf den trockenen, nicht reduzierten Katalysator beziehen, durchführt.

18. Verfahren nach einem der Ansprüche 1 bis 17, **dadurch gekennzeichnet, dass** man das erhaltene optisch aktive und/oder racemische Menthol im Rahmen eines zusätzlichen Verfahrensschritts e) destillativ aufreinigt.

19. Verfahren nach Anspruch 18, **dadurch gekennzeichnet, dass** man die destillative Aufreinigung von racemischem und/oder optisch aktivem Menthol durch destillative Abtrennung von optisch aktivem oder racemischem Menthol aus Stoffgemischen enthaltend racemisches oder optisch aktives Menthol und Diastereomere des Menthols durchführt, wobei man die Abtrennung in einer Trennwandkolonne mit 50 bis 300 theoretischen Trennstufen und einer oder mehrerer Seitenabzugsstellen bei einem absoluten Betriebsdruck von 5 bis 500 mbar durchführt.

## Claims

1. A method for producing menthol, comprising the steps
a.1) catalytic hydrogenation of neral and/or geranial to give citronellal,
b.1) cyclization of citronellal to give isopulegol in the presence of an acidic catalyst,
c.1) purification of isopulegol by crystallization and
d.1) catalytic hydrogenation of isopulegol to give menthol.

2. The method according to claim 1 for producing optically active menthol, comprising the steps
a.2) asymmetric catalytic hydrogenation of neral and/or geranial to give optically active citronellal,
b.2) cyclization of optically active citronellal to give optically active isopulegol in the presence of an acidic catalyst,
c.2) purification of optically active isopulegol by crystallization and
d.2) catalytic hydrogenation of optically active isopulegol to give optically active menthol.

3. The method according to claim 1 or 2 for producing optically active menthol and racemic or enantiomerically depleted menthol, comprising the steps
a.3) asymmetric catalytic hydrogenation of neral and/or geranial to give optically active citronellal with an enantiomer excess in the range from 70 to 99%,
b.3) cyclization of optically active citronellal to give optically active isopulegol in the presence of an acidic catalyst,
c.3) purification of optically active isopulegol by crystallization to give enantiomerically enriched isopulegol and racemic or enantiomerically depleted isopulegol and
d.3) catalytic hydrogenation of enantiomerically enriched optically active isopulegol to give optically active menthol and catalytic hydrogenation of racemic or enantiomerically depleted isopulegol to give racemic or enantiomerically depleted menthol.

4. The method according to any one of claims 1 to 3 for producing L-(-)-menthol, comprising the steps
a.4) asymmetric catalytic hydrogenation of neral to give D-(+)-citronellal,
b.4) cyclization of D-(+)-citronellal to give L-(-)-isopulegol in the presence of an acidic catalyst,
c.4) purification of L-(-)-isopulegol by crystallization and
d.4) catalytic hydrogenation of L-(-)-isopulegol to give L-(-)-menthol.

5. The method according to any one of claims 1 to 4 for producing enantiomerically enriched L-(-)-menthol and racemic or enantiomerically depleted menthol, comprising the steps
a.5) asymmetric catalytic hydrogenation of neral to give D-(+)-citronellal with an enantiomer excess of from 80 to 99%,
b.5) cyclization of D-(+)-citronellal to give L-(-)-isopulegol in the presence of an acidic catalyst,
c.5) purification of L-(-)-isopulegol by crystallization to give enantiomerically enriched L-(-)-isopulegol and racemic or enantiomerically depleted isopulegol and
d.5) catalytic hydrogenation of enantiomerically enriched L-(-)-isopulegol to give enantiomerically enriched L-(-)-menthol and catalytic hydrogenation of racemic or enantiomerically depleted isopulegol to give racemic or enantiomerically depleted menthol.

6. The method according to any one of claims 1 to 5, additionally comprising as step 0) the distillative separation of mixtures comprising geranial and neral to give enriched or pure geranial or neral.

7. The method according to claim 6, wherein the distillative separation of mixtures comprising geranial and neral is carried out in the form of a continuous method for producing neral in pure or enriched form by distillative removal of neral from substance mixtures comprising neral and geranial, where the distillative removal is carried out in a dividing wall column or in an interconnection of two distillation columns in the form of a thermal coupling having 80 to 200 theoretical plates and one or more side take-off points at an absolute operating pressure of from 5 to 200 mbar.

8. The method according to either of claims 6 and 7, wherein citral is used as mixture comprising geranial and neral.

9. The method according to any one of claims 2 to 8, wherein, according to step a), an asymmetric catalytic hydrogenation of neral and/or geranial is carried out in the presence of hydrogen and a chiral transition metal catalyst which has at least one optically active phosphine ligand.

10. The method according to any one of claims 2 to 9, wherein the asymmetric catalytic hydrogenation according to step a) is carried out in the presence of optically active transition metal catalysts which are soluble in the reaction mixture and which have at least one carbon monoxide ligand, where the catalyst is pretreated with a gas mixture comprising carbon monoxide and hydrogen and/or the asymmetric hydrogenation is carried out in the presence of carbon monoxide additionally fed to the reaction mixture.

11. The method according to any one of claims 1 to 10, wherein the cyclization according to step b) is carried out in the presence of a Lewis-acidic aluminum-containing catalyst.

12. The method according to any one of claims 1 to 11, wherein the cyclization according to step b) is carried out in the presence of diarylphenoxyaluminum compounds as catalyst which are obtainable by reacting a bis(diarylphenol) ligand of the formula (I) 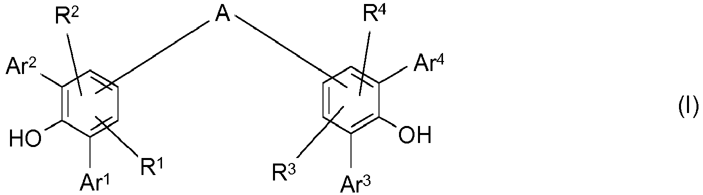 where
Ar¹, Ar², Ar³, Ar⁴ are identical or different and are in each case independently of one another an aryl radical having 6 to 15 carbon atoms or a heteroaryl radical having 2 to 15 carbon atoms which can optionally carry in each case 1 to 7 identical or different substituents selected from the group of the substituents C₁- to C₆-alkyl, C₁- to C₆-perfluoroalkyl, C₁- to C₆-alkoxy, C₇- to C₁₂-aralkyl, halogen, -SiR^{5a}R^{6a}R^{7a}, substituted or unsubstituted C₆- to C₁₀-aryl, -NR^{8a}R^{9a}, -SR^{10a} and -NO₂,
R¹, R², R³, R⁴ are identical or different and are in each case independently of one another hydrogen, C₁- to C₆-alkyl, C₁- to C₆-perfluoroalkyl, C₁- to C₆-alkoxy, C₇- to C₁₂-aralkyl, halogen, -SiR^{5b}R^{6b}R^{7b}, substituted or unsubstituted C₆- to C₁₀-aryl,-NR^{8b}R^{9b}, -SR10^{b} and/or -NO₂, and R¹ or R² and/or R³ or R⁴, together with A, can form an aromatic or nonaromatic cycle, and
A
(1) is a straight-chain or branched and/or cyclic hydrocarbon radical having 1 to 25 carbon atoms which may be saturated or mono- or polyunsaturated and/or partially aromatic and can optionally have one or more identical or different heteroatoms selected from the group of the heteroatoms 0, S and NR¹¹ and/or one or more identical or different functional groups selected from the group of the functional groups C(O), S(O) and S(O)₂, and can optionally carry one or more identical or different substituents selected from the group of the substituents C₁- to C₆-alkyl, C₁- to C₆-perfluoroalkyl, C₁- to C₆-alkoxy, C₁- to C₁₀-acyloxy, C₇- to C₁₂-aralkyl, halogen,-SiR^{5c}R^{6c}R^{7c}, substituted or unsubstituted C₆- to C₁₀-aryl, substituted or unsubstituted C₂- to C₁₀-hetaryl, -NR^{8c}R^{9c}, -SR^{10c}, -NO₂, C₁- to C₁₂-acyl and C₁- to C₁₀-carboxyl, or
(2) is an aryl radical having 6 to 15 carbon atoms or a heteroaryl radical having 2 to 15 carbon atoms which can optionally carry in each case 1 to 5 substituents selected from the group of the substituents C₁- to C₆-alkyl, C₁- to C₆-perfluoroalkyl, C₁- to C₆-alkoxy, C₇- to C₁₂-aralkyl, halogen,-SiR^{5d}R^{6d}R^{7d}, substituted or unsubstituted C₆- to C₁₀-aryl, -NR^{8d}R^{9d}, SR^{10d} and NO₂, or
(3) is a functional group or a heteroatom selected from the group -0-, -S-, -N(R¹¹) -, -S(O)-, -C(O)-, -S(O)₂-,-P(R¹¹)-, -(R¹¹)P(O)- and -Si(R¹²R¹³),
where the radicals R^{5a}, R^{6a} R^{7a}, R^{8a}, R^{9a}, R^{10a} to R^{5d}, R^{6d}, R^{7d}, R^{8d}, R^{9d}, R^{10d} and R¹¹ to R¹³ are in each case independently of one another C₁- to C₆-alkyl, C₇- to C₁₂-aralkyl and/or substituted or unsubstituted C₆- to C₁₀-aryl, and the radicals R^{8a} and R^{9a}, R^{8b} and R^{9b}, R^{8c} and R^{9c}, R^{8d} and R^{9d} can independently of one another in each case together also form a cyclic hydrocarbon radical having 2 to 8 carbon atoms which can have one or more identical or different heteroatoms selected from the group 0, S and NR^{11a}, and R^{11a} can have the meanings given for R¹¹,
with an aluminum compound of the formula (XIV)
(R¹⁴)₃₋ₚAlHₚ (XIV),
where
Al is aluminum and
R¹⁴ is a branched or unbranched alkyl radical having 1 to 5 carbon atoms and
p is 0 or an integer from 1 to 3,
and/or
with an aluminum compound of the formula (XV)
MAlH₄ (XV),
where
Al is aluminum and
M is lithium, sodium or potassium.

13. The method according to claim 12, wherein the cyclization according to step b) is carried out in the presence of a diarylphenoxyaluminum compound as catalyst which is obtainable by reacting a bis(diarylphenol) ligand of the formula (Ia₂-3) 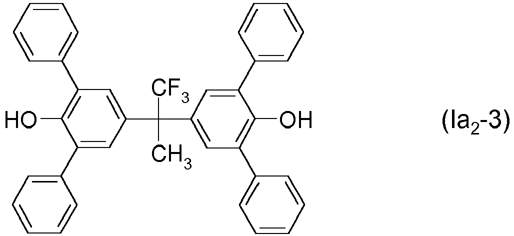 with an aluminum compound of the formula (XIV) and/or with an aluminum compound of the formula (XV).

14. The method according to any one of claims 1 to 13, wherein the resulting isopulegol is purified by distillation, where the purification is carried out in a dividing wall column having 30 to 200 theoretical plates and one or more side take-off points at an absolute operating pressure of from 10 to 500 mbar.

15. The method according to any one of claims 1 to 14, wherein the purification of isopulegol according to step c) is carried out in the form of a melt crystallization.

16. The method according to claim 15, wherein the melt crystallization is carried out in the form of a static layer crystallization.

17. The method according to any one of claims 1 to 16, wherein the catalytic hydrogenation according to step d) is carried out in the form of a method for producing racemic or optically active menthol of the formula (XXIV) 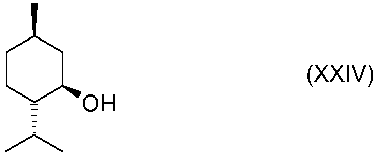 by catalytic hydrogenation of racemic or optically active isopulegol of the formula (XX) 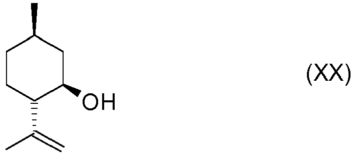 in the presence of hydrogen and a catalyst comprising
- 30 to 70% by weight of oxygen-containing compounds of nickel, calculated as NiO,
- 15 to 45% by weight of oxygen-containing compounds of zirconium, calculated as ZrO₂,
- 5 to 30% by weight of oxygen-containing compounds of copper, calculated as CuO and
- 0.1 to 10% by weight of oxygen-containing compounds of molybdenum, calculated as MoO₃,
where the data in % by weight refer to the dry, nonreduced catalyst.

18. The method according to any one of claims 1 to 17, wherein the resulting optically active and/or racemic menthol is purified by distillation in the course of an additional step e).

19. The method according to claim 18, wherein the distillative purification of racemic and/or optically active menthol is carried out by distillative removal of optically active or racemic menthol from substance mixtures comprising racemic or optically active menthol and diastereomers of menthol, where the removal is carried out in a dividing wall column having 50 to 300 theoretical plates and one or more side take-off points at an absolute operating pressure of from 5 to 500 mbar.

## Revendications

1. Procédé de fabrication de menthol, comprenant les étapes de procédé suivantes :
a.1) l'hydrogénation catalytique de néral et/ou de géranial en citronellal,
b.1) la cyclisation de citronellal en isopulégol en présence d'un catalyseur acide,
c.1) la purification de l'isopulégol par cristallisation, et
d.1) l'hydrogénation catalytique de l'isopulégol en menthol.

2. Procédé selon la revendication 1 pour la fabrication de menthol optiquement actif, comprenant les étapes de procédé suivantes :
a.2) l'hydrogénation catalytique asymétrique de néral et/ou de géranial en citronellal optiquement actif,
b.2) la cyclisation du citronellal optiquement actif en isopulégol optiquement actif en présence d'un catalyseur acide,
c.2) la purification de l'isopulégol optiquement actif par cristallisation, et
d.2) l'hydrogénation catalytique de l'isopulégol optiquement actif en menthol optiquement actif.

3. Procédé selon la revendication 1 ou 2 pour la fabrication de menthol optiquement actif et de menthol racémique ou énantiomériquement appauvri, comprenant les étapes de procédé suivantes :
a.3) l'hydrogénation catalytique asymétrique de néral et/ou de géranial en citronellal optiquement actif ayant un excès énantiomérique dans la plage allant de 70 à 99 %,
b.3) la cyclisation du citronellal optiquement actif en isopulégol optiquement actif en présence d'un catalyseur acide,
c.3) la purification de l'isopulégol optiquement actif par cristallisation avec obtention d'isopulégol énantiomériquement enrichi et d'isopulégol racémique ou énantiomériquement appauvri, et
d.3) l'hydrogénation catalytique de l'isopulégol optiquement actif énantiomériquement enrichi en menthol optiquement actif et l'hydrogénation catalytique de l'isopulégol racémique ou énantiomériquement appauvri en menthol racémique ou énantiomériquement appauvri.

4. Procédé selon l'une quelconque des revendications 1 à 3 pour la fabrication de L-(-)-menthol, comprenant les étapes de procédé suivantes :
a.4) l'hydrogénation catalytique asymétrique de néral en D-(+)-citronellal,
b.4) la cyclisation du D-(+)-citronellal en L-(-)-isopulégol en présence d'un catalyseur acide,
c.4) la purification du L-(-)-isopulégol par cristallisation, et
d.4) l'hydrogénation catalytique du L-(-)-isopulégol en L-(-)-menthol.

5. Procédé selon l'une quelconque des revendications 1 à 4 pour la fabrication de L-(-)-menthol énantiomériquement enrichi et de menthol racémique ou énantiomériquement appauvri, comprenant les étapes de procédé suivantes :
a.5) l'hydrogénation catalytique asymétrique de néral en D-(+)-citronellal ayant un excès énantiomérique de 80 à 99 %,
b.5) la cyclisation du D-(+)-citronellal en L-(-)-isopulégol en présence d'un catalyseur acide,
c.5) la purification du L-(-)-isopulégol par cristallisation avec obtention de L-(-)-isopulégol énantiomériquement enrichi et d'isopulégol racémique ou énantiomériquement appauvri, et
d.5) l'hydrogénation catalytique du L-(-)-isopulégol énantiomériquement enrichi en L-(-)-menthol énantiomériquement enrichi et l'hydrogénation catalytique de l'isopulégol racémique ou énantiomériquement appauvri en menthol racémique ou énantiomériquement appauvri.

6. Procédé selon l'une quelconque des revendications 1 à 5, comprenant en outre en tant qu'étape de procédé 0) la séparation par distillation de mélanges contenant du géranial et du néral avec obtention de géranial ou de néral enrichi ou pur.

7. Procédé selon la revendication 6, **caractérisé en ce que** la séparation par distillation de mélanges contenant du géranial et du néral est réalisée sous la forme d'un procédé continu pour la fabrication de néral sous forme pure ou enrichie par séparation par distillation de néral à partir de mélanges de matières contenant du néral et du géranial, la séparation par distillation étant réalisée dans une colonne à paroi de séparation ou dans une interconnexion de deux colonnes de distillation sous la forme d'un couplage thermique comprenant 80 à 200 étapes de séparation théoriques et un ou plusieurs emplacements de sortie latérale à une pression d'exploitation absolue de 5 à 200 mbar.

8. Procédé selon l'une quelconque des revendications 6 ou 7, **caractérisé en ce que** du citral est utilisé en tant que mélange contenant du géranial et du néral.

9. Procédé selon l'une quelconque des revendications 2 à 8, **caractérisé en ce que**, selon l'étape de procédé a), une hydrogénation catalytique asymétrique de néral et/ou de géranial est réalisée en présence d'hydrogène et d'un catalyseur de métal de transition chiral, qui comprend au moins un ligand phosphine optiquement actif.

10. Procédé selon l'une quelconque des revendications 2 à 9, **caractérisé en ce que** l'hydrogénation catalytique asymétrique selon l'étape de procédé a) est réalisée en présence de catalyseurs de métaux de transition optiquement actifs solubles dans le mélange réactionnel, qui comprennent au moins un ligand monoxyde de carbone, le catalyseur étant prétraité avec un mélange gazeux contenant du monoxyde de carbone et de l'hydrogène et/ou l'hydrogénation asymétrique étant réalisée en présence de monoxyde de carbone en outre introduit dans le mélange réactionnel.

11. Procédé selon l'une quelconque des revendications 1 à 10, **caractérisé en ce que** la cyclisation selon l'étape de procédé b) est réalisée en présence d'un catalyseur contenant de l'aluminium acide de Lewis.

12. Procédé selon l'une quelconque des revendications 1 à 11, **caractérisé en ce que** la cyclisation selon l'étape de procédé b) est réalisée en présence de composés de diaryl-phénoxy-aluminium en tant que catalyseur, qui peuvent être obtenus par mise en réaction d'un ligand bis(diarylphénol) de formule (I) 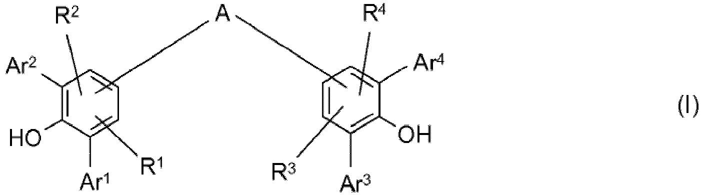 dans laquelle
Ar¹, Ar², Ar³, Ar⁴ sont identiques ou différents, et signifient chacun indépendamment les uns des autres un radical aryle de 6 à 15 atomes de carbone ou un radical hétéroaryle de 2 à 15 atomes de carbone, qui peuvent éventuellement porter chacun 1 à 7 substituants identiques ou différents, choisis dans le groupe constitué par les substituants alkyle en C₁ à C₆, perfluoroalkyle en C₁ à C₆, alcoxy en C₁ à C₆, aralkyle en C₇ à C₁₂, halogène, -SiR^{5a}R^{6a}R^{7a}, aryle en C₆ à C₁₀ substitué ou non substitué, -NR^{8a}R^{9a}, -SR^{10a} et -NO₂, R¹, R², R³, R⁴ sont identiques ou différents, et signifient chacun indépendamment les uns des autres hydrogène, alkyle en C₁ à C₆, perfluoroalkyle en C₁ à C₆, alcoxy en C₁ à C₆, aralkyle en C₇ à C₁₂, halogène,-SiR^{5b}R^{6b}R^{7b}, aryle en C₆ à C₁₀ substitué ou non substitué, -NR^{8b}R^{9b}, -SR^{10b} et/ou -NO₂, et R¹ ou R² et/ou R³ ou R⁴ peuvent former conjointement avec A un cycle aromatique ou non aromatique, et
A
(1) représente un radical hydrocarboné linéaire ou ramifié et/ou cyclique de 1 à 25 atomes de carbone, qui peut être saturé ou mono- ou polyinsaturé et/ou partiellement aromatique, et peut éventuellement comprendre un ou plusieurs hétéroatomes identiques ou différents, choisis dans le groupe des hétéroatomes 0, S et NR¹¹, et/ou un ou plusieurs groupes fonctionnels identiques ou différents, choisis dans le groupe constitué par les groupes fonctionnels C(O), S(O) et S(O)₂, et peut éventuellement porter un ou plusieurs substituants identiques ou différents choisis dans le groupe constitué par les substituants alkyle en C₁ à C₆, perfluoroalkyle en C₁ à C₆, alcoxy en C₁ à C₆, acyloxy en C₁ à C₁₀, aralkyle en C₇ à C₁₂, halogène, -SiR^{5c}R^{6c}R^{7c}, aryle en C₆ à C₁₀ substitué ou non substitué, hétaryle en C₂ à C₁₀ substitué ou non substitué, -NR^{8c}R^{9c}, -SR^{10c}, NO₂, acyle en C₁ à C₁₂ et carboxyle en C₁ à C₁₀, ou
(2) un radical aryle de 6 à 15 atomes de carbone ou un radical hétéroaryle de 2 à 15 atomes de carbone, qui peut éventuellement porter à chaque fois 1 à 5 substituants, choisis dans le groupe constitué par les substituants alkyle en C₁ à C₆, perfluoroalkyle en C₁ à C₆, alcoxy en C₁ à C₆, aralkyle en C₇ à C₁₂, halogène, -SiR^{5d}R^{6d}R^{7d}, aryle en C₆ à C₁₀ substitué ou non substitué, -NR^{8d}R^{9d}, SR^{10d} et NO₂, ou
(3) représente un groupe fonctionnel ou un hétéroatome choisi dans le groupe constitué par -O-,-S-, -N(R¹¹) -, -S(O)-, -C(O)-, -S(O)₂-, -P(R¹¹)-, - (R¹¹)P(O)- et -Si(R¹²R¹³),
les radicaux R^{5a}, R^{6a}, R^{7a}, R^{8a}, R^{9a}, R^{10a} à R^{5d}, R^{6d}, R^{7d}, R^{8d}, R^{9d}, R^{10d} et R¹¹ à R¹³ représentant chacun indépendamment les uns des autres alkyle en C₁ à C₆, aralkyle en C₇ à C₁₂ et/ou aryle en C₆ à C₁₀ substitué ou non substitué, et les radicaux R^{8a} et R^{9a}, R^{8b} et R^{9b}, R^{8c} et R^{9c}, R^{8d} et R^{9d} pouvant également chacun former ensemble indépendamment les uns des autres un radical hydrocarboné cyclique de 2 à 8 atomes de carbone, qui peut comprendre un ou plusieurs hétéroatomes identiques ou différents choisis dans le groupe constitué par 0, S et NR^{11a}, et R^{11a} pouvant avoir les significations indiquées pour R¹¹,
avec un composé d'aluminium de formule (XIV)
(R¹⁴)₃₋ₚAlHₚ (XIV)
dans laquelle
Al signifie aluminium et
R¹⁴ signifie un radical alkyle ramifié ou non ramifié de 1 à 5 atomes de carbone, et
p signifie 0 ou un nombre entier de 1 à 3,
et/ou
avec un composé d'aluminium de formule (XV)
MAlH₄ (XV)
dans laquelle
Al signifie aluminium et
M signifie lithium, sodium ou potassium.

13. Procédé selon la revendication 12, **caractérisé en ce que** la cyclisation selon l'étape de procédé b) est réalisée en présence d'un composé de diarylphénoxy-aluminium en tant que catalyseur, qui peut être obtenu par mise en réaction d'un ligand bis(diarylphénol) de formule (Ia₂-3) 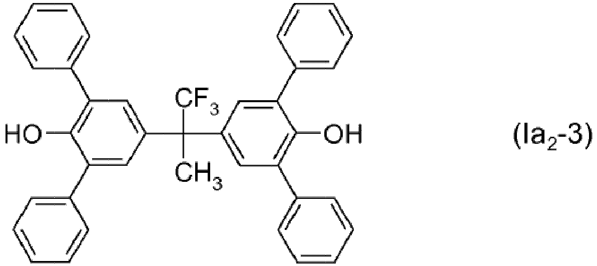 avec un composé d'aluminium de formule (XIV) et/ou avec un composé d'aluminium de formule (XV).

14. Procédé selon l'une quelconque des revendications 1 à 13, **caractérisé en ce que** l'isopulégol obtenu est purifié par distillation, la purification étant réalisée dans une colonne à paroi de séparation contenant 30 à 200 étapes de séparation théoriques et un ou plusieurs emplacements de sortie latérale à une pression d'exploitation absolue de 10 à 500 mbar.

15. Procédé selon l'une quelconque des revendications 1 à 14, **caractérisé en ce que** la purification de l'isopulégol selon l'étape de procédé c) est réalisée sous la forme d'une cristallisation à l'état fondu.

16. Procédé selon la revendication 15, **caractérisé en ce que** la cristallisation à l'état fondu est réalisée sous la forme d'une cristallisation en couches statique.

17. Procédé selon l'une quelconque des revendications 1 à 16, **caractérisé en ce que** l'hydrogénation catalytique selon l'étape de procédé d) est réalisée sous la forme d'un procédé de fabrication de menthol racémique ou optiquement actif de formule (XXIV) 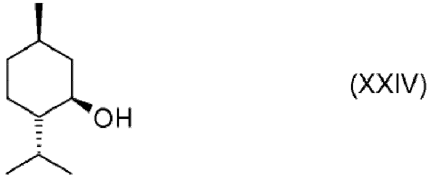 par hydrogénation catalytique d'isopulégol racémique ou optiquement actif de formule (XX) 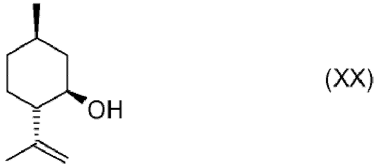 en présence d'hydrogène et d'un catalyseur, comprenant :
- 30 à 70 % en poids de composés oxygénés de nickel, calculé en tant que NiO,
- 15 à 45 % en poids de composés oxygénés de zirconium, calculé en tant que ZrO₂,
- 5 à 30 % en poids de composés oxygénés de cuivre, calculé en tant que CuO, et
- 0,1 à 10 % en poids de composés oxygénés de molybdène, calculé en tant que MoO₃,
les indications en % en poids se rapportant au catalyseur sec, non réduit.

18. Procédé selon l'une quelconque des revendications 1 à 17, **caractérisé en ce que** le menthol optiquement actif et/ou racémique obtenu est purifié par distillation dans le cadre d'une étape de procédé e) supplémentaire.

19. Procédé selon la revendication 18, **caractérisé en ce que** la purification par distillation de menthol racémique et/ou optiquement actif est réalisée par séparation par distillation de menthol optiquement actif ou racémique à partir de mélanges de matières contenant du menthol racémique ou optiquement actif et des diastéréomères de menthol, la séparation étant réalisée dans une colonne à paroi de séparation contenant 50 à 300 étapes de séparation théoriques et un ou plusieurs emplacements de sortie latérale à une pression d'exploitation de 5 à 500 mbar.
